# EUROPEAN PATENT APPLICATION

(11) **EP 2 460 539 A1**
(43) Date of publication of application: **06.06.2012**
(21) Application number: 10803811.8
(22) Date of filing: 30.07.2010
(51) Int. Cl.: A61K 47/34, A61K 9/16

(54) **NANOSPHERE OR MICROSPHERE DRUG CARRIER, PREPARATION METHOD, COMPOSITION AND USE THEREOF**

(30) Priority: 31.07.2009 WO PCT/CN2009/000862
(71) Applicant: Xi'an Libang Medical Technology Co., Ltd., Xi'an, Shaanxi 710065 (CN)
(72) Inventor: HU, Renle, Shaanxi 710065 (CN); WANG, Jiucheng, Shaanxi 710065 (CN); CHEN, Tao, Shaanxi 710065 (CN); LIANG, Hua, Shaanxi 710065 (CN); DING, Duohao, Shaanxi 710065 (CN); JIAO, Yaqi, Shaanxi 710065 (CN)
(74) Representative: O'Neill, Michelle
(86) International application number: PCT/CN2010/001154
(87) International publication number: WO 2011/011978

(57) **Abstract**

A nanosphere or microsphere drug carrier, formulations comprising the drug carrier and the preparation method of the formulations and the use of the carrier are disclosed. The carrier comprises a biodegradable methoxy end-capped polyethylene glycol-polylactide block copolymersor a derivative thereof represented by formula (I) as the main carrier material: CH₃O-[CH₂-CH₂-O]ₘ-[C(O)-CH(CH₃)-O]ₙ-R (I).

## Description

### TECHNICAL FIELD

The present invention relates to the field of medical technology. Specifically, the present invention relates to a drug carrier composition, wherein the drug loading carrier is a formulation of the drug carrier composition, and the preparation method of the formulation, as well as the use of the drug carrier composition. More specifically, the present invention relates to a composition of nanosphere or microsphere drug carrier, wherein the drug loading carrier is a formulation of the composition of nanosphere or microsphere drug carrier, and the preparation method of the formulation of nanosphere or microsphere drug carrier, as well as the use of the composition of nanosphere or microsphere drug carrier.

### BACKGROUND OF THE INVENTION

Microsphere formulation of drug carriers is a new formulation developed in recent years. Microsphere carrier as a new drug carrier, is a spherical carrier drug delivery system which is made of biodegradable materials such as starch, protein, chitosan, polylactic acid, polylactic acid- polyglycolic acid copolymer, cellulose, and gelatin etc.. Drug in the microsphere is dispersed or embed in the material to form spherical solid with the particle size is generally 0.3 ∼ 300µm. Typically, the sphere with particle size less than 1µm is called nanosphere or millimicrosphere, and the sphere with particle size more than 1µm is called microsphere. It can carry the active molecules to the diseased tissues and human organs, and then control the drug release in the target organs, which not only can reduce many adverse drug reactions, but also can improve the selectivity and therapeutic index of the drugs. It is of important significance to develop and use microsphere drug carrier for the development of controlled release and targeting drug delivery system.

Compared with the traditional formulations, the microsphere formulation has the following advantages: (1) greatly reducing the administration dose and frequency, and improving compliance of the patient; (2) long sustained release time that can prolong the action time of the short half-time drugs, and keep the drug concentration in vivo stable; (3) less toxic and side effects; (4) targeting; (5) improving the stability of drugs to protect polypeptide and protein from destruction by acid and enzyme.

With the development of the new technology, new process and new materials, long-acting biodegradable injection microsphere has become one of the most important research fields for new drug formulations. Especially in the recent ten years, new biodegradable polymers have become the important carriers for microsphere formulations, and commonly used are polylactic acid (PLA), polyglycolic acid (PGA), polylactic acid- polyglycolic acid copolymer (PLGA), polycaprolactone, polycarbonate etc., wherein PLA and PLGA have favorable security, biocompatibility and biodegradability and have been approved by FDA for clinical use. Originally, they were made into suture for surgery and screw for fixing bones etc., and now the products made of them on the market include leuprorelin microsphere (Lupron Depot), triptorelin microsphere (Trelstar Depot), octreotide microsphere (Sandostatin LAR), somatotropin microsphere (Neutropin Depot), and goserelin implant (Zoladex) etc..

Microsphere is a kind of new drug carrier with great development potential, however, it still has many problems at present, that directly result in some drugs are difficult to be put into the market. Such problems are, for example, low encapsulation efficiency and drug loading rate; non-zerolevel release of drugs caused by the shape of microsphere and the biodegradation in vivo etc.; have not realized for more effectively making drug release occurred in the most suitable period; the insufficient research on different release procedure and release rate of drugs in the sustained release system such that it can not achieve comprehensive prevention and treatment of some diseases; and intellectualization is not realized etc.. These problems are substantially resulted from the defects of property of the drug carrier materials.

Polylactic acid, polylactic acid- polyglycolic acid copolymer, and polycaprolactone etc. are all composed of liposoluble fragments, and these macromolecular compound can only regulate the drug release rate by controlling their molecule weight. However, it is difficult to obtain ideal molecule weight when the synthesis of materials, as there are many factors that influence the polymerization of macromolecular compound. Moreover, when preparing the drug loading microsphere, the drug loading rate and encapsulation efficiency of some hydrophilic compounds are lower because of the high liposolubility feature. In recent years, many researches have been focused on polylactic acid-polyglycolic acid microsphere, and it would cause strong irritation to the administration site or blood vessel after subcutaneous injection or intravenous injection, because the degradation of the carrier material would release strong acid glycolic acid. For these reasons, the use of these high molecular materials as drug carrier is restricted.

Therefore, there is a need to develop a new drug carrier system which has improved drug encapsulation efficiency and drug loading rate, steady drug release rate, no irritation to the administration site or blood vessel, and less toxic and side-effect.

### SUMMARY OF THE INVENTION

During the research work on the drug microsphere formulations, the inventor of the present invention find that using a methoxy end-capped polyethylene glycol-polylactic acid block copolymer or a derivative thereof as a carrier material of a drug microsphere can substantially solve the above problems.

Therefore, one purpose of the present invention is to provide a nanosphere or microsphere drug carrier composition that has higher drug loading rate and encapsulation efficiency, controllable drug release rate without stimulation on the administration site or blood vessel; another purpose of the present invention is to provide a drug loading nanosphere or microsphere formulation, wherein the drug loading carrier is the above mentioned drug carrier composition; yet another purpose of the present invention is to provide a method for preparing the drug loading nanosphere or microsphere formulation; further purpose of the present invention is to provide the use of said microsphere drug carrier composition.

Aiming at the above purposes of the invention, the technical solutions of the present invention are as follows:

In one aspect, the present invention provides a nanosphere or microsphere drug carrier, the carrier includes a biodegradable methoxy end-capped polyethylene glycol-polylactic acid block copolymer or a derivative thereof represented by the following formula (I): 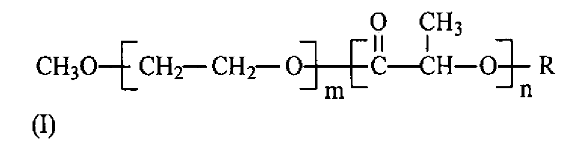 wherein:
m = 4 ∼ 454, preferably 20 ∼ 454, more preferably 120 ∼ 230 or 20 ∼ 45, and most preferably 45;
n = 4 ∼ 2778, preferably 60 ∼ 1400, more preferably 300 ∼ 1400 or 60 ∼ 150, and most preferably 400 ∼ 555;
substituent group R is selected from:
a. a neutral terminal group
-H, -CH₃, -CH₂CH₃, -CH₂(CH₂)ₓCH₃, wherein x=1-8;
b. a negatively charged terminal group
one negative charge: -COCH₂CH₂CO₂H
two negative charges: -COCH₂CH₂CONHCH(CO₂H)(CH₂)₂CO₂H
four negative charges:

-COCH₂CH₂CONHCH[CONHCH(CO₂)(CH₂)₂CO₂H](CH₂)₂[CONHCH(CO₂)(CH₂)₂CO₂H];

and
c. a positively charged terminal group
one positive charge: -COCH₂CH₂NH₂
two positive charges: -COCH₂CH₂NHCOCH(NH₂)(CH₂)₄NH₂
four positive charges:

-COCH₂CH₂NHCOCH[NHCOCH(NH₂)(CH₂)₄NH₂](CH₂)₄NH[COCH(NH₂)(CH₂)₄NH_{2]}.

Preferably, according to the above mentioned drug carrier, wherein the HLB value of the methoxy end-capped polyethylene glycol-polylactic acid block copolymer or derivative thereof is 0.01 ∼ 19.84.

Preferably, according to the above mentioned drug carrier, wherein the drug carrier further includes one or more other high molecular materials for regulating the drug release rate, preferably, the mass ratio of the other high molecular material to the biodegradable copolymer or a derivative thereof represented by formula (I)is 0% ∼ 50%.

In another aspect, the present invention provides a nanosphere or microsphere drug formulation, wherein the drug formulation includes the above drug carrier.

Preferably, according to the above mentioned nanosphere or microsphere drug formulation, wherein the nanosphere or microsphere is the nanosphere or microsphere prepared by the above drug carrier enwrapping active pharmaceutical ingredient; preferably, the active pharmaceutical ingredient is selected from one or more of the following: antituberculosis drugs, antileprosy drugs, antiviral drugs, antimalarial drugs, antiamebic drugs, antitrichomonal drugs, antifilarial drugs, anthelminthic drugs, broad-spectrum antibiotics, antifungal drugs, analgesic drugs, analgesic-antipyretic drugs, antigout drugs, antiepileptic drugs, antiparkinsonism drugs, antipsychotic drugs, antianxiety drugs, antidepressants drugs, drugs affecting brain blood vessels, cerebral metabolism and nootropic drugs, calcium antagonists, drugs for treating chronic cardiac insufficiency, antiarrhythmic drugs, peripheral vasodilators, blood lipid regulating and antiarteriosclerotic drugs, drugs for promoting proliferation of leukocyte, antiplatelet drugs, hormons drugs, contraceptive drugs, hypoglycemic drugs, thyroid hormones drugs and antithyroid drugs, antineoplastic drugs, drugs affecting immunity, slimming drugs, anti-osteoporotic drugs and drugs against prostatic hyperplasia.

Preferably, the active pharmaceutical ingredient is selected from one or more of the following: Rifampin, Amlodipine, Stavudine, Azithromycin, Naproxen, Ropinirole, Paroxetine, Cinnarizine, Lovastatin, Fulvestrant, Orlistat, Fluconazol, Tramadol hydrochloride, Carbamazepine, Clarithromycin, Meloxicam, Probenecid, Thioridazine hydrochloride, Timiperone, Chlorprothixene, Risperidone, Alprazolam, Trazodone, Famciclovir, Amitriptyline hydrochloride, Nimodipine, Donepezil, Captopril, Norethindrone, Gliclazide and Melphalan.

More preferably, the active pharmaceutical ingredient is Fulvestrant, Naproxen, or Carbamazepine.

Preferably, according to the above mentioned nanosphere or microsphere drug formulation, wherein the particle size of the drug carrier nanosphere or microsphere is 100nm ∼ 1 mm; the drug loading rate is 0.01% ∼ 30%, preferably 5% ∼ 30%, more preferably 10% ∼ 30%, and most preferably 20% ∼ 30%.

In another aspect, the present invention provides a method for preparing the above mentioned nanosphere or microsphere drug formulation, the method includes:
a. dispersing the active pharmaceutical ingredient in a solvent system containing the dissolved carrier material described above;
b. adding into a nonsolvent system to form nanosphere or microsphere;
c. solidifying, collecting, washing and drying;
preferably, the solvent of the carrier material is one or more of dichloromethane, chloroform, tetrahydrofuran, ethanol, and ethyl acetate;
preferably, the concentration of the carrier material in the solvent system is 0.1%% ∼ 50% (g/ml);
preferably, the concentration of the active pharmaceutical ingredient in the solvent system which contains the dissolved carrier material is 0.01% ∼ 80% (g/ml);
preferably, the nonsolvent system is ethyl ether, petroleum ether, n-hexane, cyclohexane, acetone;
preferably, the volume ratio of the solvent system to the nonsolvent system is 10:1 ∼ 1:10; and/or
preferably, adding one or more of polyisobutyl ester, polyethylene, and butyl rubber into the nonsolvent system as an antisticking agent; more preferably, the mass ratio of the antisticking agent to the carrier material is 0:10 ∼ 2:10.

Preferably, according to the above mentioned method for preparing nanosphere or microsphere drug formulation, the method includes:
a. dissolving the active pharmaceutical ingredient and the above mentioned carrier material in the organic solvent to make an oil phase;
b. adding the oil phase in the aqueous phase and emulsifying to get an oil-in-water (O/W) type emulsion;
c. stirring and warming up the O/W type emulsion to completely volatilize the organic solvent in the O/W type emulsion;
d. filtering, washing, collecting and drying;
preferably, the solvent of the carrier material is one or more of dichloromethane, chloroform, tetrahydrofuran, ethanol, and ethyl acetate;
preferably, the mass ratio of the drug to the carrier material is 1:50-1:3; preferable concentration of the carrier material in the oil phase is 1% ∼ 50% (g/ml);
preferably, the aqueous phase is one of or a mixed solution of two or more of surfactant solution, monosaccharide or polysaccharide solution, polylol solution, cellulose solution, and colloidal solution, and the pH value of the aqueous phase is in the range of 3.0 ∼ 10.5;
preferably, the pH adjusting agent used is selected from an inorganic acid, organic acid, inorganic base, organic base and buffer salt; and/or
preferably, the volume ratio of the oil phase to the aqueous phase is 1:300 ∼ 1:5.

Preferably, according to the above preparing method, the method includes:
a. dissolving or dispersing the drug in a solvent system containing the dissolved carrier material as described above;
b. spraying into the drying tower of a spray drying equipment in the form of spray, and drying, isolating, collecting;
wherein the solvent of the carrier material is one or more of dichloromethane, chloroform, tetrahydrofuran, ethanol, and ethyl acetate;
preferably, the concentration of the carrier material in the solvent system is 0.1% ∼ 50% (glml);
preferably, the concentration of the dissolved or dispersed drug in the solvent system of the carrier material is 0.01% ∼ 50% (g/ml); preferably, the inlet air temperature is 30°C ∼ 80°C;
preferably, the carrier material further comprises a plasticizer; more preferably, the plasticizer is one or more of dimethyl phthalate, diethyl phthalate, dibutyl benzoate, dibutyl sebacate, tributyl citrate, tributyl acetylcitrate, and glyceryl triacetate; the mass ratio of the plasticizer to the carrier material is 0% ∼ 50%; and/or
preferably, the solvent system further comprises an antisticking agent, the antisticking agent is one or more of cholesterol, glycerol monostearate, talc powder, silica gel, and magnesium stearate, the mass ratio of the antisticking agent to the carrier material is 0% ∼ 100%.

In another aspect, the present invention provides the use of a biodegradable methoxy end-capped polyethylene glycol-polylactic acid block copolymer or a derivative thereof represented by the following formula (I) in the preparation of a drug carrier, 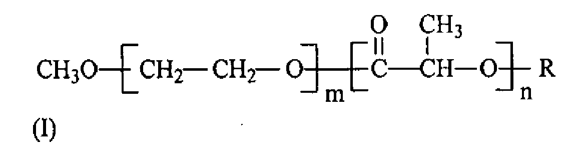 wherein:
m = 4 ∼ 454, preferably 20 ∼ 454, more preferably 120 ∼ 230 or 20 ∼ 45, and most preferably 45;
n = 4 ∼ 2778, preferably 60 ∼ 1400, more preferably 300 ∼ 1400 or 60 ∼ 150, and most preferably 400 ∼ 555;
substituent group R is selected from:

a. a neutral terminal group
   -H, -CH₃, -CH₂CH₃, -CH₂(CH₂)ₓCH₃, wherein x=1-8;
b. a negatively charged terminal group
   one negative charge: -COCH₂CH₂CO₂H
   two negative charges: -COCH₂CH₂CONHCH(CO₂H)(CH₂)₂CO₂H
   four negative charges:

   -COCH₂CH₂CONHCH[CONHCH(CO₂)(CH₂)₂CO₂H](CH₂)₂[CONHCH(CO₂)(CH₂)₂CO₂H];

   and
c. a positively charged terminal group
   one positive charge: -COCH₂CH₂NH₂
   two positive charges: -COCH₂CH₂NHCOCH(NH₂)(CH₂)₄NH₂
   four positive charges:

   -COCH₂CH₂NHCOCH[NHCOCH(NH₂)(CH₂)₄NH₂](CH₂)₄NH[COCH(NH₂)(CH₂)₄NH₂].

The purpose of the present invention can also be achieved by the following technical solutions: In one aspect, the present invention provides a nanosphere or microsphere drug carrier composition, the composition includes a biodegradable methoxy end-capped polyethylene glycol-polylactic acid block copolymeror aderivative thereof represented by the following structural formula (I) as a main carrier material, 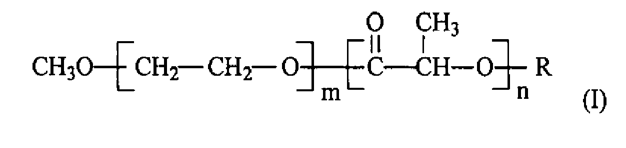
m = 4 ∼ 454
n = 4 ∼ 2778
R is selected from:
   a. a neutral terminal group
      -H, -CH₃, -CH₂CH₃, -CH₂(CH₂)ₓCH₃, wherein x=1-8;
   b. a negatively charged terminal group
      one negative charge: -COCH₂CH₂CO₂H
      two negative charges: -COCH₂CH₂CONHCH(CO₂H)(CH₂)₂CO₂H
      four negative charges:

      -COCH₂CH₂CONHCH[CONHCH(CO₂)(CH₂)₂CO₂H](CH₂)₂[CONHCH(CO₂)(CH₂)₂CO₂H];

      and
   c. a positively charged terminal group
      one positive charge: -COCH₂CH₂NH₂
      two positive charges: -COCH₂CH₂NHCOCH(NH₂)(CH₂)₄NH₂
      four positive charges:

      -COCH₂CH₂NHCOCH[NHCOCH(NH₂)(CH₂)₄NH₂](CH₂)₄NH[COCH(NH₂)(CH₂)₄NH₂].

It can be seen from the structural formula (I) that the high molecular polymer carrier material is composed of a hydrophilic fragment methoxy end-capped polyethylene glycol and lipophilic fragment polylactic acid or a derivative thereof. Because of this property, the drug carrier composition is suited for enwrapping various drugs, and able to obtain satisfactory drug loading rate and encapsulation efficiency. Meanwhile, with respect to the drug release rate, the HLB value (hydrophile-lipophile balance) of the carrier material can be regulated by controlling the size of the lipophilic and hydrophilic fragments, thereby really achieving the controllability of the drug release rate.

The relative molecular weight of the carrier material is: methoxy end-capped polyethylene glycol (212 ∼ 20000)-polylactic acid or derivative thereof (288 ∼ 200000). Preferably, the relative molecular weight of the carrier material is: methoxy end-capped polyethylene glycol (1000 ∼ 10000)-polylactic acid or derivative thereof (5000 ∼ 100000).

For example, in some embodiments of the present invention, the drug carrier composition is used for enwrapping hydrophilic drugs, or drugs which have good affinity with the polyethylene glycol fragment and the polylactic acid fragment. In addition, the drug carrier composition of the present invention is still suited for enwrapping liposoluble drugs. As for some liposoluble drugs, the terminal group -R of the polylactic acid can be modified according to their properties (such as charges etc.) to enhance the affinity of the drug with the carrier material, so as to obtain carrier microspheres with higher drug loading rate and encapsulation efficiency. Therefore, in some embodiments of the present invention, R is -H in the structural formula (I); in another embodiment, R is -CH₃ in the structural formula (1); in yet another embodiment, R is -CH₂CH₃ in the structural formula (1); in yet some other embodiments, R is -CH₂(CH₂)ₓCH₃, wherein x=1-8 in the structural formula (I). As for some liposoluble drugs, in some embodiments, R in the structural formula (I) is a negatively charged terminal group, preferably the negatively charged terminal group is charged with one negative charge, such as -COCH₂CH₂CO₂H, two negative charges, such as -COCH₂CH₂CONHCH(CO₂H)(CH₂)₂CO₂H, and four negative charges, such as -COCH₂CH₂CONHCH[CONHCH(CO₂)(CH₂)₂CO₂H](CH₂)₂[CONHCH(CO₂)(CH₂)₂CO₂H]. In some other embodiments, R in the structural formula (I) is a positively charged terminal group, preferably the positively charged terminal group is charged with one positive charge, such as -COCH₂CH₂NH₂, two positive charges, such as -COCH₂CH₂NHCOCH(NH₂)(CH₂)₄NH₂, and four positive charges, such as -COCH₂CH₂NHCOCH[NHCOCH(NH₂)(CH₂)₄NH₂](CH₂)₄NH[COCH(NH₂)(CH₂)₄NH₂]. In one preferred embodiment, R is -COCH₂CH₂CO₂H in the structural formula (I). In one preferred embodiment, R is -COCH₂CH₂CONHCH(CO₂H)(CH₂)₂CO₂H in the structural formula (I). In another preferred embodiment, R is -COCH₂CH₂CONHCH[CONHCH(CO₂)(CH₂)₂CO₂H](CH₂)₂[CONHCH(CO₂)(CH₂)₂CO₂H] in the structural formula (I). In one preferred embodiment, R is -COCH₂CH₂NH₂ in the structural formula (I). In one preferred embodiment, R is -COCH₂CH₂NHCOCH(NH₂)(CH₂)₄NH₂ in the structural formula (I). In another preferred embodiment, R is -COCH₂CH₂NHCOCH[NHCOCH(NH₂)(CH₂)₄NH₂](CH₂)₄NH[COCH(NH₂)(CH₂)₄NH₂] in the structural formula (I).

The HLB value of the carrier material used in the present invention is 0.01 ∼ 19.84. Carrier materials with different HLB value can be chosen based on the properties of the drugs enwrapped and the demand of drug release rate. In addition, the drug carrier can further include one or more other high molecular material as auxiliary material in order to regulate the drug release rate. Preferably, the mass ratio of the other high molecular material to the carrier material is 0% ∼ 50%. The other high molecular material is: polylactic acid, polyglycolic acid, poly(lactic-co-glycolic acid), polycaprolactone etc..

In one preferred embodiment, the nanosphere or microsphere drug carrier according to the present invention includes a methoxy end-capped polyethylene glycol-polylactic acid block copolymer or a derivative thereof represented by the above mentioned formula (I) wherein R is a positively charged terminal group, for example methoxy end-capped polyethylene glycol-polylactic acid-alanine, and the enwrapped active pharmaceutical ingredients are drugs with a negatively charged group, for example Naproxen etc.. Compared with the drug carrier microsphere or nanosphere formulation prepared with other carrier material, the drug carrier microsphere or nanosphere formulation prepared with the nanpsphere or microsphere drug carrier of the present invention, which includes a methoxy end-capped polyethylene glycol-polylactic acid block copolymer or derivative thereof represented by formula (I) wherein R is a positively charged terminal group, for example methoxy end-capped polyethylene glycol-polylactic acid-alanine, has excellent encapsulation efficiency, significant increased drug loading rate, and sustained drug release rate in vitro.

In another preferred embodiment, the nanosphere or microsphere drug carrier according to the present invention includes a methoxy end-capped polyethylene glycol-polylactic acid block copolymer or a derivative thereof represented by the above mentioned formula (I) wherein R is a negatively charged terminal group, for example methoxy end-capped polyethylene glycol-polylactic acid- succinic acid, and the enwrapped active pharmaceutical ingredients are drugs with positively charged group, for example Carbamazepine etc.. Compared with the drug carrier microsphere or nanosphere formulation prepared with other carrier material, the drug carrier microsphere or nanosphere formulation prepared with the nanpsphere or microsphere drug carrier of the present invention, which includes a methoxy end-capped polyethylene glycol-polylactic acid block copolymer or derivative thereof represented by formula (I) wherein R is a negatively charged terminal group , for example, methoxy end-capped polyethylene glycol-polylactic acid- succinic acid, has excellent encapsulation efficiency, significant increased drug loading rate, and sustained drug release rate in vitro.

In another aspect, the present invention provides a drug carrier nanosphere or microsphere formulation, wherein the drug carrier is the above mentioned drug carrier composition according to the present invention.

Preferably, the particle size of the drug carrier nanosphere or microsphere in the drug carrier nanosphere or microsphere formulation is 100nm ∼ 1mm.

The drug loading rate of the drug carrier nanosphere or microsphere in the drug carrier nanosphere or microsphere formulation is 0.01% ∼ 30%, preferably, the drug loading rate of the drug carrier nanosphere or microsphere is 5% ∼ 30%, more preferably, the drug loading rate of the drug carrier nanosphere or microsphere is 10% ∼ 30%, and most preferably, the drug loading rate of the drug carrier nanosphere or microsphere is 20% ∼ 30%.

The drug carrier nanosphere or microsphere formulation of the present invention can be used in different administration routes. In one embodiment, the nanosphere or microsphere formulation of the present invention is oral preparation. In another embodiment, the nanosphere or microsphere formulation of the present invention is made into injection for intravenous or subcutaneous injection use.

In another aspect, the present invention further provides a preparation method for the drug carrier nanosphere or microsphere formulations. The drug carrier nanosphere or microsphere formulation of the present invention can be prepared with various methods such as phase separation method, in-liquid drying method or spray drying method etc..

According to one preferred embodiment of the present invention, phase separation method is used for preparing the drug carrier nanosphere or microsphere formulation of the present invention, the method includes the following steps:
a. dispersing the drug in the solvent system containing the dissolved carrier material of the present invention;
b. adding into nonsolvent system to form microsphere or nanosphere, preferably adding slowly and dispersing under stirring or high speed shearing or high pressure homogenizing or using microjet pump;
c. solidifying, collecting, washing and drying;
preferably the concentration of the carrier material in the solvent system is 0.1% ∼ 50% (g/ml);
preferably, the concentration of the drug in the solvent system dissolved the carrier material is 0.01% ∼ 80% (g/ml); preferably the stirring rate is 100 ∼ 1000rpm, the shearing rate is 1000 ∼ 10000rpm, the pressure of the high pressure homogenizer is 200 ∼ 2000bar, once ∼ 10 times, the pressure of the microjet pump is 100 ∼ 2000bar, once ∼ 10 times; preferably the nonsolvent system is ethyl ether, petroleum ether, n-hexane, cyclohexane, acetone; preferably the volume ratio of the solvent system to the nonslovent system is 10:1 ∼ 1:10; preferably, adding one or more of polyisobutyl ester, polyethylene, and butyl rubber into the nonsolvent system as an antisticking agent; more preferably, the mass ratio of the antisticking agent to the carrier material is 0:10 ∼ 2:10.

According to another preferred embodiment of the present invention, in-liquid drying method is used for preparing the drug carrier nanosphere or microsphere formulation of the present invention, the method includes the following steps:
a. dissolving the drug and the carrier material of the present invention in the organic solvent to make an oil phase;
b. adding the oil phase into the aqueous phase and emulsifying to get the oil-in-water (O/W) type emulsion, preferably emulsifying under stirring or high speed shearing or high pressure homogenizing or using microjet pump;
c. stirring and warming up the O/W type emulsion to completely volatilize the organic solvent in the O/W type emulsion;
d. filtering, washing, collecting and drying;
wherein the mass ratio of the drug to the carrier material is 1:50-1:3; preferably the concentration of the carrier material in the oil phase is 1% ∼ 50% (g/ml); preferably, the aqueous phase is one of or a mixed solution of two or more of surfactant solution, monosaccharide or polysaccharide solution, polylol solution, cellulose solution, and colloidal solution, and the pH value of aqueous phase is in the range of 3.0 ∼ 10.5; preferably, the material used for adjusting pH value is inorganic acid, organic acid, inorganic base, organic base or buffer salt; preferably, the volume ratio of the oil phase to the aqueous phase is 1:300 ∼ 1:5, preferably the mechanical stirring rate is 100 ∼ 1000rpm, the shearing rate is 1000 ∼ 10000rpm, the pressure of the high pressure homogenizer and the microjet pump is 100 ∼ 1500bar, once ∼ 10 times.

According to another preferred embodiment of the present invention, spray drying method is used for preparing the drug carrier nanosphere or microsphere formulation of the present invention, the method includes the following steps:
a. dissolving or dispersing the drug in the solvent system of the carrier material of the present invention;
b. spraying into the drying tower of a spray drying equipment in the form of spray, and drying, isolating, collecting;
wherein preferably the concentration of the carrier material in the solvent system is 0.1% ∼ 50% (g/ml); preferably, the concentration of the dissolved or dispersed drug in the solvent system of the carrier material is 0.01% ∼ 50% (g/ml); preferably, the inlet air temperature is 30°C ∼ 80°C;
preferably, the carrier material further comprises plasticizer; preferably, the plasticizer is one or more of dimethyl phthalate, diethyl phthalate, dibutyl benzoate, dibutyl sebacate, tributyl citrate, tributyl acetylcitrate, and glyceryl triacetate; the mass ratio of the plasticizer to the carrier material is 0% ∼ 50%; preferably, the solvent system further comprises an antisticking agent, the antisticking agent is one or more of cholesterol, glycerol monostearate, talc powder, silica gel, and magnesium stearate, the mass ratio of the antisticking agent to the carrier material is 0% ∼ 100%.

In the preparation method of the drug carrier nanosphere or microsphere formulation disclosed in the present invention, the nanosphere/microsphere can be dried under atmospheric pressure, under reduced pressure, or dried by lyophilization. In a preferred embodiment, the temperature of atmospheric drying and drying under reduced pressure is 25°C ∼ 80°C. In a preferred embodiment, the pre-freezed temperature of lyophilization is -25°C ∼ -45°C, and the primary drying temperature is 15°C ∼ 40°C.

In addition, in the preparation method of the drug carrier nanosphere or microsphere formulation disclosed in the present invention, the solvent of the carrier material can be dichloromethane, chloroform, tetrahydrofuran, ethanol, and ethyl acetate, and the aforesaid solvents can be used alone or in mixture.

In another aspect, the present invention further provides the use of the drug carrier nanosphere or microsphere formulation of the present invention for treating diseases. The type of the disease to be treated is depending on the drug contained in the drug carrier nanosphere or microsphere formulation.

In yet another aspect, the present invention further provides the use of the nanosphere or microsphere drug carrier composition of the present invention in the preparation of medicaments.

Compared with the prior art, the advantageous technical effects of the present invention include: With regard to the microsphere drug carrier of the present invention, as the synthesis of the block copolymeric high molecular carrier material of the present invention is controllable, which makes it possible to select suitably the molecular weight, ratio of hydrophilic/ lipophilic fragment of the high molecular copolymer and positioning different active functional groups according to different properties of the drugs enwrapped, thereby increasing the drug encapsulation efficiency and the drug loading rate, and achieving the controllability of the drug release rate. For example, when synthesizing these new high molecular materials, HLB value (hydrophile-lipophile balance) can be controlled by pre-calculating the molecular weight of the methoxy end-capped polyethylene glycol and polylactic acid and derivative thereof, which enables the carrier material to be suited for different drugs, and gives the drug carriers with different drug release features, so as to be suited for different clinical treatment and achieve different purposes of medication.

With regard to the drug composition that uses the drug carrier of the present invention, for example drug carrier nanosphere or microsphere formulations, the concentration of the drug in vivo can be maintained at a steady level for a long time due to their sustained drug release rate and it avoids the change of the blood concentration caused by frequent administration, increases the stability of the drugs, reduces the toxic and side effect, and improves the drug safety of the patients, as compared with the common formulations. In addition, the degradation of the high molecular polymer will not produce glycolic acid, and will not cause stimulation on the administration site or blood vessel, which greatly improves the drug safety.

As for the preparation methods of the copolymer microsphere, in-liquid drying method is mostly used in the present techniques, and the mostly used solvents in in-liquid drying method is dichloromethane and someone also use acetone. The aqueous solution comprising surfactant is mostly used as the continuous phase and someone also use deionized water. Stirring is mostly used in the method of emulsification and someone also use supersonic. The continuous drying method with slowly warming-up is mostly used as the method for solidification and someone also use rapid rotating evaporation. Phase separation method and spray drying method are used in the present invention, and both of them have achieved good preparation effect. Meanwile, the present invention has improved the in-liquid drying method for preparing drug carrier nanosphere or microsphere formulations, which has the following distinct features and advantages as compared with the prior art:
1) Single or mixed organic solvents of dichloromethane, chloroform, tetrahydrofuran, ethanol, and ethyl acetate etc. are used in the present invention, and all of these organic solvents have certain solubility in water, therefore they are particularly suited for in-liquid drying method;
2) One of or a mixed solution of two or more of surfactant solution, monosaccharide or polysaccharide solution, polylol solution, cellulose solution, and colloidal solution are used as continuous phase of the present invention, which are more beneficial to the formation and stabilization of the emulsion droplets as compared with the deionized water used in the prior art;
3) Emulsification methods of mechanical agitation, high speed shearing, high pressure homogenizing or microjet pump etc. used in the present is easier to control the particle size of the microsphere than the supersonic emulsification method;
4) Compared with the rapid drying rotating evaporation method, the continuous drying method with slowly warming-up for solidification used in the present invention is easy to ensure the microsphere to keep its shape during solidification, but difficult to get together and break down, which result in the reduce of the encapsulation efficiency. In addition, the drug release rate of the microsphere of the present invention is more stable.

### BRIEF DESCRIPTION OF THE DRAWINGS

Fig.1 shows the testing spectrum of molecular weight distribution of the methoxy end-capped polyethylene glycol-polylactic acid block copolymer (mPEG-PLA, 10000/10000) prepared in Example 1.
Fig.2 shows the H-NMR testing spectrum of the methoxy end-capped polyethylene glycol-polylactic acid block copolymer (mPEG-PLA, 5000/8000) prepared in Example 2.
Fig.3 shows the H-NMR testing spectrum of the methoxy end-capped polyethylene glycol-polylactic acid block copolymer (mPEG-PLA, 2000/20000) prepared in Example 3.
Fig.4 shows the DSC-Tg testing spectrum of the methoxy end-capped polyethylene glycol-polylactic acid block copolymer (mPEG-PLA, 2000/20000) prepared in Example 3.
Fig.5 shows the DSC-Tf testing spectrum of the methoxy end-capped polyethylene glycol-polylactic acid block copolymer (mPEG-PLA, 2000/20000) prepared in Example 3.
Fig.6 shows the testing spectrum of molecular weight distribution of the methoxy end-capped polyethylene glycol-polylactic acid block copolymer (mPEG-PLA, 10000/10000) prepared in Example 4.
Fig.7 shows the H-NMR testing spectrum of the methoxy end-capped polyethylene glycol-polylactic acid block copolymer (mPEG-PLA, 20000/120000) prepared in Example 5.
Fig.8 shows the DSC-Tg testing spectrum of the methoxy end-capped polyethylene glycol-polylactic acid block copolymer (mPEG-PLA, 20000/120000) prepared in Example 5.
Fig.9 shows the DSC-Tf testing spectrum of the methoxy end-capped polyethylene glycol-polylactic acid block copolymer (mPEG-PLA, 20000/120000) prepared in Example 5.
Fig.10 shows the H-NMR testing spectrum of the methoxy end-capped polyethylene glycol-polylactic acid block copolymer (mPEG-PLA, 15000/55000) prepared in Example 6.
Fig.11 shows the H-NMR testing spectrum of the methoxy end-capped polyethylene glycol-polylactic acid block copolymer (mPEG-PLA, 2000/40000) prepared in Example 7.
Fig. 12A and Fig.12B respectively show ¹H-NMR and ¹³C-NMR testing spectrums of the methoxy end-capped polyethylene glycol-polylactic acid block copolymer (mPEG-PLA (2000/20000)-decane) prepared in Example 9.
Fig. 13 shows GPC testing spectrum of the molecular weight distribution of the methoxy end-capped polyethylene glycol-polylactic acid block copolymer (mPEG-PLA (2000/20000)-decane) prepared in Example 9.
Fig. 14A and Fig.14B respectively show ¹H-NMR and ¹³C-NMR testing spectrums of the methoxy end-capped polyethylene glycol-polylactic acid block copolymer (mPEG-PLA(2000/40000)-succinic acid) prepared in Example 10.
Fig. 15 shows GPC testing spectrum of the molecular weight distribution of the methoxy end-capped polyethylene glycol-polylactic acid block copolymer (mPEG-PLA(2000/40000)-succinic acid) prepared in Example 10.
Fig. 16A and Fig.16B respectively show ¹H-NMR and ¹³C-NMR testing spectrums of the methoxy end-capped polyethylene glycol-polylactic acid block copolymer (mPEG-PLA(2000/40000)-succinic acid-glutamic acid) prepared in Example 11.
Fig. 17A and Fig.17B respectively show ¹H-NMR and ¹³C-NMR testing spectrums of the methoxy end-capped polyethylene glycol-polylactic acid block copolymer (mPEG-PLA(2000/40000)-succinic acid-glutamic acid-glutamic acid₂) prepared in Example 12.
Fig. 18 shows GPC testing spectrum of the molecular weight distribution of the methoxy end-capped polyethylene glycol-polylactic acid block copolymer (mPEG-PLA(2000/40000)-succinic acid-glutamic acid-glutamic acid₂) prepared in Example 12.
Fig. 19A and Fig.19B respectively show ¹H-NMR and ¹³C-NMR testing spectrums of the methoxy end-capped polyethylene glycol-polylactic acid block copolymer (mPEG-PLA(2000/20000)-alanine) prepared in Example 13.
Fig. 20 shows GPC testing spectrum of the molecular weight distribution of the methoxy end-capped polyethylene glycol-polylactic acid block copolymer (mPEG-PLA(2000/20000)-alanine) prepared in Example 13.
Fig. 21A and Fig.21B respectively show ¹H-NMR and ¹³C-NMR testing spectrums of the methoxy end-capped polyethylene glycol-polylactic acid block copolymer (mPEG-PLA(2000/20000)- alanine-lysine) prepared in Example 14.
Fig. 22 shows GPC testing spectrum of the molecular weight distribution of the methoxy end-capped polyethylene glycol-polylactic acid block copolymer (mPEG-PLA(2000/20000)-alanine-lysine) prepared in Example 14.
Fig. 23A and Fig.23B respectively show ¹H-NMR and ¹³C-NMR testing spectrums of the methoxy end-capped polyethylene glycol-polylactic acid block copolymer (mPEG-PLA(2000/20000)- alanine-lysine) prepared in Example 15.
Fig. 24 shows GPC testing spectrum of the molecular weight distribution of the methoxy end-capped polyethylene glycol-polylactic acid block copolymer (mPEG-PLA(2000/20000)-alanine-lysine) prepared in Example 15.
Fig. 25 shows the drug concentration in plasma-time curve in rats after subcutaneous injection of Fulvestrant microsphere Sample 1 (polylactic acid-glycolic acid copolymer is used as the main carrier material) in Example 46.
Fig. 26 shows the concentration in plasma-time curve in rats after subcutaneous injection of Fulvestrant microsphere Sample 2 (methoxy end-capped polyethylene glycol-polylactic acid block copolymer is used as the main carrier material) in Example 46.
Fig. 27 shows the in vitro drug release curve of Naproxen microsphere Sample 1 (polylactic acid-glycolic acid copolymer is used as the main carrier material) in Example 47.
Fig. 28 shows the in vitro drug release curve of Naproxen microsphere Sample 2 (methoxy end-capped polyethylene glycol-polylactic acid- alanine block copolymer is used as the main carrier material) in Example 47.
Fig. 29 shows the in vitro drug release curve of Carbamazepine microsphere Sample 1 (polylactic acid is used as the main carrier material) in Example 48.
Fig. 30 shows the in vitro drug release curve of Carbamazepine microsphere Sample 2 (methoxy end-capped polyethylene glycol-polylactic acid-succinic acid block copolymer is used as the main carrier material) in Example 48.
Fig.31 shows the drug release curves of the Carbamazepine microsphere prepared by compound carrier material and single carrier material in Example 49.

### BEST MODE FOR CARRYING OUT THE INVENTION

The present invention will be further illustrated in detail with reference to the following examples, but the invention is not limited to the following Examples.

In the following Examples, the sources of the drugs used, standards for and manufacturers of the reagents used, and models and manufacturers of the instruments etc. are shown in Tables 1-4.

**Table 1 Name. Quality Standards and Sources of Drugs**

| Name of Drugs | Standards | Sources |
|---|---|---|
| Rifampin | CP2005 | Shaanxi Weinan Huaren Pharmaceutical Co., Ltd., China |
| Amlodipine | Enterprise Standard | Xi'an Libang Pharmaceutical Co., Ltd., China |
| Stavudine | H10920013 | Wuhan Yuancheng Technology Development Co., Ltd., China |
| Azithromycin | CP2005 | Xi'an Lijun Pharmaceutical Co., Ltd., China |
| Naproxen | CP2005 | Chongqing Southwest No. 2 Pharmaceutical Factory Co., Ltd., China |
| Ropinirole | USP28 | Xi'an Libang Pharmaceutical Co., Ltd., China |
| Paroxetine | USP26 | Zhejiang Linhai Jinqiao Chemical Co., Ltd., China |
| Cinnarizine | CP2005 | Wuhan Weishunda Technology Development Co., Ltd., China |
| Lovastatin | USP26 | Wuhan Wuchang Yuancheng Technology Development Co., Ltd., China |
| Fulvestrant | USP28 | Xi'an Libang Pharmaceutical Co., Ltd., China |
| Orlistat | EP5.0 | Wuhan Wuchang Yuancheng Technology Development Co., Ltd., China |
| Fluconazol | CP2005 | Changzhou Lanling Pharmaceutical Co., Ltd., China |
| Tramadol hydrochloride | CP2005 | Shandong Xinhua Pharmaceutical Co., Ltd., China |
| Carbamazepine | CP2005 | Changzhou Yabang Pharmaceutical Co., Ltd., China |
| Clarithromycin | CP2005 | Xi'an Lijun Pharmaceutical Co., Ltd., China |
| Meloxicam, | CP2005 | Ningbo DHY Pharmaceutical Co., Ltd., China |
| Probenecid | CP2005 | Shanghai Sine Pharmaceutical Co., Ltd., China |
| Thioridazine hydrochloride | CP2005 | Hunan Dongting Pharmaceutical Co. Ltd., China |
| Timiperone | EP5.0 | Xi'an Libang Pharmaceutical Co., Ltd., China |
| Chlorprothixene | CP2005 | Changzhou Yabang Pharmaceutical Co., Ltd., China |
| Risperidone | EP5.0 | Zhejiang Huahai Pharmaceuticals Co., Ltd., China |
| Alprazolam | CP2005 | Xi'an Libang Pharmaceutical Co., Ltd., China |
| Trazodone | USP25 | Wuhan Weishunda Technology Development Co., Ltd., China |
| Famciclovir | CP2005 | Zhejiang Hisun Pharmaceutical Co. Ltd., China |
| Amitriptyline hydrochloride | CP2005 | Shanghai Sine Pharmaceutical Co., Ltd., China |
| Nimodipine | CP2005 | Shandong Xinhua Pharmaceutical Co., Ltd., China |
| Donepezil | EP5.0 | Beijing Merson Pharmaceutical Technology Development Co., China |
| Captopril | CP2005 | Chongqing Southwest No. 2 Pharmaceutical Factory Co., Ltd., China |
| Norethindrone | CP2005 | Zhejiang Xianju Pharmaceutical Co., Ltd., China |
| Gliclazide | CP2005 | Zhejiang Jiuzhou Pharmaceutical Co., Ltd., China |
| Melphalan | EP5.0 | Xi'an Libang Pharmaceutical Co., Ltd., China |

**Table 2 Grades and Sources of Excipients**

| Name of Excipients | Standards | Sources |
|---|---|---|
| methoxy end-capped polyethylene glycol | enterprise standard | Xi'an Libang Medical Technology Co., Ltd., China |
| methoxy end-capped polyethylene glycol-polylactic acid block copolymer and derivative thereof | | |
| polylactic acid-glycolic acid copolymer (50/50, Mw=40000) | enterprise standard | Chengdu Organic Chengdu Organic Chemicals Co. Ltd., China |
| polylactic acid-glycolic acid copolymer (75/25, Mw= 40000) | | |
| polylactic acid (Mw= 40000) | | |

**Table 3 Name of Reagents, Standards and Sources thereof**

| Name of Reagents | Grades | Standards | Sources |
|---|---|---|---|
| dichloromethane | analytically pure | 500m | Tianjm Kennel Chemical Reagent Co., Ltd., China |
| polyvinyl alcohol | chemically pure | 500g | |
| glycerin monostearate | chemically pure | 100g | |
| hexadecyl trimethyl ammonium bromide | analytically pure | 100g | |
| methylcellulose | officinal | 500g | Shandong Ruitai Chemicals Co. Shandong Ruitai Chemicals Co. Ltd., China |
| hydroxypropyl methyl cellulose | | 500g | |
| citric acid analytically | pure | 500g | Tianjin No. 1 Chemical Reagent |
| disodium hydrogen phosphate | analytically pure | 500g | Xi'an Chemical Reagent Factory, China |
| chloroform | analytically pure | 500ml | |
| sodium dodecyl sulfate | analytically pure | 500g | |
| potassium dihydrogen phosphate | analytically pure | 500g | |
| phosphoric acid | analytically pure | 500ml | |
| glacial acetic acid | analytically pure | 500ml | |
| silica gel | analytically pure | 500g | |
| anhydrous ethanol | analytically pure | 500ml | |
| ethyl acetate | analytically pure | 500m | |
| Polyisobutyl ester | chemically pure | 250g | Linyi Hengyuan Plastic Auxiliary Co. Ltd., China |
| dimethyl phthalate | | 100g | |
| diethyl phthalate | | 100g | |
| dibutyl sebacate | | 100g | |
| triethyl citrate | | 500m | |
| Tween-80 | enterprise standard | 500g | Croda Inc., Britain |
| Span-85 | | 500g | |
| sodium oleate | officinal | 100g | Xi'an Libang Pharmaceutical |
| cholesterol | | 100g | Co., Ltd., China |
| D,L-lactide | chemically pure | 100g | Shandong Medical Instruments Institute, China |
| L-lactide | chemically pure | -- | Xi'an Libang Medical Technology Co., Ltd., China |
| stannous octoate | analytically pure | 500ml | Sinopharm Chemical Reagent Co. Ltd. |
| acacia | food grade | 500g | Tai'an Lida Gum Industry Co., Ltd., China |
| trehalose | food grade | 1.0kg | Sinozyme Biotechnology Co., Ltd., Haining, China |
| petroleum ether | analytically pure | 500ml | Shantou Guanghua Chemical Factory Co., Ltd., China |
| silicone oil | chemically pure | 500ml | Jinan Longcheng Organosilicon Co. Ltd., China |
| anhydrous ethyl ether | chemically pure | 500ml | Tianjin Chemical Reagent Co., Ltd., China |
| cyclohexane | analytically pure | 500ml | Tianjin Dengfeng Chemical Co. Ltd., China |
| saccharose | food grade | 500g | Xi'an confectionery Co., Ltd., China |
| acetonitrile | chromatographic pure | 4L | Tedia Company Inc., US |
| methanol | | | |

**Table 4 Names, Types and Manufacturers of Instruments and Equipments**

| Name of Instruments and Equipments | Types | Manufacturers |
|---|---|---|
| High Pressure Homogenizer | NS1001L2K | GEA Niro Soavi S.p.A., Italy |
| Microjet Pump | 110L | Newton Masschusetts Co., US |
| High Shear Mulser | BME-100L | Shanghai Weiyu Mechano-electronic Mechano-electronic Manufacturing Co., Ltd., China |
| High Shear Mulser | C1Y500 | |
| Lab Spray Dryer | L-117 | Beijing Laiheng Science and Trading Co. Ltd., China |
| Lab Lyophilizer | R404A-R508 | Martin Christ GmbH, Germany |
| Constant Temperature Magnetic Stirrer | DF-101S | Shaanxi Taikang Biotechnology Co. Ltd., China |
| Heat Collection-Constant Temperature Type Magnetic Stirrer | DF-101T | Gongyi Yingyu Yuhua Instrument Plant, China |
| Vacuum Drying Oven | ZKAB-35 | Beijing Huabolian Medical |
| Drying Oven | CS101-1FB | Chongqing Hengda Instrument Plant, China |
| Water Vacuum Pump | SHB-III | Taikang Scientific Teaching Instruments Factory, Henan Province, China |
| Oil Vacuum Pump | Y90S-4 | Wendeng Mechanical and Electric Equipment Factory Co. Ltd., China |
| Analytical Balance | One Ten-Thousandth | Beijing Sartorius Balance Co., Ltd., China |
| High Performance Liquid Chromatograph | Type 2695 | Waters Corp., US |
| High Performance Liquid Chromatograph | UV-2075 UV Detector, PU-2089 injection pump, AS-2055 Autosampler | JASCO Inc., Japan |
| Visible-Ultraviolet Spectrophotometer | UV-7502PC | Shanghai Xinmao Instrument Co., Ltd., China |
| Water-bathing Constant Temperature Oscillator | SHA-A | Jintan Zhengji Instruments Co. Ltd., China |
| Microscope | Nikon SE | Nikon Corporation, Japan |

The following Examples 1-14 exemplify the preparation of the representative methoxy end-capped polyethylene glycol-polylactic acid block copolymer and derivative thereof of the present invention.

Reagent: D, L-lactide, L-lactide, methoxy end-capped polyethylene glycol (mPEG), stannous octoate, dichloromethane, anhydrous ethyl ether, anhydrous ethanol and ethyl acetate.

Instruments: High Performance Liquid Chromatograph (Waters 2695, US Waters Corp.); Electronic Analytical Balance (Beijing Sartorius Balance Co., Ltd., China); ZKAB-35 Vacuum Drying Oven; Constant Temperature Drying Oven; Desiccator; SHB-III Water Circulation Vacuum Pump; Y90S-4 Oil Vacuum Pump; DF-101S Constant Temperature Magnetic Stirrer etc..

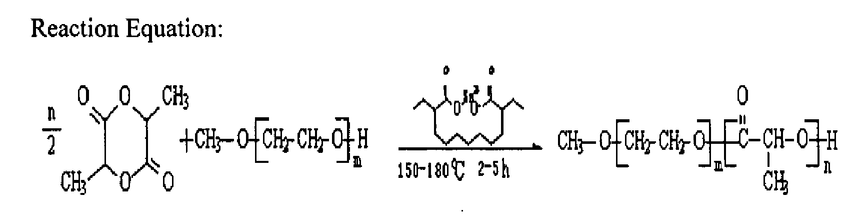

### Example 1:

### Preparation of methoxy end-capped polyethylene glycol-polylactic acid block copolymer (mPEG-PLA, 10000/10000)

Rate of charge : 4g of D, L- lactide, 4g of methoxy end-capped polyethylene glycol (mPEG, Mw=10000), 0.16g of stannous octoate

### Operation:

D, L- lactide, and mPEG are added into a flask, and then stannous octoate is dropped to form a mixture. The flask is sealed with a plug and vacuumized. Then the mixture is heated to 80°C, dewatered under vacuum degree≤200pa for 30min. Keep the pressure≤200pa, and heat up the mixture to 120°C rapidly with a temperature rising rate of 50°C/min. After the vacuum is shut off (the flask is still in sealed state), the mixture is continued to be heated up to 170°C, and reacted for 2h under 10rpm of mechanical agitation. After finishing the reaction, the reaction product is cooled to room temperature, into which suitable amount of dichloromethane is added to dissolve the product, and then placed overnight. On the next day, the resulting solution is dropped into about tenfold volume of ethyl ether to precipitate. After filtration, the filter cake is collected, vacuum dried under 40°C to obtain the product. The weight of the product is about 6g, and the yield is about 75%.

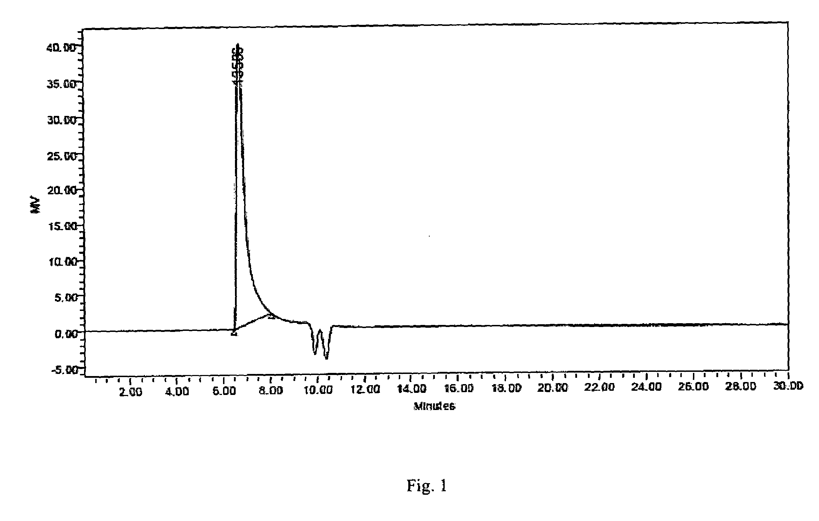

Testing result: the distribution result of the molecular weight determined by GPC is shown in Fig. 1 and Table 5.

**Table 5 Testing result of the molecular weight distribution of mPEG-PLA (10000/10000)**

| Batch number | Sample name | Mn | Mw | Mp | Mz | P.D. |
|---|---|---|---|---|---|---|
| 20070502 | mPEG-PLA (10000/10000) | 7671 | 10537 | 13586 | 12267 | 1.374 |

### Example 2:

### Preparation of methoxy end-capped polyethylene glycol-polylactic acid block copolymer (mPEG-PLA, 5000/8000)

Rate of charge : 9g of D, L- lactide, 5g of mPEG (Mw=5000), 1g of stannous octoate

### Operation:

D, L- lactide, and mPEG are added into a flask, and then stannous octoate is dropped to form a mixture. The flask is sealed with a plug and vacuumized. Then the mixture is heated to 60°C, dewatered under vacuum degrees≤150pa for 30min. Keep the pressure≤150pa, and heat up the mixture to 110°C rapidly with a temperature rising rate of 50°C/min. After the vacuum is shut off (the flask is still in sealed state), the mixture is continued to be heated up to 150°C, and reacted for 4h under 10rpm of mechanical agitation. After finishing the reaction, the reaction product is cooled to room temperature, into which suitable amount of dichloromethane is added to dissolve the product, and then placed overnight. On the next day, the resulting solution is dropped into about tenfold volume of ethyl ether to precipitate. After filtration, the filter cake is collected, vacuum dried under 40°C to obtain the product. The weight of the product is about 12g, and the yield is about 85.7%.

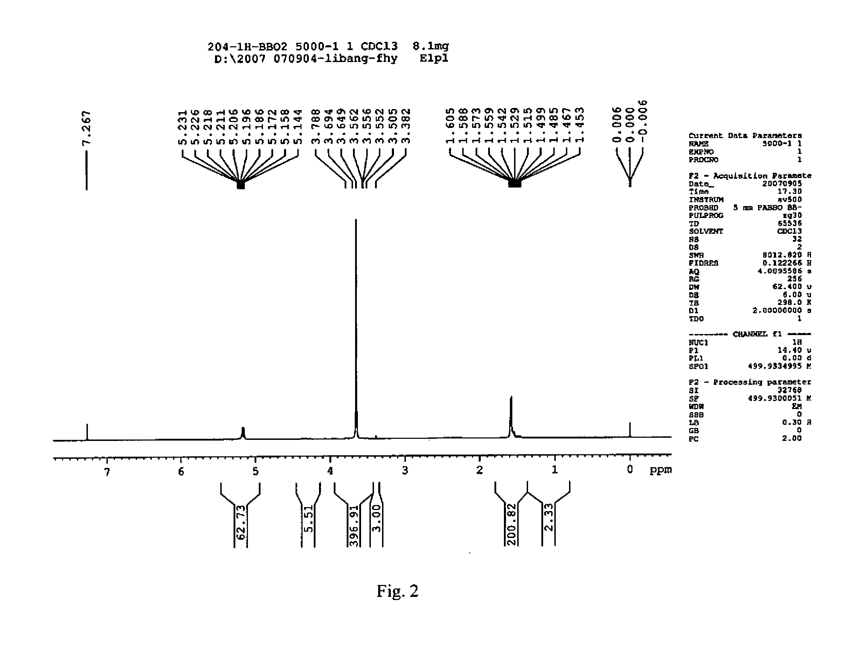

Testing result: H-NMR testing result is shown in Fig. 2 and Table 6.

**Table 6 H-NMR testing result of mPEG-PLA (5000/8000)**

| Batch number | Name | H-NMR | HLB |
|---|---|---|---|
| 20070507 | mPEG -PLA (5000/8000) | 2470-1520 | 13.3 |

### Example 3:

### Preparation of methoxy end-capped polyethylene glycol-polylactic acid block copolymer (mPEG-PLA, 2000/20000)

Rate of charge : 10.4g of D, L- lactide, 1.01g of mPEG (Mw=2000), 0.26g of stannous octoate

### Operation:

D, L- lactide, and mPEG are added into a flask, and then stannous octoate is dropped to form a mixture. The flask is sealed with a plug and vacuumized. Then the mixture is heated to 60°C, dewatered under vacuum degree≤180pa for 30min. Keep the pressure≤180pa, and heat up the mixture to 110°C rapidly with a temperature rising rate of 50°C/min. After the vacuum is shut off (the flask is still in sealed state), the mixture is continued to be heated up to 170°C, and reacted for 4h under 10rpm of mechanical agitation. After finishing the reaction, the reaction product is cooled to room temperature, into which suitable amount of dichloromethane is added to dissolve the product, and then placed overnight. On the next day, the solution is dropped into about tenfold volume of ethyl ether to precipitate. After filtration, the filter cake is collected, vacuum dried under 40°C to obtain the product. The weight of the product is about 6.6g, and the yield is about 59.8%.

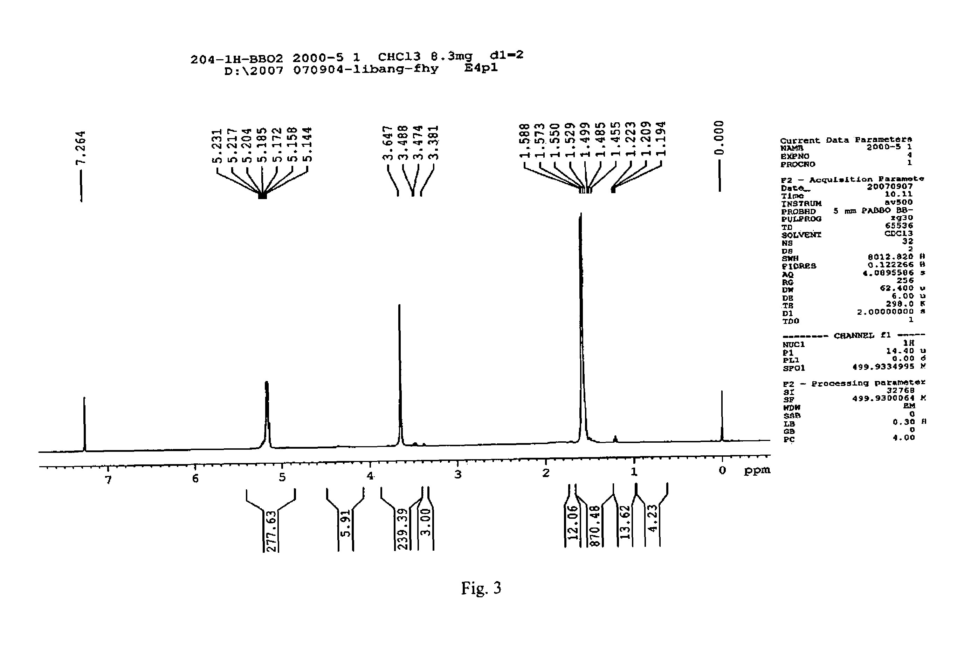

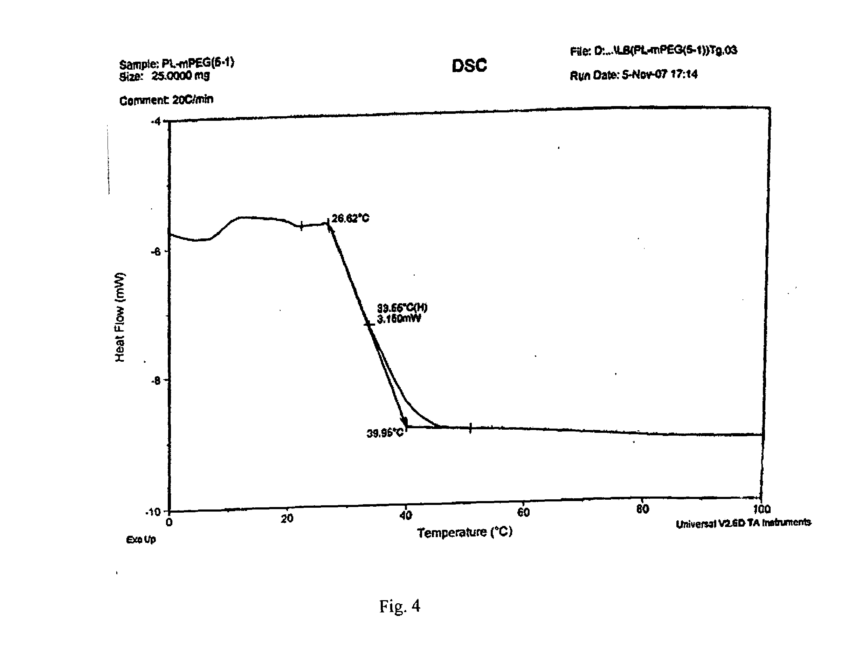

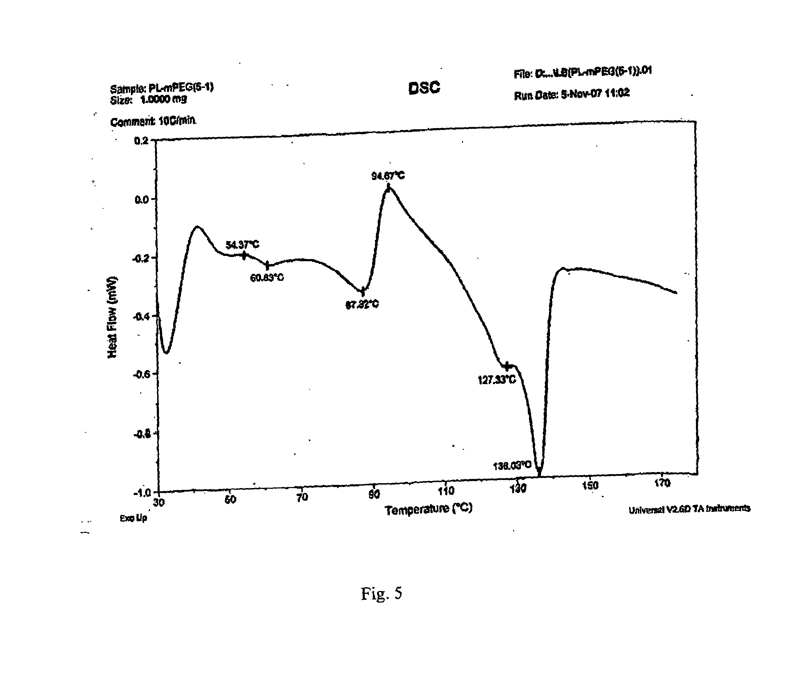

Testing result: H-NMR and DSC testing result is shown in Figs 3-5 and Tables 7-8.

**Table 7 H-NMR testing result of mPEG-PLA (2000/20000)**

| Batch number 20070508 | Name mPEG-PLA | H-NMR | HLB |
|---|---|---|---|
| | (2000/20000) | 2664-20000 | 3.49 |

**Table 8 DSC testing result of mPEG-PLA (2000/20000)**

| Batch number | Name | Tg(°C) | Tf(°C) |
|---|---|---|---|
| 20070508 | mPEG -PLA (2000/20000) | 33.55 | 54.37, 60.83, 87.32, 94.67, 127.33, 136.03 |

### Example 4:

### Preparation of methoxy end-capped polyethylene glycol-polylactic acid block copolymer (mPEG-PLA, 10000/10000)

Rate of charge : 4g of D, L- lactide, 4g of mPEG (Mw=10000), 0.16g of stannous octoate

### Operation:

D, L- lactide, and mPEG are added into a flask, and then stannous octoate is dropped to form a mixture. The flask is sealed with a plug and vacuumized. Then the mixture is heated to 80°C, dewatered under vacuum degree≤190pa for 30min. Keep the pressure≤180pa, and heat up the mixture to 120°C rapidly with a temperature rising rate of 50°C/min. After the vacuum is shut off (the flask is still in sealed state), the mixture is continued to be heated up to 170°C, and reacted for 2h under 10rpm of mechanical agitation. After finishing the reaction, the reaction product is cooled to room temperature, into which suitable amount of dichloromethane is added to dissolve the product, and then placed overnight. On the next day, the resulting solution is dropped into about tenfold volume of ethyl ether to precipitate. After filtration, the filter cake is collected, vacuum dried under 40°C to obtain the product. The weight of the product is about 6g, and the yield is about 75%.

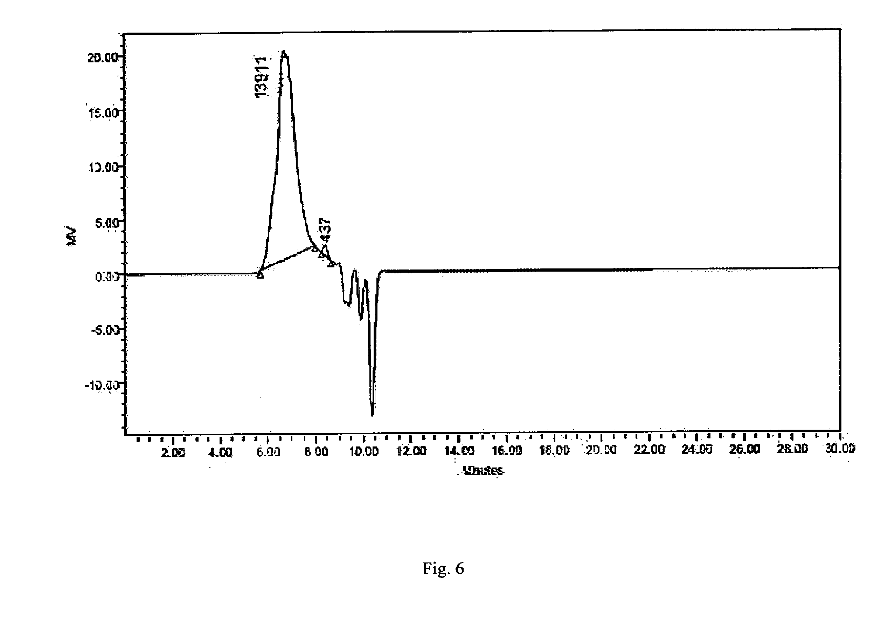

Testing result: GPC testing result of distribution of molecular weight is shown in Fig. 6 and Table 9.

**Table 9 GPC testing result of the distribution of molecular weight of mPEG-PLA (10000/10000)**

| Batch number | Name | Mn | Mw | Mp | Mz | P.D. |
|---|---|---|---|---|---|---|
| 20070522 | PLA-mPEG (10000/10000) | 7671 | 10537 | 13586 | 12267 | 1.374 |

### Example 5:

### Preparation of methoxy end-capped polyethylene glycol-polylactic acid block copolymer (mPEG-PLA, 20000/120000)

Rate of charge : 8g of D, L- lactide, 1.5g of mPEG ( Mw=20000), 0.06g of stannous octoate

### Operation:

D, L- lactide, and mPEG are added into a flask, and then stannous octoate is dropped to form a mixture. The flask is sealed with a plug and vacuumized. Then the mixture is heated to 80°C, dewatered under vacuum degree≤150pa for 30min. Keep the pressure≤150pa, and heat up the mixture to 120°C rapidly with a temperature rising rate of 50°C/min. After the vacuum is shut off (the flask is still in hermetically-sealed condition), the mixture is continued to be heated up to 170°C, and reacted for 4h under 10rpm of mechanical agitation. After finishing the reaction, the reaction product is cooled to room temperature, into which suitable amount of dichloromethane is added to dissolve the product, and then placed overnight. On the next day, the resulting solution is dropped into about tenfold volume of ethyl ether to precipitate. After filtration, the filter cake is collected, vacuum dried under 40°C to obtain the product. The weight of the product is about 7.6g, and the yield is about 80%.

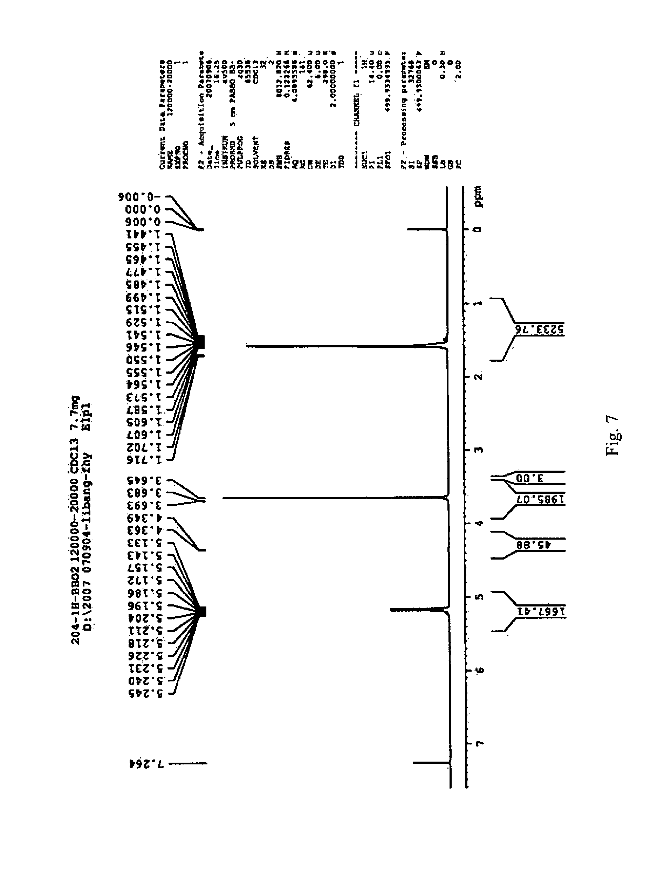

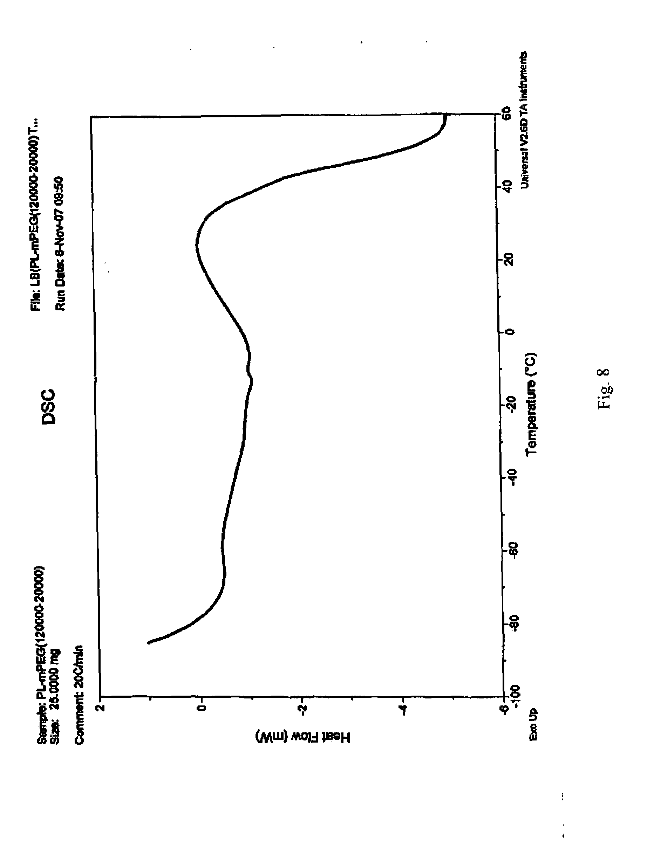

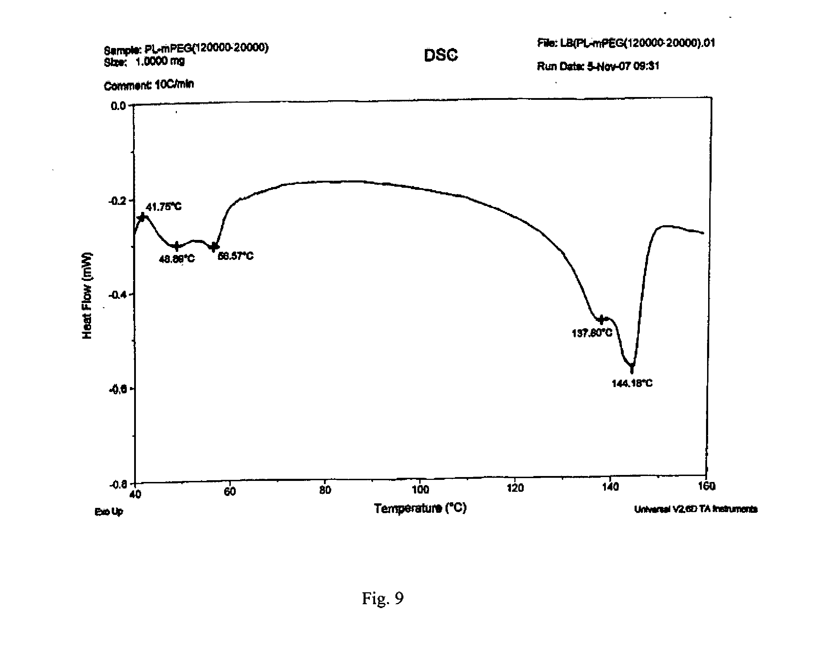

Testing result: H-NMR and DSC testing result is shown in Figs 7-9 and Tables 10-11.

**Table 10 H-NMR testing result of mPEG-PLA (20000/120000)**

| Batch number | Name | H-NMR | HLB |
|---|---|---|---|
| 20070523 | mPEG-PLA(20000/120000) | 21866-120000 | 4.588 |

**Table 11 DSC testing result of mPEG-PLA (20000/120000)**

| Batch number | Name | Tg(°C) | Tf(°C) |
|---|---|---|---|
| 20070523 | mPEG -PLA(20000/120000) | -- | 41.75, 48.89, 56.57, 137.80, 144.18 |

### Example 6:

### Preparation of methoxy end-capped polyethylene glycol-polylactic acid block copolymer (mPEG-PLA, 15000/55000)

Rate of charge : 25.5g of D, L- lactide, 7g of mPEG (Mw=15000), 0.28g of stannous octoate

### Operation:

D, L- lactide, and mPEG are added into a flask, and then stannous octoate is dropped to form a mixture. The flask is sealed with a plug and vacuumized. Then the mixture is heated to 70°C, dewatered under vacuum degree≤170pa for 30min. Keep the pressure≤150pa, and heat up the mixture to 110°C rapidly with a temperature rising rate of 50°C/min. After the vacuum is shut off (the flask is still in sealed state), the mixture is continued to be heated up to 150°C, and reacted for 4h under 10rpm of mechanical agitation. After finishing the reaction, the reaction product is cooled to room temperature, and into which suitable amount of dichloromethane is added to dissolve the product, and then placed overnight. On the next day, the resulting solution is dropped into about tenfold volume of ethyl ether to precipitate. After filtration, the filter cake is collected, vacuum dried under 40°C to obtain the product. The weight of the product is about 21 g, and the yield is about 64%.

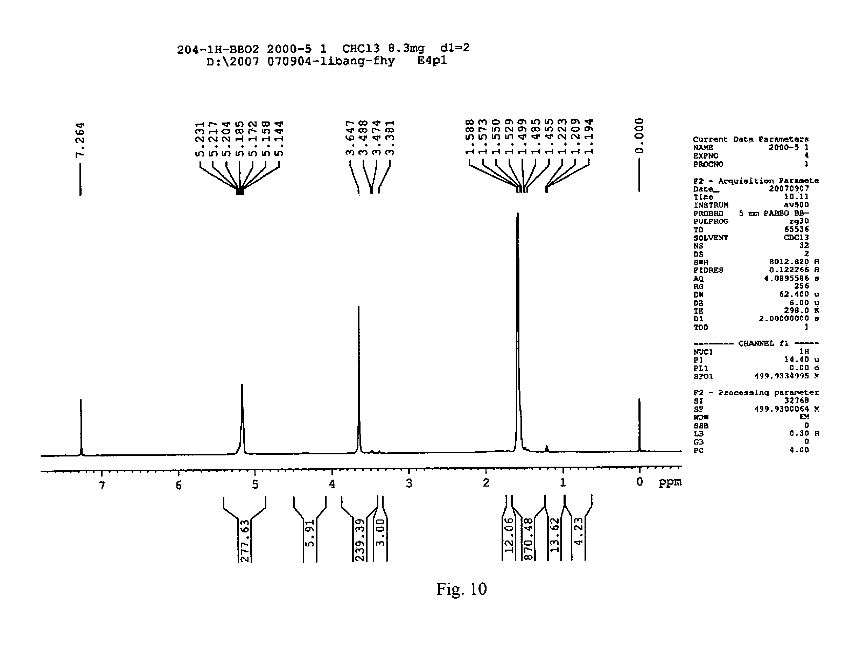

Testing result: H-NMR testing result is shown in Fig. 10 and Table 12.

**Table 12 H-NMR testing result of mPEG-PLA (15000/55000)**

| Batch number | Name | H-NMR | HLB |
|---|---|---|---|
| 20070620 | mPEG-PLA (15000/55000) | 17500-56200 | 11.2 |

### Example 7:

### Preparation of methoxy end-capped polyethylene glycol-polylactic acid block copolymer (mPEG-PLA, 2000/40000)

Rate of charge : 30g of D, L- lactide, 1.5g of mPEG (Mw=2000), 0.3g of stannous octoate

### Operation:

D, L- lactide, and mPEG are added into a flask, and then stannous octoate is dropped to form a mixture. The flask is sealed with a plug and vacuumized. Then the mixture is heated to 70°C, dewatered under vacuum degree≤200pa for 30min. Keep the pressure≤200pa, and heat up the mixture to 120°C rapidly with a temperature rising rate of 50°C/min. After the vacuum is shut off (the flask is still in sealed state), the mixture continues to be heated up to 150°C, and reacted for 4h under 10rpm of mechanical agitation. After finishing the reaction, the reaction product is cooled to room temperature, into which suitable amount of dichloromethane is added to dissolve the product, and then placed overnight. On the next day, the resulting solution is dropped into about tenfold volume of ethyl ether to precipitate. After filtration, the filter cake is collected, vacuum dried under 40°C to obtain the product. The weight of the product is about 27.5g, and the yield is about 87.5%.

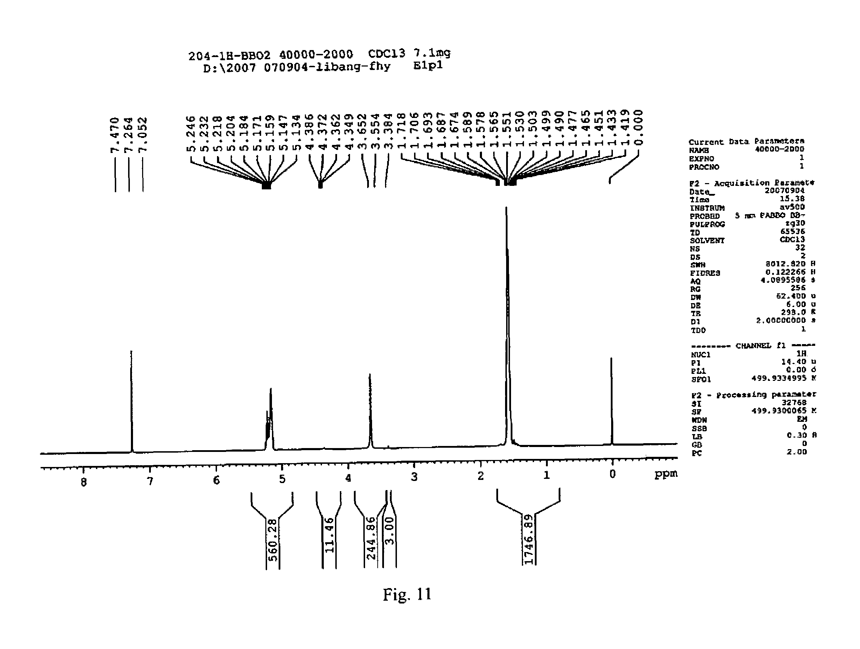

Testing result: H-NMR testing result is shown in Fig. 11 and Table 13.

**Table 13 H-NMR testing result of mPEG-PLA (2000/40000)**

| Batch number Name | mPEG-PLA | H-NMR | HLB |
|---|---|---|---|
| 20070711 | (2000/40000) | 2725-40300 | 1.92 |

### Example 8:

### Preparation of mPEG-PLA (2000/40000)- methyl

Rate of charge : 7.0g of mPEG-PLA (2000/40000) polymer, 1.0g of sodium hydride, 2ml of iodomethane

Operation: mPEG-PLA (2000/40000) polymer and sodium hydride are added into 80ml of dry tetrahydrofuran, and reacted with stirring at 25°C for 1.5h . Then iodomethane is added to the mixture, and reacted with stirring at 25°C for 24h. 1ml of anhydrous ethanol is added to the mixture and stirred for 30min. The solvent of the mixture is evaporated under reduced pressure, and then 20ml of dichloromethane is added to the mixture. After filtration, the filtrate is poured into 250ml of anhydrous ethyl ether. The obtained mixture is placed into a 50°C vacuum drying oven and dried for 1-2 days to obtain 6.1g of white powdery solid.

### Structural formula:

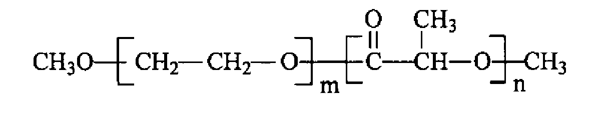

Testing result: As the product is insoluble in organic solvents such as tetrahydrofuran etc., it can not be detected.

### Example 9:

### Preparation of mPEG-PLA (2000/20000)- decane

Rate of charge : 7.0g of mPEG-PLA (2000/20000) polymer, 0.45g of sodium hydride, 2ml of bromodecane

Operation: mPEG-PLA (2000/20000) polymer and 0.45g of sodium hydride are added into 80ml of dry tetrahydrofuran, reacted under stirring at 25°C for 1.5h. Then bromodecane is added to the mixture, and reacted with stirring at 25°C for 42h. The mixture is evaporated to dryness, and then 80ml of dichloromethane is added. After filtration, the filtrate is evaporated. 20ml of dichloromethane is added again to dissolve the product, and then the solution is poured into 500ml of anhydrous ethyl ether. The obtained mixture is placed into a 50°C vacuum drying oven and dried for 1-2 days to obtain 4.88g of white powdery solid.

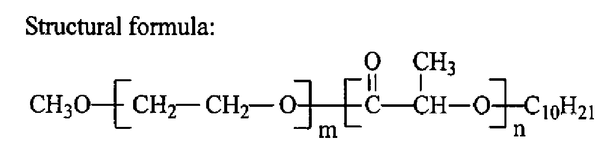

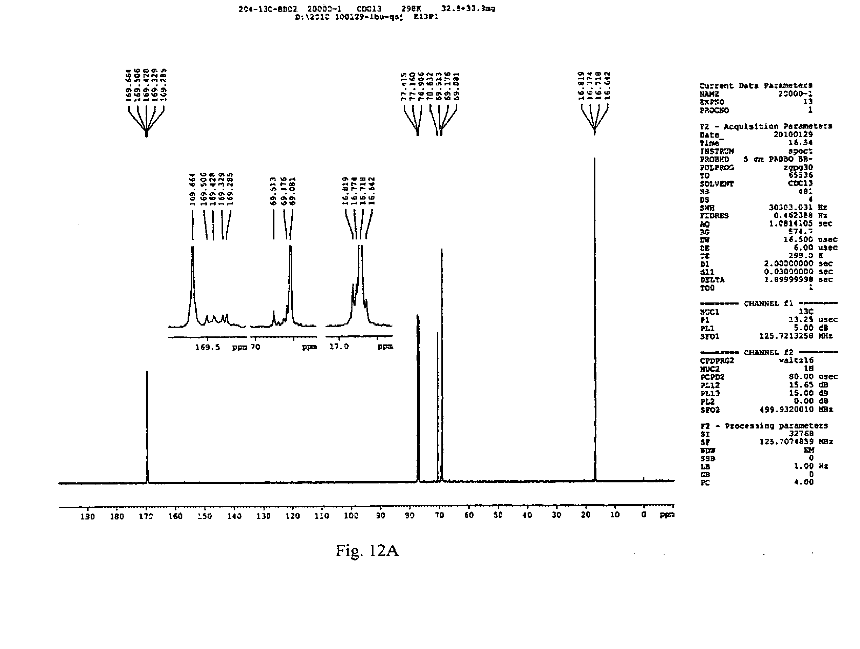

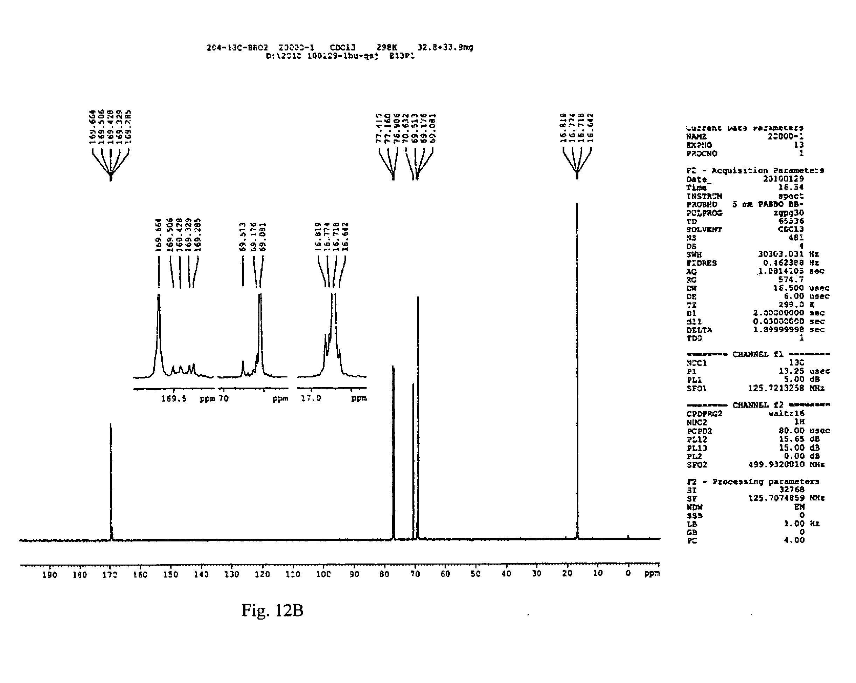

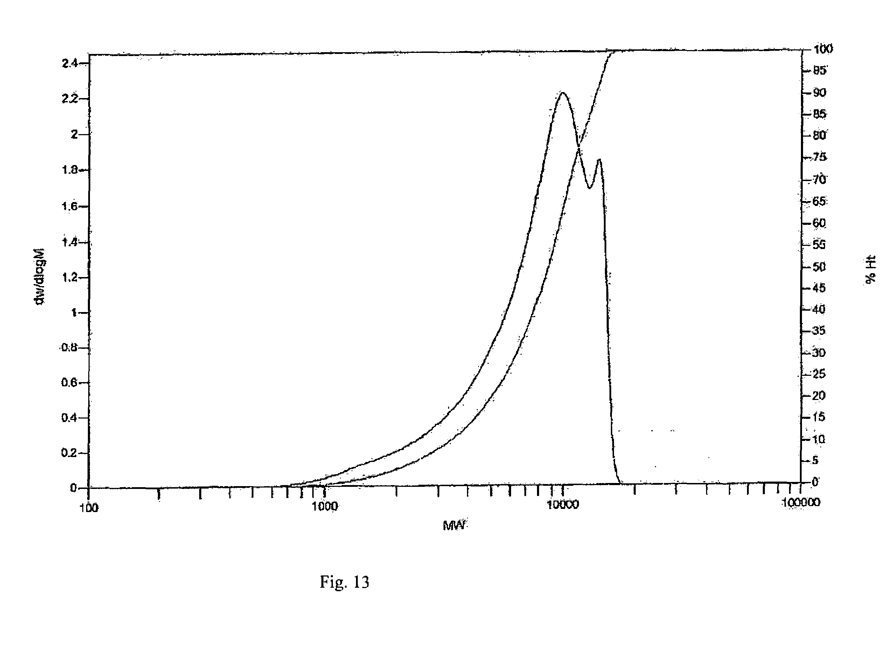

Testing result is shown in Table 14, ¹H-NMR and ¹³C-NMR spectrums are shown in Figs 12A and 12B, GPC testing result is shown in Fig. 13.

**Table 14 Testing result of the molecular weight distribution of mPEG-PLA (2000/20000) - decane**

| Name | ¹H-NMR | GPC | | | | |
|---|---|---|---|---|---|---|
| mPEG-PLA (2000/20000)-decane | 32545 | Mn | Mw | Mp | Mz | P.D. |
| | | 6056 | 8479 | 10043 | 10112 | 1.4001 |

### Example 10:

### Preparation of mPEG-PLA (2000/40000)- succinic acid

Rate of charge: 30.0g of mPEG-PLA (2000/40000) polymer, 1.0g of succinic anhydride, 0.1g of dicyclohexylcarbodiimide (DCC)

Operation: 30.05g of mPEG-PLA (2000/40000) polymer, 1.07g of succinic anhydride, 0.1g of DCC, and 130ml of dichloromethane are added into a 250 ml three-neck flask. Then 20ml of DMF is added, and stirred at 25°C for 24h. The solvent is evaporated to obtain a yellowish-brown viscous liquid. 80ml of dichloromethane is added to fully dissolve the viscous liquid, and then the resulting solution is poured into 1800ml of anhydrous ethyl ether with voilent agitation. 10ml of concentrated hydrochloric acid is then added to the mixture, and stirred for 1h. After filtration, the filter cake is dried naturally, dissolved in 80ml of dichloromethane, and then poured into 500ml of iced methanol. After filtration, the filter cake is washed with anhydrous ethyl ether for several times, then vacuum dried at 50°C for 2 days to obtain 5.4g of white floccus solid.

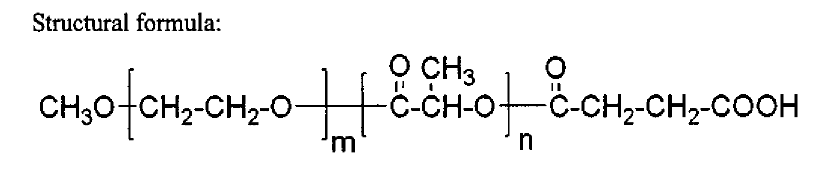

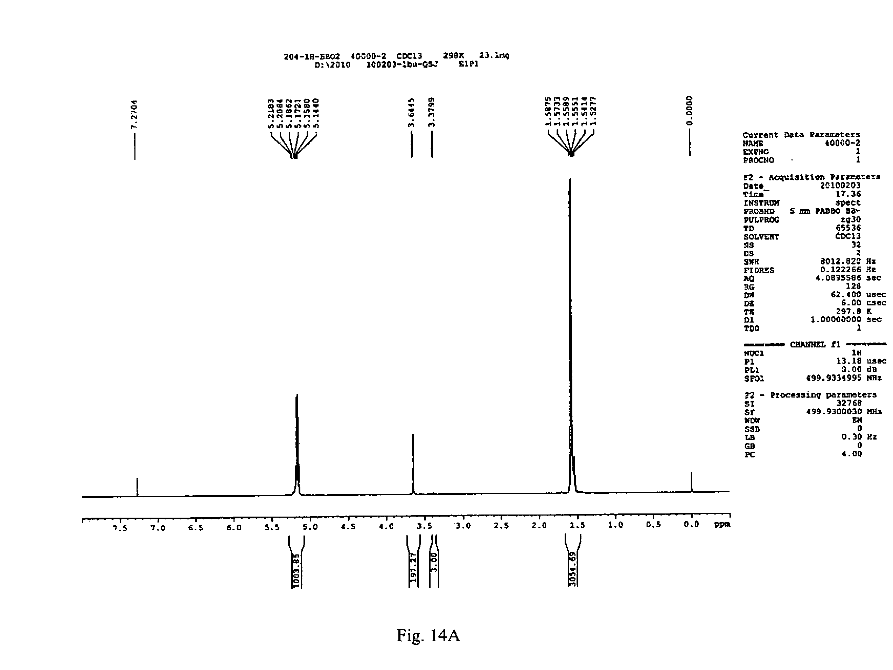

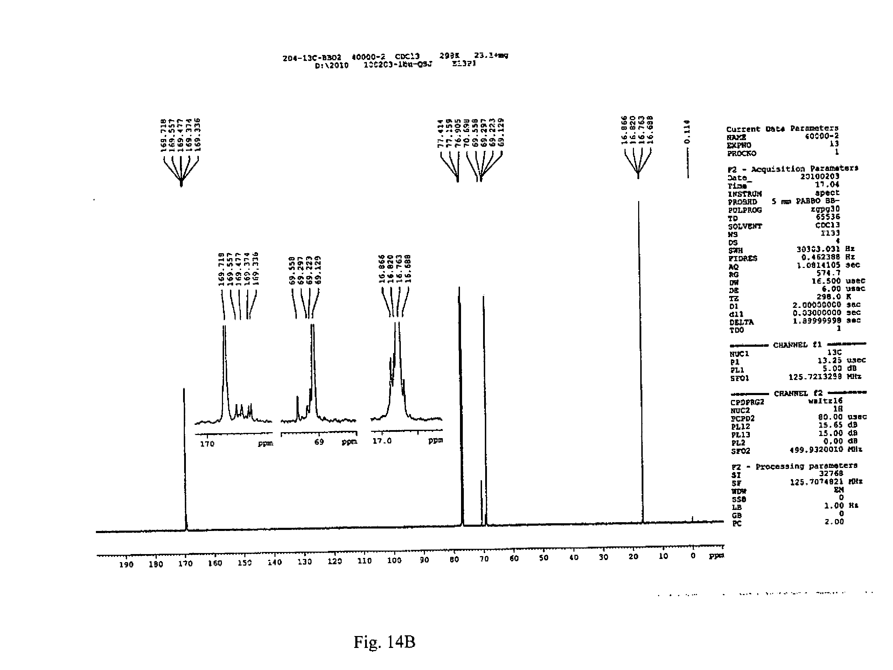

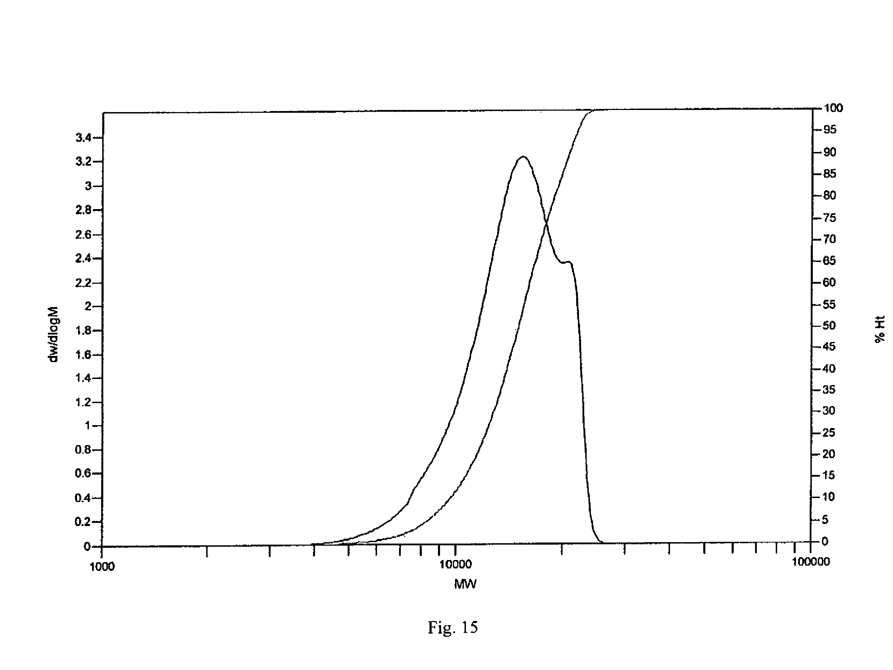

Testing result is shown in Table 15, ¹H-NMR and ¹³C-NMR spectrums are shown in Figs 14A and 14B, and GPC testing result is shown in Fig. 15.

**Table 15 Testing result of the molecular weight distribution of mPEG-PLA (2000/40000)-succinic acid**

| Name | 1H-NMR | GPC | | | | |
|---|---|---|---|---|---|---|
| mPEG-PLA (2000/20000)- succinic acid | 74277 | Mn | Mw | Mp | 16109 | P.D. |
| | | 13674 | 8198 | 15291 | 16109 | 1.0952 |

### Example 11:

### Preparation ofmPEG-PLA (2000/40000)- succinic acid- glutamic acid

Rate of charge : 10.0g of mPEG-PLA (2000/40000)- succinic acid, 0.17g HoBt, 0.1g of dicyclohexylcarbodiimide (DCC), 0.1g of L-glutamic acid

Operation: 10.0g of mPEG-PLA (2000/40000)- succinic acid and 70ml of dichloromethane are added into a 100 ml three-neck flask, stirred to dissolve. 0.09g of DCC and 0.17g of HoBt are added into the resulting solution, then 20ml of DMF is added. The mixture is cooled to 0°C, and stirred for 8h. The reaction mixture is filtered, then 0.10g of L- glutamic acid is added to the filtrate, stirred overnight, warmed up naturally, and reacted at 25°C for 10h. The reaction mixture is poured into 500ml of anhydrous ethyl ether, filtered and the filter cake is dried naturally, and then dissovled in 50ml of dichloromethane. Then the resulting solution is poured into 500ml of iced methanol. After filtration, the filter cake is washed with ethyl ether for several times, then vacuum dried at 50°C for 2 days to obtain 7.0g of grey white floccus solid.

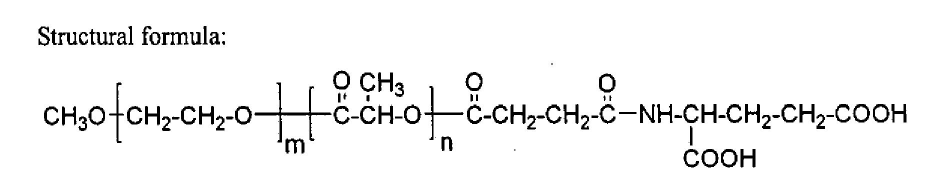

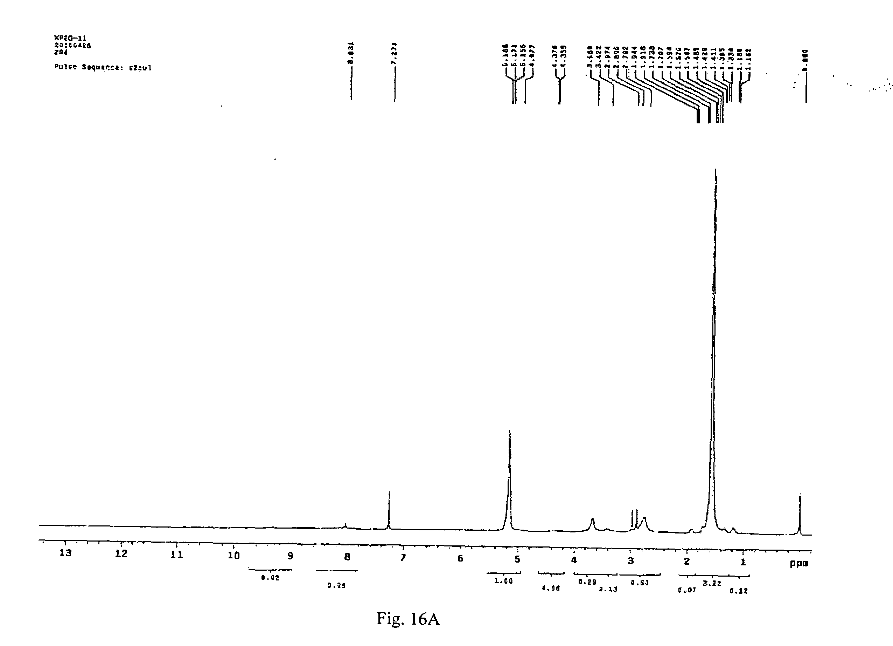

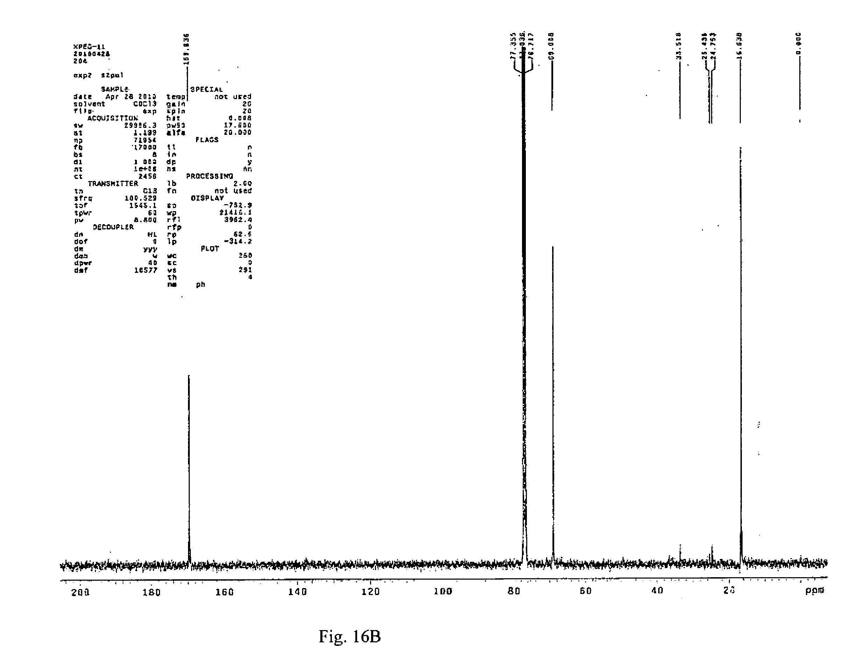

Testing result is shown in Table 16, ¹H-NMR and ¹³C-NMR spectrums are shown in Figs 16A and 16B.

**Table 16 Testing result of molecular weight of mPEG-PLA (2000/40000)- succinic acid-glutamic acid**

| Name | H-NMR |
|---|---|
| mPEG-PLA(2000/40000)-succinic acid-glutamic acid | 44912 |

### Example 12:

### Preparation of mPEG-PLA (2000/40000)- succinic acid- glutamic acid- glutamic acid₂

### Rate of charge :

first step: 2.0g of N-Boc-glutamic acid, 3.51g of DCC, 2.35g of HoBt, 2.57g of L-glutamic acid
second step: 1.07g of the product of the first step
third step: 10.05g of mPEG-PLA(2000/40000)-succinic acid-glutamic acid, 0.11g of DCC, 0.18g of HoBt, 0.3 1 g of the product of the second step

### Operation:

First step: To a 100ml three-neck flask, 2.0g of N-Boc-glutamic acid, 3.51g of DCC, 2.35g of HoBt are added, and then 70ml of tetrahydrofuran are added, stirred to dissolve, cooled to 0°C and stirred for 4h. The reaction mixture is filtered, and the filtrate is added into a solution of 2.57g of L-glutamic acid () in 120ml of tetrahydrofuran, stirred overnight, warmed up naturally, followed by reacting at 25°C for 10h. The reaction mixture is evaporated, to which 100ml of dichloromethane is added, then stirred for 0.5h, and filtered. The filtrate is washed twice respectively with saturated sodium bicarbonate solution and saturated citric acid solution, then washed once with saturated saline solution, dried, and evaporated. 5ml of anhydrous ethyl ether is added to the residue, followed by rubbing the wall of the bottle softly, solid is precipitated gradually, and cooled in refrigerator overnight, then filtered to obtain 2.3g of white solid (I).

Second step: 1.07g of solid (I) obtained in the first step is added into 20ml of dichloromethane and stirred at room temperature, and the solid is substantially insoluble. After 2ml of trifluoroacetic acid is added slowly, the solid is soon dissolved completely, and stirred for 30min. The resulting solution is evaporated, and into which 20ml of dichloromethane is added, stirred until totally dissolved, and evaporated. This procedure is repeated once again. 5ml of anhydrous ethyl ether is added to the distilling flask, and quickly followed by the appearance of white precipitate, cooled in the refrigerator overnight, and then filtered. The filter cage is then vacuum dried at 30°C for 2 days to obtain 0.85g of white solid (II).

Third step: To a 100ml three-neck flask, 10.05g of mPEG-PLA(2000/40000)-succinic acid-glutamic acid, 0.11 g of DCC and 0.18g of HoBt are added, followed by addition of 70ml of dichloromethane and 20ml of DMF. The mixture is stirred, then cooled in icewater bath overnight. The 0.31 g of above prepared solid (II) is added to the system at 0°C, followed by warming up to 25°C and reacted for 10h. The reaction mixture is poured into 500ml of anhydrous ethyl ether, filtered, and the filter cake is dried naturally, then dissolved in 50ml of dichloromethane. The resulting solution is poured into 500ml of iced methanol. After filtration, the filter cake is washed with anhydrous ethyl ether for several times, then vacuum dried at 50°C for 2 days to obtain 7.0g of grey white floccus solid.

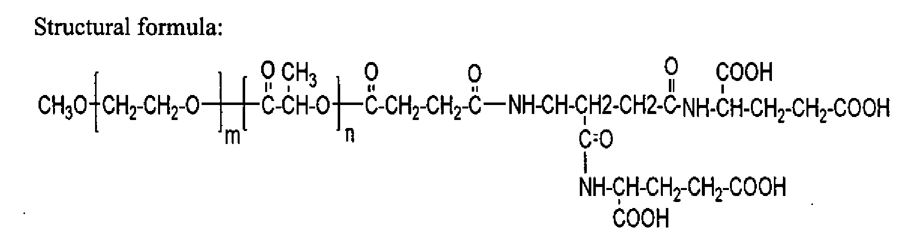

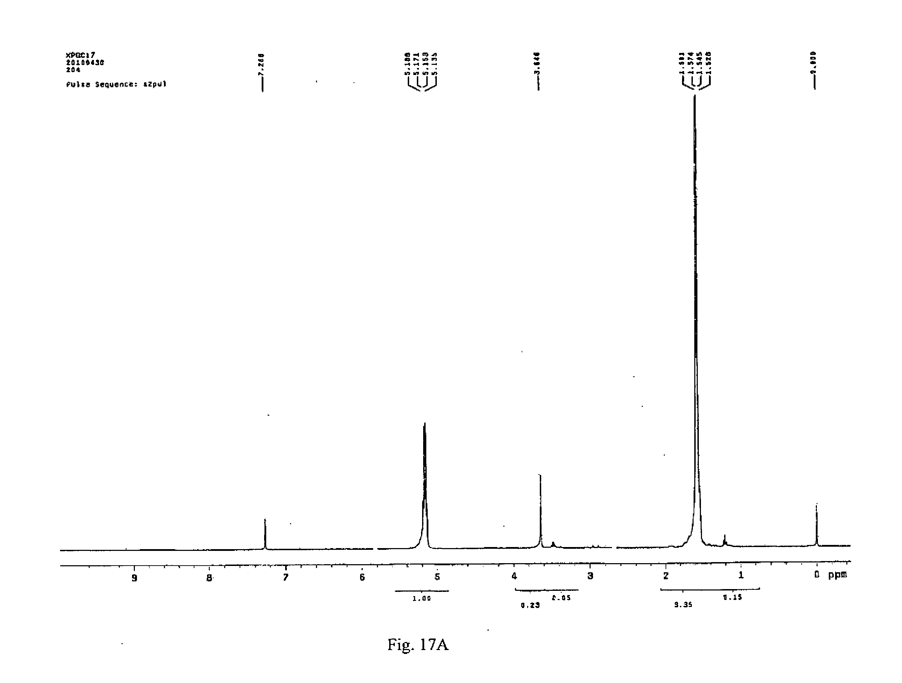

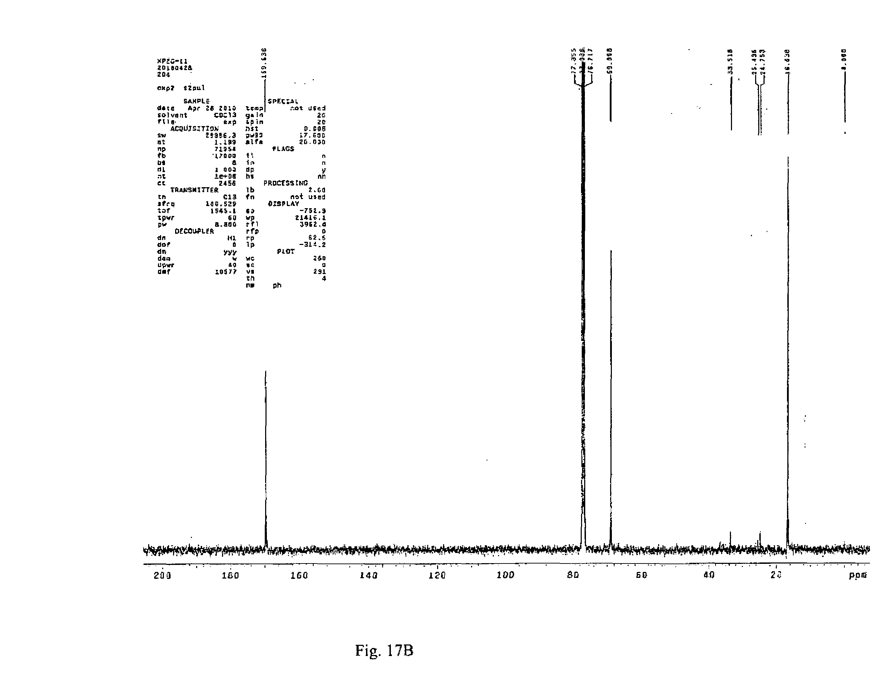

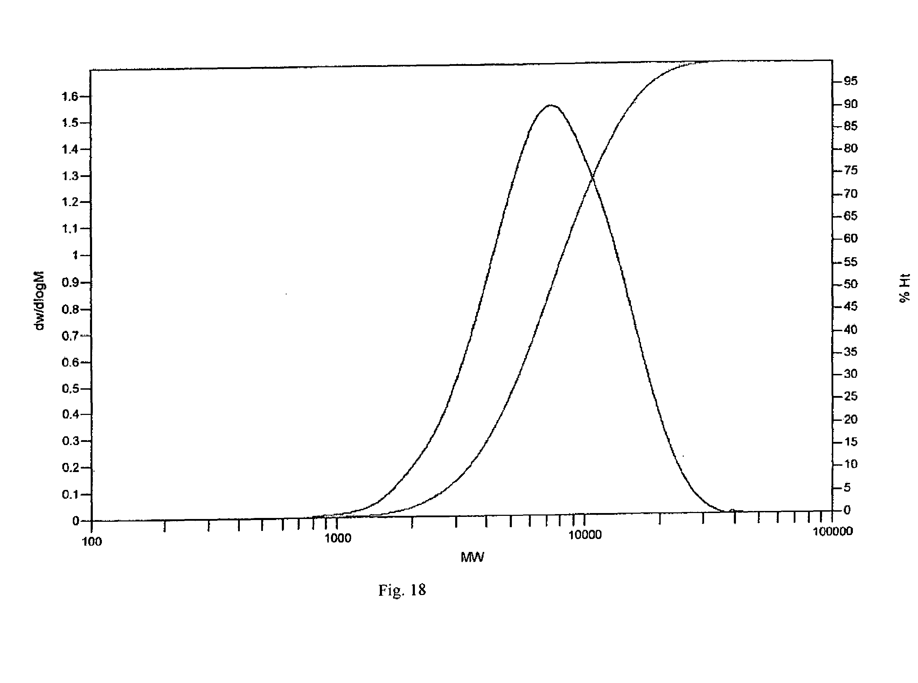

Testing result is shown in Table 17, ¹H-NMR and ¹³C-NMR spectrums are shown in Figs17A and 17B, GPC testing result is shown in Fig. 18.

**Table 17 Testing result of mPEG-PLA (2000/40000)-succinic acid-glutamic acid-glutamic acid₂**

| Name | ¹H-NMR | GPC | | | | |
|---|---|---|---|---|---|---|
| mPEG-PLA(2000/20000)- succinic acid -glutamic acid-glutamic acid₂ | 58035 | Mn | Mw | Mp | Mz | P.D. |
| | | 5734 | 8085 | 7624 | 10881 | 1.4100 |

### Example 13:

### Preparation of mPEG-PLA (2000/20000)- alanine

Rate of charge : 15.0g of mPEG-PLA (2000/20000) polymer, 0.93g of DCC, 0.05g of DMAP, 0.51g of N-Boc-Ala

Operation: To a 100 ml three-neck flask, 15.0g of mPEG-PLA (2000/20000) polymer, 0.93g of DCC, 0.05g of DMAP, and 0.51g of N-Boc-Ala are added, followd by addition of dichloromethane (70ml) and DMF (20ml). The mixture is stirred to dissolve and reacted at 25°C for 24h, then filtered. 5ml of trifluoroacetic acid is slowly added to the filtrate, the resulting mixture is stirred at room temperature for 1h. Part of the solvent is evaporated, then the mixture is poured into 750ml of anhydrous ethyl ether, filtered and the filter cake is dried naturally and then dissolved in 70ml of dichloromethane. Then the solution is poured into 500ml of iced methanol. After filtration, the filter cake is washed with ethyl ether for several times, and vacuum dried at 50°C for 2 days to obtain 12.85g of grey white floccus solid.

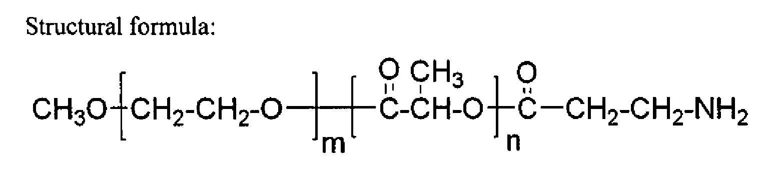

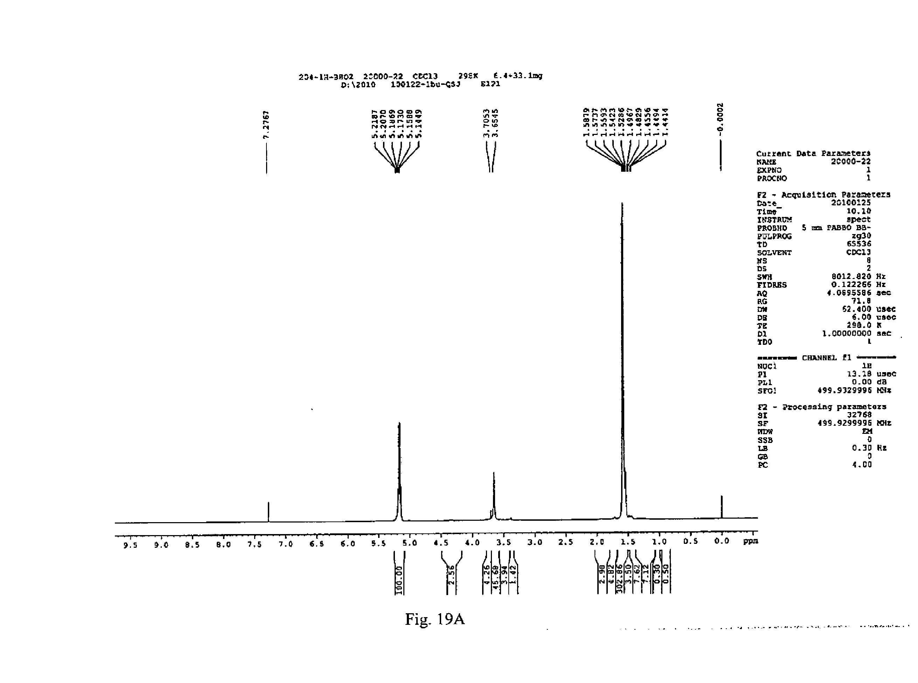

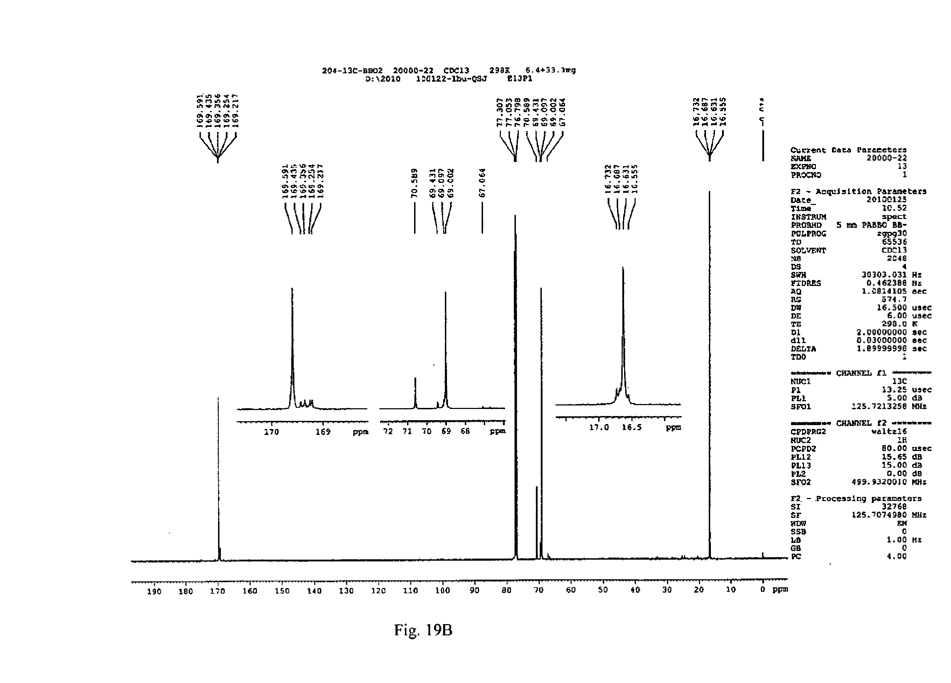

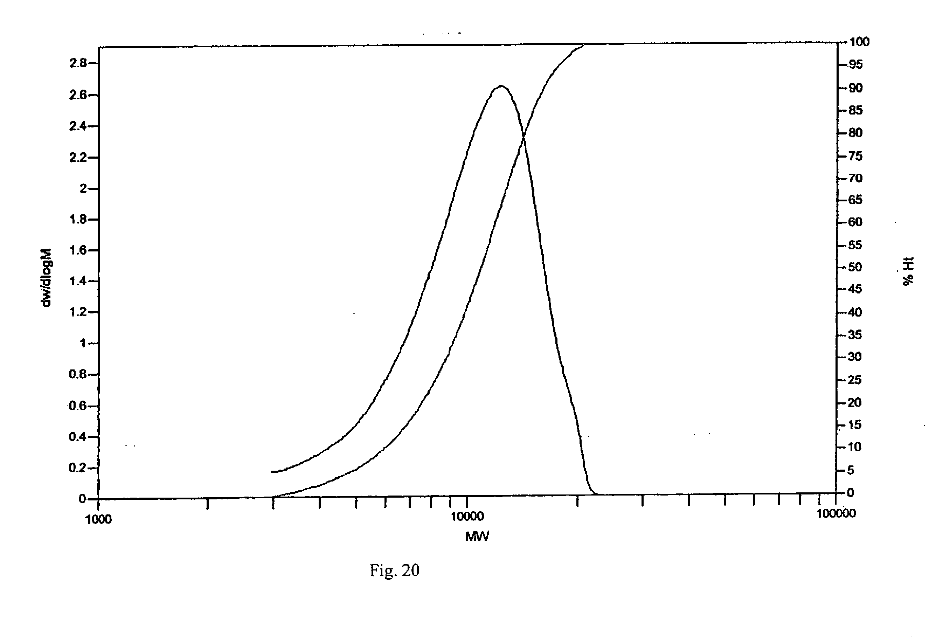

Testing result is shown in Table 18, ¹H-NMR and ¹³C-NMR spectrums are shown in Figs 19A and 19B, and GPC testing result is shown in Fig. 20.

**Table 18 Testing result of the molecular weight distribution of mPEG-PLA (2000/20000)-alanine**

| Name | ¹H-NMR | GPC | | | | |
|---|---|---|---|---|---|---|
| mPEG-PLA(2000/20000)- alanine | 17912 | Mn | Mw | Mp | Mz | P.D. |
| | | 9290 | 10850 | 12325 | 12187 | 1.1680 |

### Example 14:

### Preparation of mPEG-PLA (2000/20000)- alanine-lysine

Rate of charge : 6.0g of mPEG-PLA(2000/20000)-alanine, 0.12g of DCC, 0.16g of N-Boc-N-Fmoc-lysine, 0.09g of HoBt

Operation: To a 100 ml three-neck flask, 0.16g of N-Boc-N-Fmoc-lysine and 0.12g of DCC are added, followed by the addition of 20ml of dichloromethane. Then the mixture is stirred and cooled to 0°C, followed by the addition of 0.09g of HoBt. The resulting mixture is stirred at 0°C for 7h, and filtered. The filtrate is added into a solution of 6.0g of mPEG-PLA(2000/20000)-alanine in 30ml of dichloromethane, stirred under ice water bath for 1h, followed by stirring at 25°C overnight,

5ml of trifluoroacetic acid is added into the reaction mixture, and stirred at room temperature for 6h; then 15ml of triethylamine is added, and stirred at room temperature overnight.

The reaction mixture is poured into 500ml of anhydrous ethyl ether, filtered, and the filter cake is dried naturally, then dissolved in 50ml of dichloromethane. The resulting solution is poured into 500ml of iced methanol. After filtration, the filter cake is washed with ethyl ether for several times, then vacuum dried at 50°C for 2 days to obtain 5.2g of grey white floccus solid of.

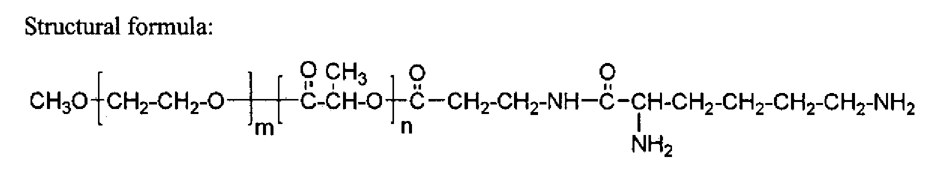

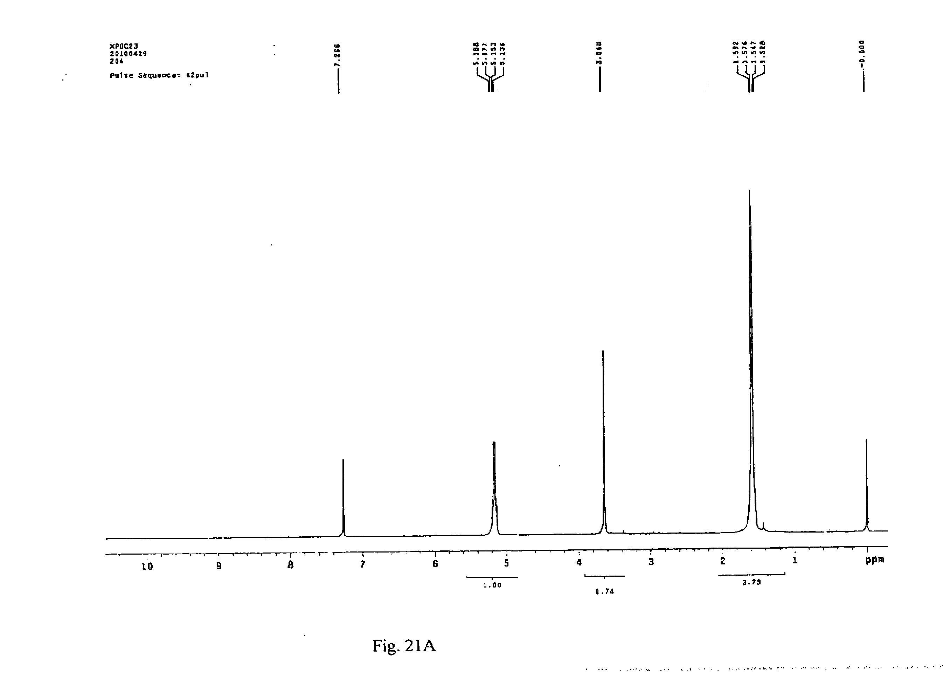

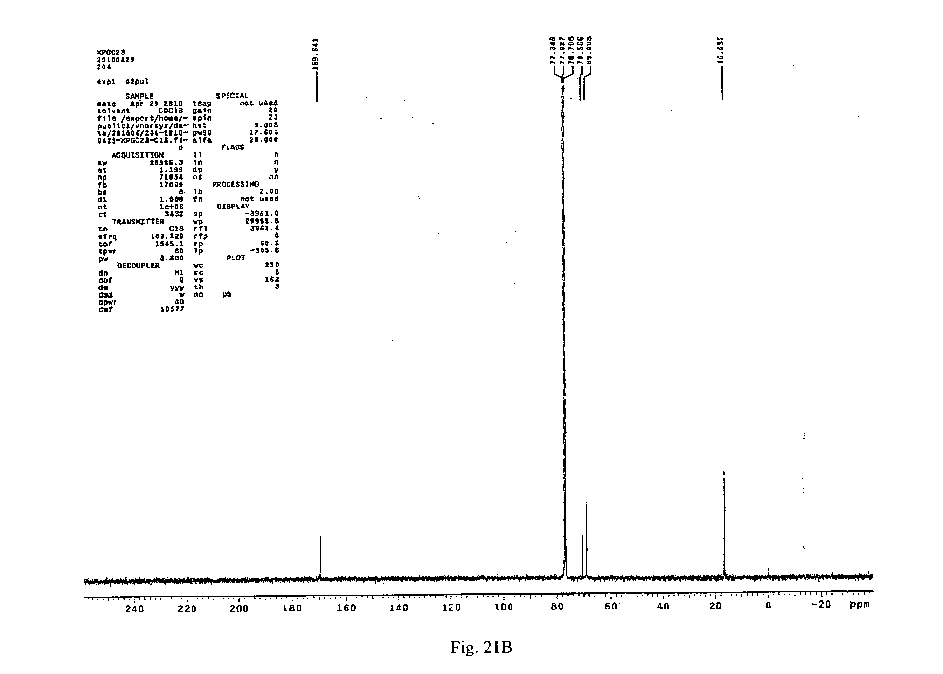

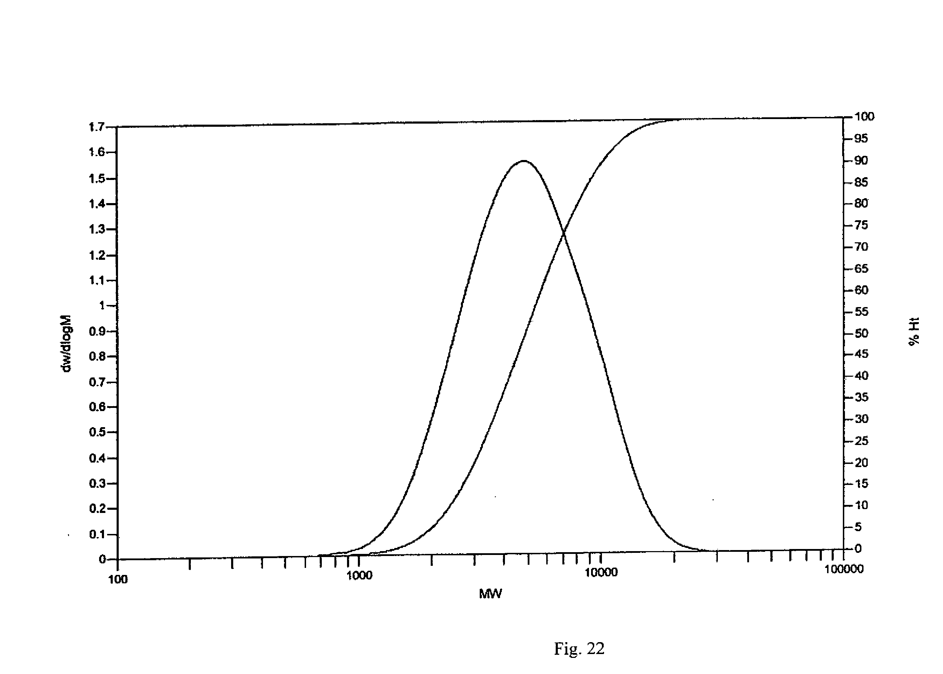

Testing result is shown in Table 19, ¹H-NMR and ¹³C-NMR spectrums are shown in Figs 21A and 21B, and GPC testing result is shown in Fig. 22.

**Table 19 Testing result of molecular weight of mPEG-PLA (2000/20000)-alanine-lysine**

| Name | ¹H-NMR | GPC | | | | |
|---|---|---|---|---|---|---|
| mPEG-PLA(2000/20000)- alanine-lysine | 20504 | Mn | Mw | Mp | Mz | P.D. |
| | | 3991 | 5426 | 4982 | 7230 | 1.3596 |

### Example 15:

### Preparation of mPEG-PLA (2000/20000)-alanine-lysine-lysine ₂

### Rate of charge :

First step: 2.81g ofN-Boc-N-Fmoc-lysine, 0.91g of HoBt, 1.42g of DCC, 0.65g of lysine Second step: 0.16g of the product of the first step, 0.07g of DCC, 0.06g of HoBt, 6.62g of mPEG-PLA(2000/20000)-alanine-lysine

### Operation:

First step: To a 100ml three-neck flask, 2.81g of N-Boc-N-Fmoc-lysine and 30ml of THF are added, followed by the addition of 0.91g of HoBt, cooled to 0°C and 1.42g of DCC is added, then stirred at 0°C for 6h, and filtered. The filtrate is added into a solution of 0.65g of lysine in 30ml of THF stirred at 0°C for 1h, and then stirred at 25°C for 10h. The reaction mixture is evaporated, followed by addition of 10ml of dichloromethane, and filtered. The filtrate is washed twice with saturated sodium carbonate solution, saturated citric acid solution, and saturated sodium chloride solution respectively, dried, and evaporated to near to dryness. 2ml of anhydrous ethyl ether is added to the residue, followed by shaking slowly, and solid is then appeared. The mixture is cooled in refrigerator overnight, filtered, and the filter cake is vacuum dried at 50°C to obtain 1.53g of white solid (I).

Second step: 0.16g of white solid (1) obtained from the above step, 0.07g of DCC (), 0.06g of HoBt and 20ml of dichloromethane are stirred for 8h in a ice water bath. After filtration, the filtrate is added into a solution of 6.62g of mPEG-PLA (2000/20000)-alanine-lysine in 50ml of dichloromethane, followed by stirring at 25°C overnight.

5ml of trifluoroacetic acid is added into the reaction mixture, and stirred at room temperature for 2h; then 15ml of triethylamine is added, and stirred at room temperature overnight.

The reaction mixture is poured into 500ml of anhydrous ethyl ether, filtered, and the filter cake is dried naturally and then dissolved in 50ml of dichloromethane. The resulting solution is poured into 500ml of iced methanol. After filtration, the filter cake is washed with ethyl ether for several times, then vacuum dried at 50°C for 2 days to obtain 5.6g of grey white floccus solid.

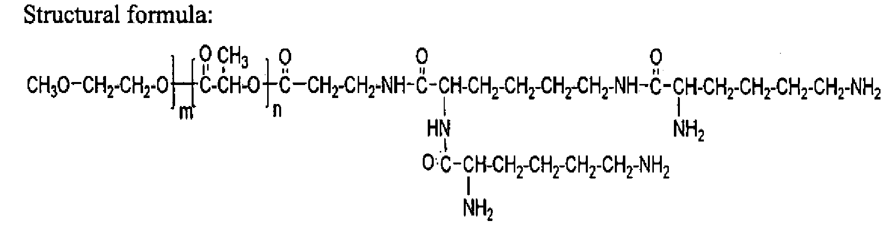

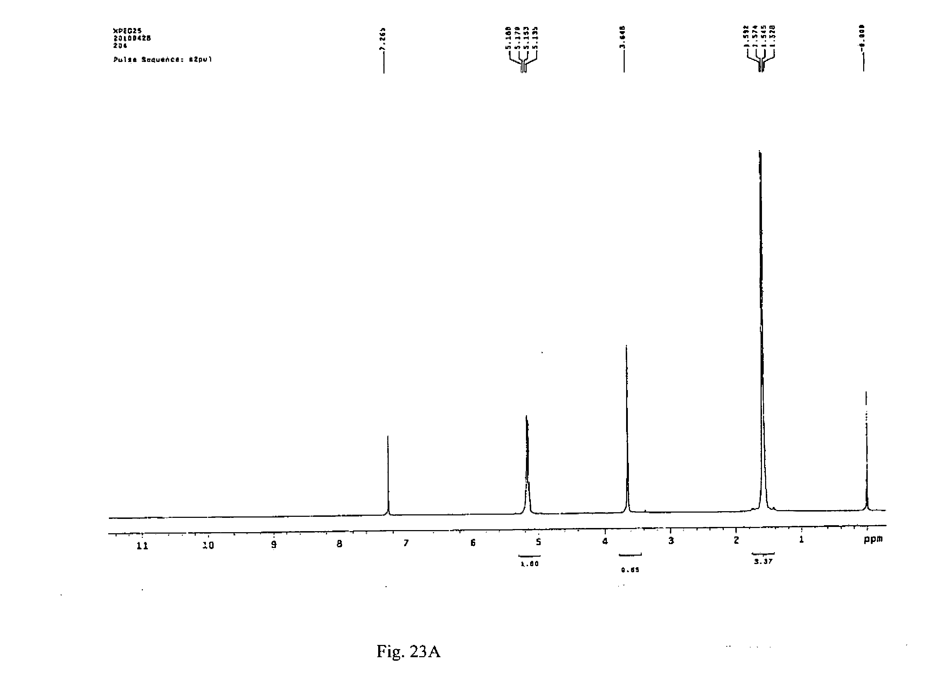

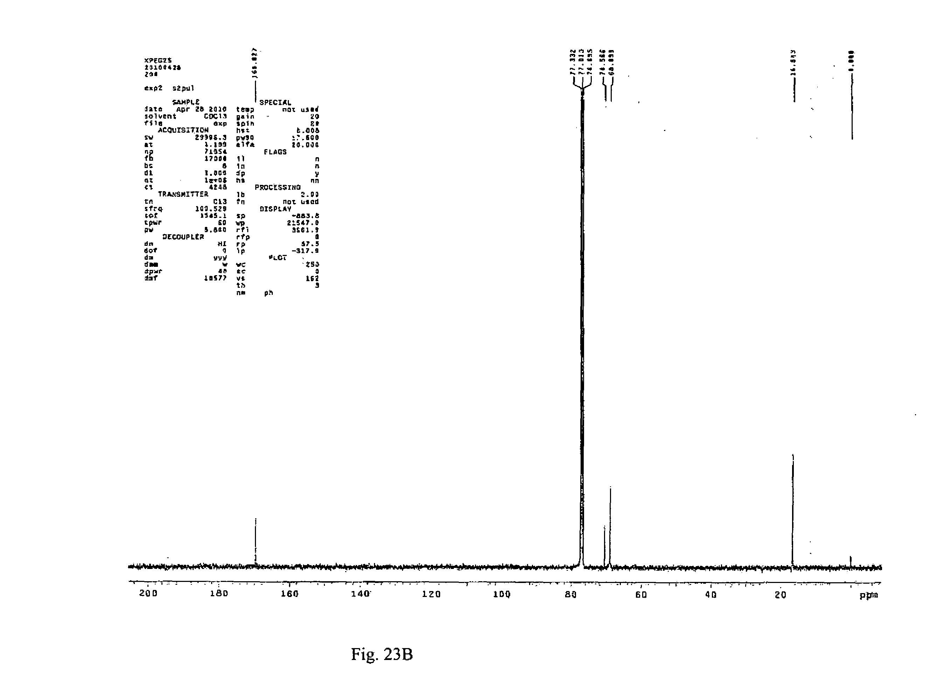

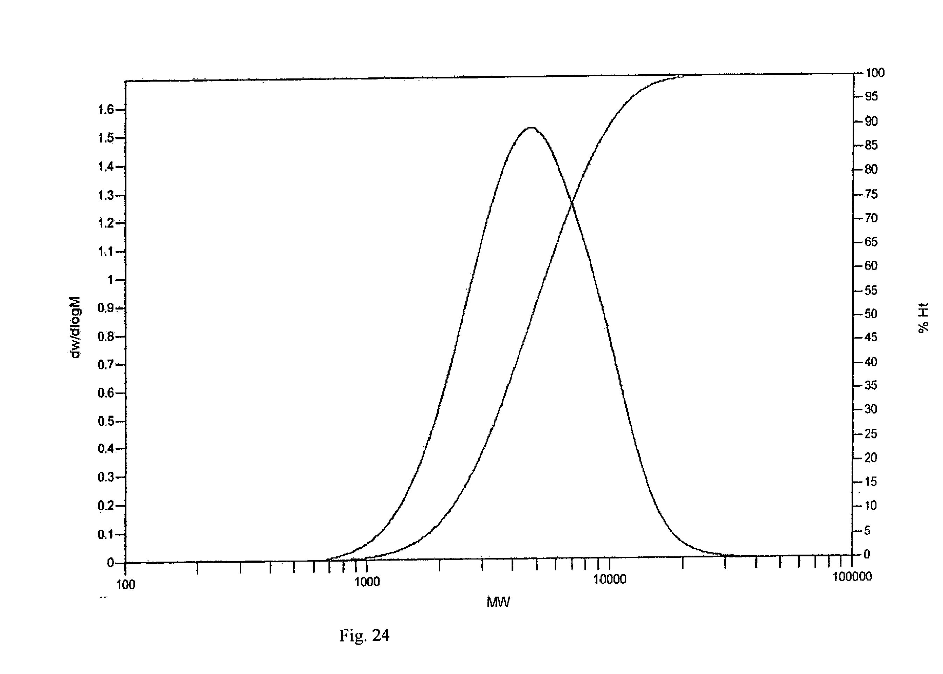

Testing result is shown in Table 20, ¹H-NMR and ¹³C-NMR spectrums are shown in Figs 23A and 23B, and GPC testing result is shown in Fig. 24.

**Table 20 Testing result of the molecular weight distribution of mPEG-PLA (2000/20000) -alanine-lysine-lysine₂**

| Name | 1H-NMR | GPC | | | | |
|---|---|---|---|---|---|---|
| mPEG-PLA (2000/20000)-alanine-lysine-lysine₂ | 21152 | Mn | Mw | Mp | Mz | P.D. |
| | | 3869 | 5388 | 4916 | 7326 | 1.3926 |

The following Examples 16-45 provide typical examples of drug carrier nanosphere or microsphere formulations which are prepared by the above mentioned methods, such as phase separation method, in-liquid drying method or spray drying method etc. using the representative methoxy end-capped polyethylene glycol-polylactic acid block copolymer or derivative thereof of the present invention as carriers.

### Example 16:

### Preparation of Rifampin microsphere

### Prescription:

| | | |
|---|---|---|
| Oil Phase : | Rifampin | 0.2g |
| | methoxy end-capped polyethylene glycol-polylactic acid block copolymer | 1.0g |
| | dichloromethane | 10ml |
| Water Phase : | 2.0% polyvinyl alcohol | 200ml |

Note: The weight average molecular weight (Mw) of methoxy end-capped polyethylene glycol-polylactic acid block copolymer is about 2000/30000 and the structural formula of which is

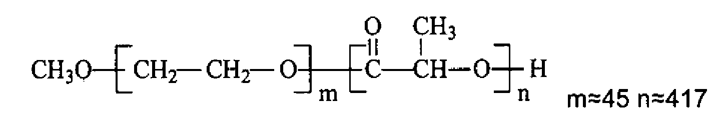

Preparation method: The in-liquid drying method is used. Rifampin and methoxy end-capped polyethylene glycol-polylactic acid block copolymer are added into dichloromethane and sonicated to dissolve. The resulting solution is slowly added to the water phase under 2500rpm high speed shear, and further sheared for 3 min, and then homogenized with microjet pump for 3 times at the pressure of 1000Bar. The resulting emulsion is placed at room temperature and stirred for 3 hours with the stirring rate of 250rpm, slowly heated to 30°C and continues to stir for 1h, further heated to 40°C and stirred for 0.5 hour, followed by filtering with 1µm of sieve mesh. The filtrate is collected and filtered with 0.2µm sieve mesh. The microspheres are collected and washed with 200ml of water for 3 times. The wet microspheres are vacuum dried at 40°C for 2h to obtain the product.

Indications: mainly used for tuberculosis and other phthisis or lepriasis. This product can be orally administrated, subcutaneously injected or intravenously injected.

Principal ingredients: Rifampin, methoxy end-capped polyethylene glycol-polylactic acid block copolymer (Mw=2000/30000)

Range of particle size and shape of microsphere: 0.2 ∼ 1µm, mostly 0.6 ∼ 0.8µm; the shape of microsphere is round.

Drug loading rate: drug loading rate is 17.3%, which is determined by HPLC method.

Encapsulation efficiency: 78.4%.

### Example 17:

### Preparation of Amlodipine microsphere

### Prescription:

| | | |
|---|---|---|
| Oil Phase : | Amlodipine | 0.1g |
| | methoxy end-capped polyethylene glycol-polylactic acid block copolymer | 0.5g |
| | dichloromethane | 10ml |
| Water Phase : | 300ml of 0.2% methylcellulose solution, adjusting the pH value to be 8.0 with citric acid and disodium hydrogen phosphate buffer salt system | |

Note: The weight average molecular weight of methoxy end-capped polyethylene glycol-polylactic acid block copolymer is 10000/40000 and the structural formula of which is

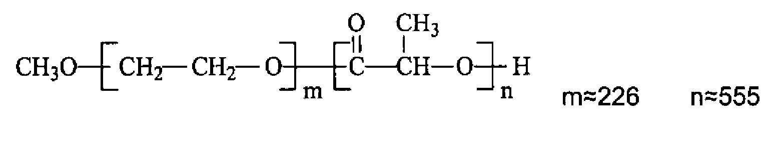

Preparation method: The in-liquid drying method is used. Amlodipine and methoxy end-capped polyethylene glycol-polylactic acid block copolymer are added into dichloromethane and sonicated to dissolve. The resulting solution is slowly added to the water phase under 3000rpm high speed shear, and further sheared for 3 min. The resulting emulsion is placed at 35°C water bath and continues to stir for 1h with the stirring rate of 300rpm, further heated to 40°C and stirred for 0.5 hour, followed by filtering with 150µm sieve mesh. The filtrate is collected and further filtered with 10µm sieve mesh. The microspheres are collected and washed with 300ml of water for 3 times. The wet microspheres are vacuum dried at 40°C for 2h to obtain the product.

Indications: mainly used for hypertension. This product can be orally administrated or subcutaneously injected.

Principal ingredients: Amlodipine, methoxy end-capped polyethylene glycol-polylactic acid block copolymer (Mw=10000/40000)

Range of particle size and shape of microsphere: 10 ∼ 150µm, mostly 20 ∼ 30µm; the shape of microsphere is relatively round.

Drug loading rate: drug loading rate is 16.5%, which is determined by HPLC method.

Encapsulation efficiency: 75.3%.

### Example 18:

### Preparation of Stavudine microsphere

### Prescription:

| | | |
|---|---|---|
| Solvent Phase : | Stavudine | 0.5g |
| | methoxy end-capped polyethylene glycol-polylactic acid block copolymer | 1g |
| | chloroform | 20ml |
| Non-solvent Phase : | Polyisobutyl ester | 0.06g |
| | cyclohexane | 200ml |

Note: The weight average molecular weight of methoxy end-capped polyethylene glycol-polylactic acid block copolymer is 5000/30000 and the structural formula of which is

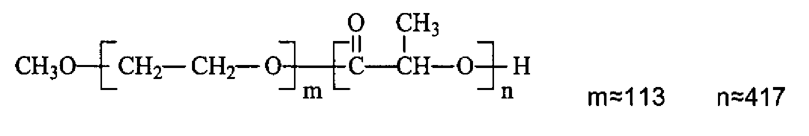

Preparation method: The phase separation method is used for preparation. Polyisobutyl ester is added into cyclohexane and sonicated to dissolve. The resulting solution is used as a non-solvent phase to reserve. Methoxy end-capped polyethylene glycol-polylactic acid block copolymer is added into chloroform and sonicated to dissolve. Then Stavudine which is micronized to particle size of less than 50µm is added, followed by strong agitation until it is uniformly dispersed. The resulting mixture is used as a solvent phase, and slowly added to the non-solvent phase under 6000rpm high speed shear, and further sheared for 10 min, then stirred for 30min with the stirring rate of 300rpm, followed by filtering with 1mm sieve mesh. The filtrate is collected and filtered with 50µm sieve mesh. The microspheres are collected and washed with 200ml of water for 5 times, vacuum dried at 40°C for 2h to obtain the product.

Indications: mainly used for AIDS infection as well as other virus infections. This microsphere can be made into oral preparations, and can also be subcutaneously injected.

Principal ingredients: Stavudine, methoxy end-capped polyethylene glycol-polylactic acid block copolymer (Mw=5000/30000)

Range of particle size and shape of microsphere: 50µm ∼ 1mm, mostly 250 ∼ 800µm; the shape of microsphere is relatively round.

Drug loading rate: drug loading rate is 43.8%, which is determined by HPLC method.

Encapsulation efficiency: 83.6%.

### Example 19:

### Preparation of Azithromycin nanosphere

### Prescription:

| | | |
|---|---|---|
| Oil Phase : | Azithromycin | 0.15g |
| | methoxy end-capped polyethylene glycol-polylactic acid | 1.0g |
| | block copolymer | |
| | dichloromethane | 10ml |
| Water Phase : | 1.0% Polyvinyl alcohol solution+0.1% Tween-80 solution | 150ml |

Note: The weight average molecular weight of methoxy end-capped polyethylene glycol-polylactic acid block copolymer is 2000/10000 and the structural formula of which is

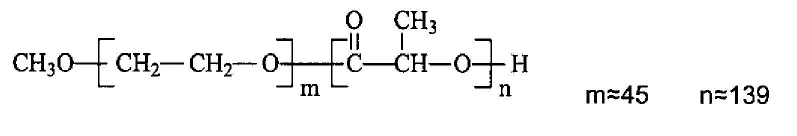

Preparation method: The in-liquid drying method is used. Azithromycin and methoxy end-capped polyethylene glycol-polylactic acid block copolymer are added into dichloromethane and sonicated to dissolve. The resulting solution is slowly added to the water phase at room temperature under 6000rpm high speed shear, and further sheared for 3 min, followed by homogenizing for 3 times with high pressure homogenizer at room temperature and the pressure of 800bar. The emulsion is placed in 30°C water bath and stirred for 2h with the stirring rate of 300rpm, further heated to 35°C and stirred for 0.5 hour, then heated to 40°C and stirred for 0.5 hour, followed by filtering with 1µm sieve mesh. The filtrate is collected and filtered with 0.2µm sieve mesh. The microspheres are collected and washed with 100ml of water for 5 times. The wet microspheres are vacuum dried at 40°C for 2h to obtain the product.

Indications: mainly used for infections of respiratory passage, skin, and soft tissue caused by sensitive microorganisms. This product can be intravenously injected or subcutaneously injected, and can also be made into oral preparations.

Principal ingredients: Azithromycin, methoxy end-capped polyethylene glycol-polylactic acid block copolymer (Mw=2000/10000)

Range of particle size and shape of microsphere: 0.2 ∼ 1µm, mostly 0.5 ∼ 0.8µm; the shape of microsphere is relatively round.

Drug loading rate: drug loading rate is 10.2%, which is determined by HPLC method.

Encapsulation efficiency: 64.3%.

### Example 20:

### Preparation of Naproxen microsphere

### Prescription:

| | | |
|---|---|---|
| Oil Phase : | Naproxen | 0.1g |
| | methoxy end-capped polyethylene glycol- | 0.5g |
| | 3-aminopropionyl end-capped polylactic acid block copolymer | |
| | dichloromethane | 10ml |
| Water Phase : | 0.1% sodium oleate solution | 200ml |

Note: The weight average molecular weight of methoxy end-capped polyethylene glycol-3-aminopropionyl polylactic acid block copolymer is 2000/40000 and the structural formula of which is

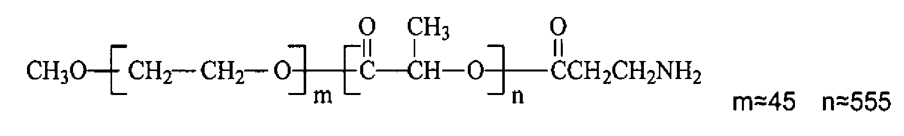

Preparation method: The in-liquid drying method is used. Naproxen and methoxy end-capped polyethylene glycol-3-aminopropionyl end-capped polylactic acid block copolymer are added into dichloromethane and sonicated to dissolve. The resulting solution is slowly added to the water phase under 4000rpm high speed shear, and further sheared for 5 min. The resulting emulsion is placed in 30°C water bath and stirred for 2h with the stirring rate of 300rpm, further heated to 35°C and stirred for 0.5 hour, then heated to 40°C and stirred for 0.5 hour, followed by filtering with 150µm sieve mesh. The filtrate is collected and further filtered with 10µm sieve mesh. The microspheres are collected and washed with 200ml of water for 5 times. The wet microspheres are vacuum dried at 40°C for 2h to obtain the product.

Indications: mainly used for rheumatoid arthritis, osteoarthritis, ankylosing spondylitis and gout etc.. This product can be subcutaneously injected and intraarticularly injected, and can also be made into oral preparations.

Principal ingredients: Naproxen, methoxy end-capped polyethylene glycol-3-aminopropionyl end-capped polylactic acid block copolymer (Mw=2000/40000)

Range of particle size and shape of microsphere: 10 ∼ 150µm, mostly 20 ∼ 30µm; the shape of microsphere is relatively round.

Drug loading rate: drug loading rate is 17.4%, which is determined by HPLC method.

Encapsulation efficiency: 89.2%.

### Example 21:

### Preparation of Ropinirole microsphere

### Prescription:

| | |
|---|---|
| Ropinirole | 2.5g |
| methoxy end-capped polyethylene glycol-polylactic acid block copolymer | 10.0g |
| glycerin monostearate | 0.1g |
| triethyl citrate | 0.5g |
| dichloromethane | 50ml |

Note: The weight average molecular weight of methoxy end-capped polyethylene glycol-polylactic acid block copolymer is 2000/20000 and the structural formula of which is

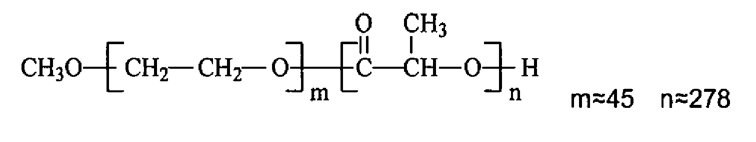

Preparation method: Methoxy end-capped polyethylene glycol-polylactic acid block copolymer is added into dichloromethane and stirred to dissolve, then glycerin monostearate, triethyl citrate and Ropinirole are added in sequence and stirred to dissolve, and followed by spray drying with the ring fan blowing rate of 90%, nitrogen pressure of 4L/min, inlet air temperature of 40°C and the feed speed of peristaltic pump of 20%. After finishing drying, microspheres are collected to obtain the product.

Indications: mainly used for Parkinson's disease. This product can be subcutaneously injected, and can also be made into oral preparations.

Principal ingredients: Ropinirole, methoxy end-capped polyethylene glycol-polylactic acid block copolymer (Mw=2000/20000)

Range of particle size and shape of microsphere: 10 ∼ 30µm; the shape of microsphere is relatively round.

Drug loading rate: drug loading rate is 22.6%, which is determined by HPLC method.

Encapsulation efficiency: 55.1%.

### Example 22:

### Preparation of Paroxetine microsphere

### Prescription:

| | | |
|---|---|---|
| Oil Phase : | Paroxetine | 0.1g |
| | methoxy end-capped polyethylene glycol-polylactic acid block copolymer | 1.0g |
| | dichloromethane | 10ml |
| Water Phase : | 0.7% acacia solution | 200ml |

Note: The weight average molecular weight of methoxy end-capped polyethylene glycol-polylactic acid block copolymer is 10000/15000 and the structural formula of which is

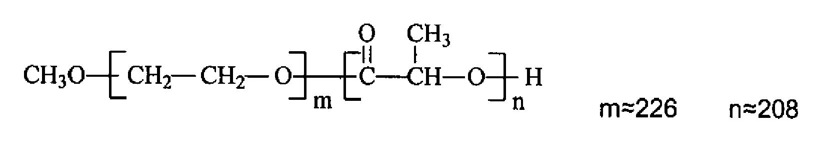

Preparation method: The in-liquid drying method is used. Paroxetine and methoxy end-capped polyethylene glycol-polylactic acid block copolymer are added into dichloromethane and sonicated to dissolve. The resulting solution is slowly added to the water phase under 6000rpm high speed shear, and further sheared for 5 min. The resulting emulsion is placed in 30°C water bath and stirred for 2h with the stirring rate of 300rpm, further heated to 35°C and stirred for 0.5 hour, then heated to 40°C and stirred for 0.5 hour, followed by filtering with 150µm sieve mesh. The filtrate is collected and further filtered with 10µm sieve mesh. The microspheres are collected and washed with 200ml of water for 5 times. The wet microspheres are lyophilized (The pre-freezed temperature is -40°C, and the primary drying temperature is 30°C) to obtain the product.

Indications: mainly used for the treatment of depression. This product can be subcutaneously injected, and can also be made into oral preparations.

Principal ingredients: Paroxetine, methoxy end-capped polyethylene glycol-polylactic acid block copolymer (Mw=10000/15000)

Range of particle size and shape of microsphere: 10 ∼ 150µm, mostly 10 ∼ 20µm; the shape of microsphere is relatively round.

Drug loading rate: drug loading rate is 8.4%, which is determined by HPLC method.

Encapsulation efficiency: 63.2%.

### Example 23:

### Preparation of Cinnarizine microsphere

### Prescription:

| | | |
|---|---|---|
| Oil Phase : | Cinnarizine | 0.2g |
| | methoxy end-capped polyethylene glycol-methyl end-capped polylactic acid block copolymer | 1.0g |
| | dichloromethane | 10ml |
| Water Phase : | 20.0% trehalose solution | 150ml |

Note: The weight average molecular weight of methoxy end-capped polyethylene glycol-methyl end-capped polylactic acid block copolymer is 5000/8000 and the structural formula of which is

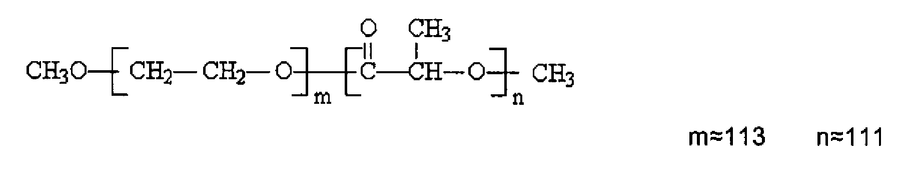

Preparation method: The in-liquid drying method is used. Cinnarizine and methoxy end-capped polyethylene glycol-methyl end-capped polylactic acid block copolymer are added into dichloromethane and sonicated to dissolve. The resulting solution is slowly added to the water phase under 5000rpm high speed shear, and further sheared for 5 min. The resulting emulsion is placed in 30°C water bath and stirred for 2h with the stirring rate of 300rpm, further heated to 35°C and stirred for 0.5 hour, then heated to 40°C and stirred for 0.5 hour, followed by filtering with 150µm sieve mesh. The filtrate is collected and further filtered with 10µm sieve mesh. The microspheres are collected and washed with 200ml of water for 5 times. The wet microspheres are dried at 40°C to obtain the product.

Indications: mainly used for cerebral thrombosis, cerebral infarction, cerebral arteriosclerosis, convalescence of cerebral hemorrhage, convalescence of subarachnoid hemorrhage, post-traumatic brain syndrome, para-equilibrium and equilibrium disorder, cronary arteriosclerosis and blood-supply disorder, and diseases caused by unhealthy peripheral circulation. This product can be subcutaneously injected, and can also be made into oral preparations.

Principal ingredients: Cinnarizine, methoxy end-capped polyethylene glycol-methyl end-capped polylactic acid block copolymer (Mw=5000/8000)

Range of particle size and shape of microsphere: 10 ∼ 150µm, mostly 20 ∼ 50µm; the shape of microsphere is relatively round.

Drug loading rate: drug loading rate is 16.4%, which is determined by HPLC method.

Encapsulation efficiency: 82.0%.

### Example 24:

### Preparation of Lovastatin microsphere

Prescription:

| | |
|---|---|
| Lovastatin | 1.5g |
| methoxy end-capped polyethylene glycol-polylactic acid block copolymer | 5.0g |
| cholesterol | 0.1g |
| diethyl phthalate | 0.5g |
| chloroform | 30ml |

Note: The weight average molecular weight of methoxy end-capped polyethylene glycol-polylactic acid block copolymer is 5000/40000 and the structural formula of which is

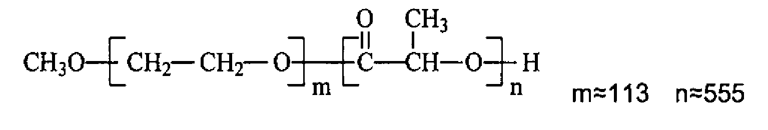

Preparation method: Methoxy end-capped polyethylene glycol-polylactic acid block copolymer is added into dichloromethane and stirred to dissolve, then cholesterol, diethyl phthalate and Lovastatin are added in sequence and stirred to dissolve, and followed by spray drying with the ring fan blowing rate of 90%, nitrogen pressure of 4L/min, inlet air temperature of 60°C and the feed speed of peristaltic pump of 15%. After finishing drying, microspheres are collected to obtain the product.

Indications: mainly used for hyperlipemia. This product can be subcutaneously injected, and can also be made into oral preparations.

Principal ingredients: Lovastatin, methoxy end-capped polyethylene glycol-polylactic acid block copolymer (Mw=5000/40000)

Range of particle size and shape of microsphere: 10 ∼ 20µm; the shape of microsphere is relatively round.

Drug loading rate: drug loading rate is 27.2%, which is determined by HPLC method.

Encapsulation efficiency: 54.4%.

### Example 25:

### Preparation of Fulvestrant microsphere

### Prescription:

| | | |
|---|---|---|
| Oil Phase : | Fulvestrant | 0.05g |
| | methoxy end-capped polyethylene glycol-polylactic acid block copolymer | 0.5g |
| | dichloromethane | 5ml |
| Water Phase : | 1.0% Polyvinyl alcohol +0.1% Tween-80 solution | 300ml |

Note: The weight average molecular weight of methoxy end-capped polyethylene glycol-polylactic acid block copolymer is 2000/20000 and the structural formula of which is

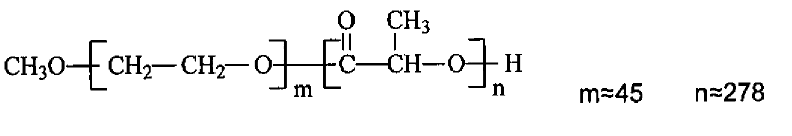

Preparation method: The in-liquid drying method is used. Fulvestrant and methoxy end-capped polyethylene glycol-polylactic acid block copolymer are added into dichloromethane and sonicated to dissolve. The resulting solution is slowly added to the water phase under 3000rpm high speed shear, and further sheared for 3 min. The resulting emulsion is placed in 30°C water bath and stirred for 2h with the stirring rate of 300rpm, further heated to 35°C and stirred for 0.5 hour, then heated to 40°C and stirred for 0.5 hour, followed by filtering with 150µm sieve mesh. The filtrate is collected and further filtered with 10µm sieve mesh. The microspheres are collected and washed with 200ml of water for 5 times. The wet microspheres are dried at 40°C to obtain the product.

Indications: mainly used for the treatment of breast cancer. This product can be subcutaneously injected.

Principal ingredients: Fulvestrant, methoxy end-capped polyethylene glycol-polylactic acid block copolymer (Mw=2000/20000)

Range of particle size and shape of microsphere: 10 ∼ 150µm, mostly 20 ∼ 50µm; the shape of microsphere is relatively round.

Drug loading rate: drug loading rate is 9.5%, which is determined by HPLC method.

Encapsulation efficiency: 68.0%.

### Example 26:

### Preparation of Orlistat microsphere

### Prescription:

| | | |
|---|---|---|
| Solvent Phase : | Orlistat | 1.0g |
| | methoxy end-capped polyethylene glycol-polylactic acid block copolymer | 1.0g |
| | dichloromethane | 20ml |
| Non-solvent Phase : | sodium oleate | 0.3g |
| | pure water | 300ml |

Note: The weight average molecular weight of methoxy end-capped polyethylene glycol-polylactic acid block copolymer is 2000/30000 and the structural formula of which is

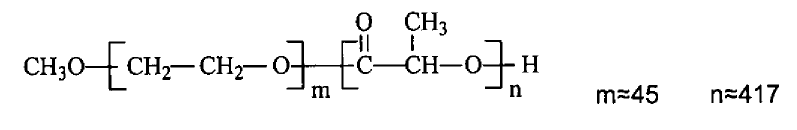

Preparation method: The phase separation method is used for preparation. Sodium oleate is added into pure water and stirred to dissolve. The resulting solution is used as a non-solvent phase to reserve. Methoxy end-capped polyethylene glycol-polylactic acid block copolymer and Orlistat are added into dichloromethane and sonicated to dissolve. The resulting solution is used as a solvent phase, and slowly added to the non-solvent phase under 800rpm high speed shear, and further sheared for 10 min, then stirred for 30min with the stirring rate of 300rpm, followed by filtering with 800µm sieve mesh. The filtrate is collected and further filtered with 10µm sieve mesh. The microspheres are collected and washed with 100ml of water for 5 times, vacuum dried at 40°C for 2h to obtain the product.

Indications: mainly used for adiposity and hyperlipemia. This microsphere can be made into oral preparations, and can also be subcutaneously injected.

Principal ingredients: Orlistat, methoxy end-capped polyethylene glycol-polylactic acid block copolymer (Mw=2000/30000)

Range of particle size and shape of microsphere: 10 ∼ 800µm, mostly 150 ∼ 600µm; the shape of microsphere is relatively round.

Drug loading rate: drug loading rate is 56.6%, which is determined by HPLC method.

Encapsulation efficiency: 75.1%.

### Example 27:

### Preparation of Fluconazol microsphere

### Prescription:

| | | |
|---|---|---|
| Solvent Phase : | Fluconazol | 1.5g |
| | methoxy end-capped polyethylene glycol-polylactic acid block copolymer | 1.0g |
| | dichloromethane | 10ml |
| Non-solvent Phase : | sodium oleate | 0.4g |
| | pure water | 200ml |

Note: The weight average molecular weight of methoxy end-capped polyethylene glycol-polylactic acid block copolymer is 20000/80000 and the structural formula of which is

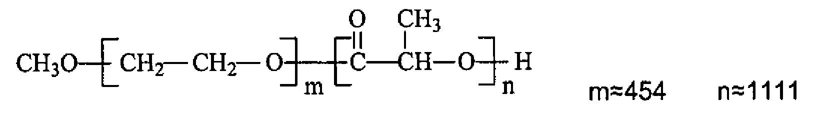

Preparation method: The phase separation method is used for preparation. Sodium oleate is added into pure water and stirred to dissolve. The resulting solution is used as a non-solvent phase to reserve. Methoxy end-capped polyethylene glycol-polylactic acid block copolymer is added into dichloromethane and sonicated to dissolve. Then micronized Fluconazol (particle size is less than 50µm) is added, followed by strong agitation until it is uniformly dispersed. The resulting mixture is used as a solvent phase, and slowly added to the non-solvent phase under 800rpm high speed shear, and further stirred for 10 min, then stirred for 30min with the stirring rate of 300rpm, followed by filtering with 1mm sieve mesh. The filtrate is collected and filtered with 80µm sieve mesh. The microspheres are collected and washed with 100ml of water for 5 times, dried at 40°C for 2h to obtain the product.

Indications: mainly used for fungal infection. This microsphere can be made into oral preparations, and can also be subcutaneously injected.

Principal ingredients: Fluconazol, methoxy end-capped polyethylene glycol-polylactic acid block copolymer (Mw=20000/80000)

Range of particle size and shape of microsphere: 80µm ∼ 1mm, mostly 250 ∼ 850µm; the shape of microsphere is relatively round.

Drug loading rate: drug loading rate is 73.3%, which is determined by HPLC method.

Encapsulation efficiency: 69.5%.

### Example 28:

### Preparation of Tramadol hydrochloride microsphere

### Prescription:

| | | |
|---|---|---|
| Solvent Phase : | Tramadol hydrochloride | 0.5g |
| | methoxy end-capped polyethylene glycol-polylactic acid block copolymer | 0.4g |
| | Span-85 | 0.1g |
| | dichloromethane | 10ml |
| | silicone oil | 40ml |
| Non-solvent | petroleum ether | appropriate amount |
| Phase : | | |

Note: The weight average molecular weight of methoxy end-capped polyethylene glycol-polylactic acid block copolymer is 1000/5000 and the structural formula of which is

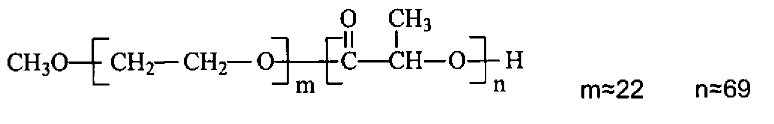

Preparation method: The phase separation method is used for preparation.
□ 5ml of dichloromethane and prescription amount of Span-85 are added into silicone oil, homogenizedto reserve.
□ Tramadol hydrochloride and methoxy end-capped polyethylene glycol-polylactic acid block copolymer are added into 5ml of dichloromethane and sonicated to dissolve.
□ ② is added into ①, homogenized, and slowly added to petroleum ether under stirring rate of 1000rpm until microspheres are no longer produced, then stirred for 30min with the stirring rate of 300rpm, followed by filtering with 1mm sieve mesh. The filtrate is collected and filtered with 50µm sieve mesh. The microspheres are collected and washed with 100ml of water for 5 times, dried at 40°C for 2h to obtain the product.

Indications: mainly used for acesodyne. This microsphere can be made into oral preparations, and can also be subcutaneously injected.

Principal ingredients: Tramadol hydrochloride, methoxy end-capped polyethylene glycol-polylactic acid block copolymer (Mw=1000/5000).

Range of particle size and shape of microsphere: 50 ∼ 1000µm, mostly 150 ∼ 600µm; the shape of microsphere is relatively round.

Drug loading rate: drug loading rate is 65.8%, which is determined by HPLC method.

Encapsulation efficiency: 66.7%.

### Example 29:

### Preparation of Clarithromycin microsphere

### Prescription:

| | | |
|---|---|---|
| Oil Phase : | Clarithromycin | 0.3g |
| | methoxy end-capped polyethylene glycol-polylactic acid block copolymer | 1.0g |
| | dichloromethane | 10ml |
| Water Phase : | 0.1 % sodium dodecyl sulfate solution | 500ml |

Note: The weight average molecular weight of methoxy end-capped polyethylene glycol-polylactic acid block copolymer is about 2000/10000 and the structural formula of which is

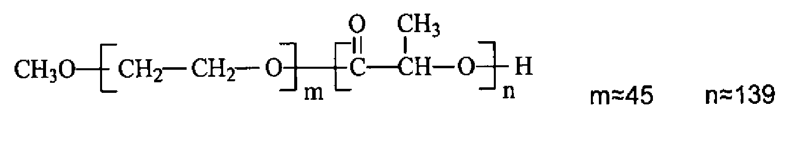

Preparation method: The in-liquid drying method is used. Clarithromycin and methoxy end-capped polyethylene glycol-polylactic acid block copolymer are added into dichloromethane and sonicated to dissolve. The resulting solution is slowly added to the water phase under 6000rpm high speed shear, and further sheared for 3 min, followed by homogenizing for 2 times with high pressure homogenizer at the pressure of 800bar. The resulting emulsion is placed in 30°C water bath and stirred for 2h with the stirring rate of 300rpm, further heated to 35°C and stirred for 0.5 hour, then heated to 40°C and stirred for 0.5 hour, followed by filtering with 1µm sieve mesh. The filtrate is collected and filtered with 0.2µm sieve mesh. The microspheres are collected and washed with 100ml of water for 5 times. The wet microspheres are lyophilized (The pre-freezed temperature is -40°C, and the primary drying temperature is 35°C) to obtain the product.

Indications: mainly used for infections caused by sensitive microorganisms. This product can be intravenously injected or subcutaneously injected, and can also be made into oral preparations.

Principal ingredients: Clarithromycin, methoxy end-capped polyethylene glycol-polylactic acid block copolymer (Mw=2000/10000)

Range of particle size and shape of microsphere: 0.2 ∼ 1µm, mostly 0.5 ∼ 0.8µm; the shape of microsphere is relatively round.

Drug loading rate: drug loading rate is 21.0%, which is determined by HPLC method.

Encapsulation efficiency: 78.3%.

### Example 30:

### Preparation of Meloxicam microsphere

### Prescription:

| | | |
|---|---|---|
| Oil Phase : | Meloxicam | 0.1g |
| | methoxy end-capped polyethylene glycol-polylactic acid block copolymer | 0.5g |
| | dichloromethane | 10ml |
| Water Phase : | 0.1% hydroxypropyl methyl cellulose solution | 150ml |

Note: The weight average molecular weight of methoxy end-capped polyethylene glycol-polylactic acid block copolymer is about 1000/40000 and the structural formula of which is

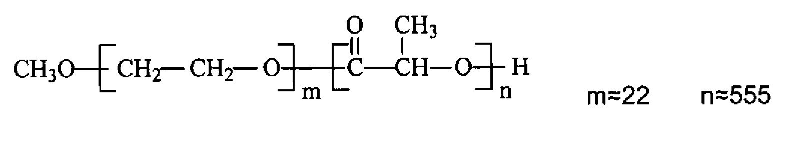

Preparation method: The in-liquid drying method is used. Meloxicam and methoxy end-capped polyethylene glycol-polylactic acid block copolymer are added into dichloromethane and sonicated to dissolve. The resulting solution is slowly added to the water phase under 3000rpm high speed shear, and further sheared for 5 min. The resulting emulsion is placed in 30°C water bath and stirred for 2h with the stirring rate of 300rpm, further heated to 35°C and stirred for 0.5 hour, then heated to 40°C and stirred for 0.5 hour, followed by filtering with 150µm sieve mesh. The filtrate is collected and further filtered with 10µm sieve mesh. The microspheres are collected and washed with 200ml of water for 5 times. The wet microspheres are dried at 40°C to obtain the product.

Indications: mainly used for rheumatoid arthritis, ankylosing spondylitis and osteoarthritis etc.. This product can be intraarticularly injected and subcutaneously injected, and can also be made into oral preparations.

Principal ingredients: Meloxicam, methoxy end-capped polyethylene glycol-polylactic acid block copolymer (Mw=1000/40000).

Range of particle size and shape of microsphere: 10 ∼ 150µm, mostly 30 ∼ 50µm; the shape of microsphere is relatively round.

Drug loading rate: drug loading rate is 14.3%, which is determined by HPLC method.

Encapsulation efficiency: 73.8%.

### Example 31:

### Preparation of Probenecid microsphere

### Prescription:

| | | |
|---|---|---|
| Oil Phase : | Probenecid | 0.05g |
| | methoxy end-capped polyethylene glycol-polylactic acid block copolymer | 0.5g |
| | dichloromethane | 5ml |
| Water Phase : | 1.0% polyvinyl alcohol solution | 75ml |

Note: The weight average molecular weight of methoxy end-capped polyethylene glycol-polylactic acid block copolymer is about 2000/30000 and the structural formula of which is

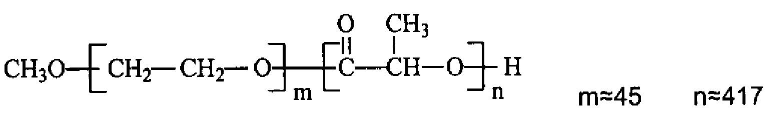

Preparation method: The in-liquid drying method is used. Probenecid and methoxy end-capped polyethylene glycol-polylactic acid block copolymer are added into dichloromethane and sonicated to dissolve. The resulting solution is slowly added to the water phase under 2000rpm high speed shear, and further sheared for 5 min. The resulting emulsion is placed in 30°C water bath and stirred for 2h with the stirring rate of 300rpm, further heated to 35°C and stirred for 0.5 hour, then heated to 40°C and stirred for 0.5 hour, followed by filtering with 150µm sieve mesh.

The filtrate is collected and further filtered with 10µm sieve mesh. The microspheres are collected and washed with 100ml of water for 5 times. The wet microspheres are dried at 40°C to obtain the product.

Indications: mainly used for the treatment of gout etc.. This product can be subcutaneously injected, and can also be made into oral preparations.

Principal ingredients: Probenecid, methoxy end-capped polyethylene glycol-polylactic acid block copolymer (Mw=2000/30000).

Range of particle size and shape of microsphere: 10 ∼ 150µm, mostly 30 ∼ 70µm; the shape of microsphere is relatively round.

Drug loading rate: drug loading rate is 9.7%, which is determined by HPLC method.

Encapsulation efficiency: 88.2%.

### Example 32:

### Preparation of Thioridazine hydrochloride microsphere

### Prescription:

| | | |
|---|---|---|
| Oil Phase : | Thioridazine hydrochloride | 0.05g |
| | methoxy end-capped polyethylene glycol-polylactic acid block copolymer | 0.5g |
| | dichloromethane | 5ml |
| Water Phase : | 1.0% polyvinyl alcohol+0.1% sodium dodecyl sulfate solution | 75ml |

Note: The weight average molecular weight of methoxy end-capped polyethylene glycol-polylactic acid block copolymer is about 2000/30000 and the structural formula of which is

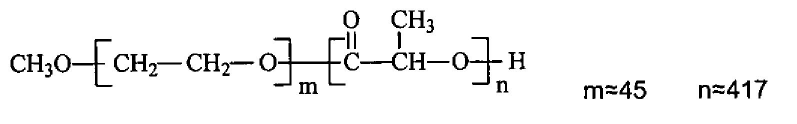

Preparation method: The in-liquid drying method is used. Thioridazine hydrochloride and methoxy end-capped polyethylene glycol-polylactic acid block copolymer are added into dichloromethane and sonicated to dissolve. The resulting solution is slowly added to the water phase under 3000rpm high speed shear, and further sheared for 5 min. The resulting emulsion is placed in 30°C water bath and stirred for 2h with the stirring rate of 300rpm, further heated to 35°C and stirred for 0.5 hour, then heated to 40°C and stirred for 0.5 hour, followed by filtering with 150µm sieve mesh. The filtrate is collected and further filtered with 10µm sieve mesh. The microspheres are collected and washed with 100ml of water for 5 times. The wet microspheres are dried at 40°C to obtain the product.

Indications: mainly used for the treatment of schizophrenia, and is applicable to schizophrenia, vesania and climacteric syndrome with agitation, anxiety and tension. This product can be subcutaneously injected.

Principal ingredients: Thioridazine hydrochloride, methoxy end-capped polyethylene glycol-polylactic acid block copolymer (Mw=2000/30000).

Range of particle size and shape of microsphere: 10 ∼ 150µm, mostly 30 ∼ 60µm; the shape of microsphere is relatively round.

Drug loading rate: drug loading rate is 9.6%, which is determined by HPLC method.

Encapsulation efficiency: 88.7%.

### Example 33:

### Preparation of Timiperone microsphere

### Prescription:

| | | |
|---|---|---|
| Oil Phase : | Timiperone | 0.2g |
| | methoxy end-capped polyethylene glycol-polylactic acid block copolymer | 1.0g |
| | dichloromethane | 10ml |
| Water Phase : | 1.0% polyvinyl alcohol+0.1% sodium dodecyl sulfate solution | 200ml |

Note: The weight average molecular weight of methoxy end-capped polyethylene glycol-polylactic acid block copolymer is about 2000/40000 and the structural formula of which is

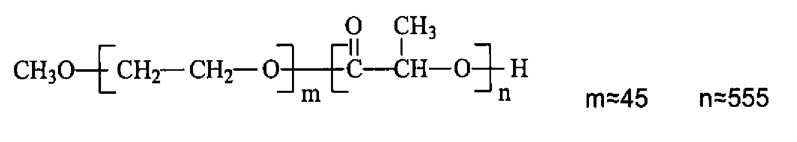

Preparation method: The in-liquid drying method is used. Timiperone and methoxy end-capped polyethylene glycol-polylactic acid block copolymer are added into dichloromethane and sonicated to dissolve. The resulting solution is slowly added to the water phase under 3000rpm high speed shear, and further sheared for 5 min. The resulting emulsion is placed in 30°C water bath and stirred for 2h with the stirring rate of 300rpm, further heated to 35°C and stirred for 0.5 hour, then heated to 40°C and stirred for 0.5 hour, followed by filtering with 150µm sieve mesh. The filtrate is collected and further filtered with 10µm sieve mesh. The microspheres are collected and washed with 200ml of water for 5 times. The wet microspheres are vacuum dried at 40°C to obtain the product.

Indications: mainly used for the treatment of schizophrenia. This product can be subcutaneously injected.

Principal ingredients: Timiperone, methoxy end-capped polyethylene glycol-polylactic acid block copolymer (Mw=2000/40000).

Range of particle size and shape of microsphere: 10 ∼ 150µm, mostly 30 ∼ 60µm; the shape of microsphere is relatively round.

Drug loading rate: drug loading rate is 15.6%, which is determined by HPLC method.

Encapsulation efficiency: 78.8%.

### Example 34:

### Preparation of Chlorprothixene microsphere

### Prescription:

| | | |
|---|---|---|
| Oil Phase : | Chlorprothixene | 0.1g |
| | methoxy end-capped polyethylene glycol-polylactic acid block copolymer | 0.5g |
| | dichloromethane | 5ml |
| Water Phase: | 1.0% polyvinyl alcohol+0.1% sodium dodecyl sulfate solution | 75ml |

Note: The weight average molecular weight of methoxy end-capped polyethylene glycol-polylactic acid block copolymer is about 2000/40000 and the structural formula of which is the same as the above Example.

Preparation method: The in-liquid drying method is used. Chlorprothixene and methoxy end-capped polyethylene glycol-polylactic acid block copolymer are added into dichloromethane and sonicated to dissolve. The resulting solution is slowly added to the water phase under 3000rpm high speed shear, and further sheared for 5 min. The resulting emulsion is placed in 30°C water bath and stirred for 2h with the stirring rate of 300rpm, further heated to 35°C and stirred for 0.5 hour, then heated to 40°C and stirred for 0.5 hour, followed by filtering with 150µm sieve mesh. The filtrate is collected and further filtered with 10µm sieve mesh. The microspheres are collected and washed with 100ml of water for 5 times. The wet microspheres are vacuum dried at 40°C to obtain the product.

Indications: mainly used for schizophrenia with anxiety or depression, climacteric depression, and anxiety neurosis etc.. This product can be subcutaneously injected.

Principal ingredients: Chlorprothixene, methoxy end-capped polyethylene glycol-polylactic acid block copolymer (Mw=2000/40000).

Range of particle size and shape of microsphere: 10 ∼ 150µm, mostly 30 ∼ 50µm; the shape of microsphere is relatively round.

Drug loading rate: drug loading rate is 17.5%, which is determined by HPLC method.

Encapsulation efficiency: 87.3%.

### Example 35:

### Preparation of Risperidone microsphere

### Prescription:

| | | |
|---|---|---|
| Oil Phase : | Risperidone | 0.1g |
| | methoxy end-capped polyethylene glycol-polylactic acid block copolymer | 0.5g |
| | dichloromethane | 5ml |
| Water Phase : | 1.0% polyvinyl alcohol + 0.1% sodium dodecyl sulfate solution | 75ml |

Note: The weight average molecular weight of methoxy end-capped polyethylene glycol-polylactic acid block copolymer is about 2000/40000 and the structural formula of which is the same as the above Example.

Preparation method: The in-liquid drying method is used. Risperidone and methoxy end-capped polyethylene glycol-polylactic acid block copolymer are added into dichloromethane and sonicated to dissolve. The resulting solution is slowly added to the water phase under 3000rpm high speed shear, and further sheared for 5 min. The resulting emulsion is placed in 30°C water bath and stirred for 2h with the stirring rate of 300rpm, further heated to 35°C and stirred for 0.5 hour, then heated to 40°C and stirred for 0.5 hour, followed by filtering with 150µm sieve mesh. The filtrate is collected and further filtered with 10µm sieve mesh. The microspheres are collected and washed with 100ml of water for 5 times. The wet microspheres are vacuum dried at 40°C to obtain the product.

Indications: mainly used for the treatment of schizophrenia, and especially have a better therapeutic effect for positive and negative symptoms and their concomitant affective symptoms (such as anxiety and depression etc.). This product can be subcutaneously injected.

Principal ingredients: Risperidone, methoxy end-capped polyethylene glycol-polylactic acid block copolymer (Mw=2000/40000).

Range of particle size and shape of microsphere: 10 ∼ 150µm, mostly 30 ∼ 70µm; the shape of microsphere is relatively round.

Drug loading rate: drug loading rate is 16.0%, which is determined by HPLC method.

Encapsulation efficiency: 86.7%.

### Example 36:

### Preparation of Alprazolam microsphere

### Prescription:

| | | |
|---|---|---|
| Oil Phase : | Alprazolam | 0.05g |
| | methoxy end-capped polyethylene glycol-polylactic acid block copolymer | 0.5g |
| | dichloromethane | 5ml |
| Water Phase : | 0.1% hydroxypropyl methyl cellulose solution | 75ml |

Note: The weight average molecular weight of methoxy end-capped polyethylene glycol-polylactic acid block copolymer is about 2000/40000 and the structural formula of which is the same as the above Example.

Preparation method: The in-liquid drying method is used. Alprazolam and methoxy end-capped polyethylene glycol-polylactic acid block copolymer are added into dichloromethane and sonicated to dissolve. The resulting solution is slowly added to the water phase under 3000rpm high speed shear, and further sheared for 5 min. The resulting emulsion is placed in 30°C water bath and stirred for 2h with the stirring rate of 300rpm, further heated to 35°C and stirred for 0.5 hour, then heated to 40°C and stirred for 0.5 hour, followed by filtering with 150µm sieve mesh. The filtrate is collected and further filtered with 10µm sieve mesh. The microspheres are collected and washed with 100ml of water for 5 times. The wet microspheres are dried at 40°C to obtain the product.

Indications: mainly used for treating anxiety, depression, and insomnia. This product can be subcutaneously injected.

Principal ingredients: Alprazolam, methoxy end-capped polyethylene glycol-polylactic acid block copolymer (Mw=2000/40000).

Range of particle size and shape of microsphere: 10 ∼ 150µm, mostly 30 ∼ 60µm; the shape of microsphere is relatively round.

Drug loading rate: drug loading rate is 9.5%, which is determined by HPLC method.

Encapsulation efficiency: 83.3%.

### Example 37:

### Preparation of Trazodone microsphere

### Prescription:

| | | |
|---|---|---|
| Oil Phase : | Trazodone | 0.1g |
| | methoxy end-capped polyethylene glycol-polylactic acid block copolymer | 0.5g |
| | dichloromethane | 5ml |
| Water Phase : | 0.1% hydroxypropyl methyl cellulose solution | 75ml |

Note: The weight average molecular weight of methoxy end-capped polyethylene glycol-polylactic acid block copolymer is about 10000/100000 and the structural formula of which is:

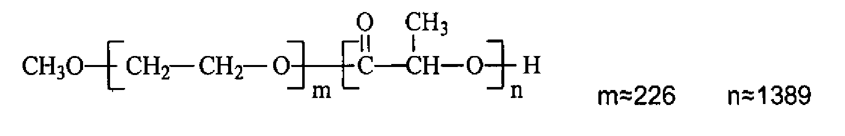

Preparation method: The in-liquid drying method is used. Trazodone and methoxy end-capped polyethylene glycol-polylactic acid block copolymer are added into dichloromethane and sonicated to dissolve. The resulting solution is slowly added to the water phase under 5000rpm high speed shear, and further sheared for 3 min. The resulting emulsion is placed in 35°C water bath and stirred for 2h, then heated to 40°C and stirred for 0.5 hour, followed by filtering with 150µm sieve mesh. The filtrate is collected and further filtered with 10µm sieve mesh. The microspheres are collected and washed with 100ml of water for 5 times. The wet microspheres are dried at 40°C to obtain the product.

Indications: mainly used for treating depression. This product can be subcutaneously injected.

Principal ingredients: Trazodone, methoxy end-capped polyethylene glycol-polylactic acid block copolymer (Mw=10000/100000).

Range of particle size and shape of microsphere: 10 ∼ 150µm, mostly 10 ∼ 30µm; the shape of microsphere is relatively round.

Drug loading rate: drug loading rate is 17.5%, which is determined by HPLC method.

Encapsulation efficiency: 90.6%.

### Example 38:

### Preparation of Famciclovir microsphere

### Prescription:

| | |
|---|---|
| Famciclovir | 0.2g |
| methoxy end-capped polyethylene glycol-polylactic acid block copolymer | 5.0g |
| glycerin monostearate | 0.1g |
| dichloromethane | 50ml |

Note: The weight average molecular weight of methoxy end-capped polyethylene glycol-polylactic acid block copolymer is 2000/20000 and the structural formula of which is

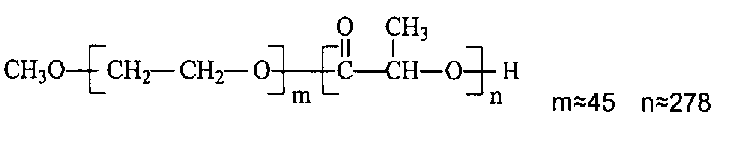

Preparation method: Methoxy end-capped polyethylene glycol-polylactic acid block copolymer is added into dichloromethane and stirred to dissolve, then glycerin monostearate and Famciclovir are added in sequence and stirred to dissolve, and followed by spray drying with the ring fan blowing rate of 90%, nitrogen pressure of 5L/min, inlet air temperature of 40°C and the feed speed of peristaltic pump of 10%. After finishing drying, microspheres are collected to obtain the product.

Indications: mainly used for virus infections. This product can be subcutaneously injected, and can also be made into oral preparations.

Principal ingredients: Famciclovir, methoxy end-capped polyethylene glycol-polylactic acid block copolymer (Mw=2000/20000)

Range of particle size and shape of microsphere: 5 ∼ 30µm; the shape of microsphere is relatively round.

Drug loading rate: drug loading rate is 36.9%, which is determined by HPLC method.

Encapsulation efficiency: 56.7%.

### Example 39:

### Preparation ofAmitriptyline hydrochloride microsphere

### Prescription:

| | |
|---|---|
| Amitriptyline hydrochloride | 0.25g |
| methoxy end-capped polyethylene glycol-polylactic acid block copolymer | 5.0g |
| dibutyl sebacate | 0.5g |
| silica gel | 0.1g |
| dichloromethane | 50ml |

Note: The weight average molecular weight of methoxy end-capped polyethylene glycol-polylactic acid block copolymer is 2000/40000 and the structural formula of which is

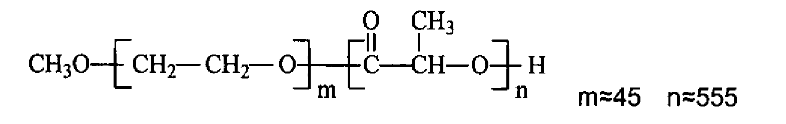

Preparation method: Methoxy end-capped polyethylene glycol-polylactic acid block copolymer is added into dichloromethane and stirred to dissolve, then dibutyl sebacate and Amitriptyline hydrochloride are added in sequence and stirred to dissolve. Then silica gel powder is added to the resulting solution, followed by strong agitation until it is uniformly dispersed, and then spray dried with the ring fan blowing rate of 90%, nitrogen pressure of 5L/min, inlet air temperature of 40°C and the feed speed of peristaltic pump of 20%. After finishing drying, microspheres are collected to obtain the product.

Indications: mainly used for treating various depressions and depressive states. This product can be subcutaneously injected, and can also be made into oral preparations.

Principal ingredients: Amitriptyline hydrochloride, methoxy end-capped polyethylene glycol-polylactic acid block copolymer (Mw=2000/40000)

Range of particle size and shape of microsphere: 10 ∼ 30µm; the shape of microsphere is relatively round.

Drug loading rate: drug loading rate is 33.4%, which is determined by HPLC method.

Encapsulation efficiency: 52.1%.

### Example 40:

### Preparation of Nimodipine microsphere

### Prescription:

| | |
|---|---|
| Nimodipine | 0.25g |
| methoxy end-capped polyethylene glycol-polylactic acid block copolymer | 5.0g |
| dichloromethane | 50ml |

Note: The weight average molecular weight of methoxy end-capped polyethylene glycol-polylactic acid block copolymer is 2000/30000 and the structural formula of which is

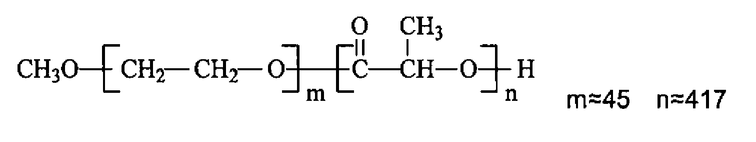

Preparation method: Nimodipine and methoxy end-capped polyethylene glycol-polylactic acid block copolymer are added into dichloromethane and sonicated to dissolve, followed by spray drying with the ring fan blowing rate of 90%, nitrogen pressure of 6L/min, inlet air temperature of 40°C and the feed speed of peristaltic pump of 10%. After finishing drying, microspheres are collected to obtain the product.

Indications: mainly used for improving blood circulation during the convalescence of acute cerebrovascular disease and cerebrovascular spasm after subarachnoid hemorrhage caused by various reasons. This product can be subcutaneously injected.

Principal ingredients: Nimodipine, methoxy end-capped polyethylene glycol-polylactic acid block copolymer (Mw=2000/30000)

Range of particle size and shape of microsphere: 10 ∼ 20µm; the shape of microsphere is relatively round.

Drug loading rate: drug loading rate is 44.7%, which is determined by HPLC method.

Encapsulation efficiency: 56.5%.

### Example 41:

### Preparation of Donepezil microsphere

### Prescription:

| | |
|---|---|
| Donepezil | 0.15g |
| methoxy end-capped polyethylene glycol-polylactic acid block copolymer | 5.0g |
| dimethyl phthalate | 0.25g |
| dichloromethane | 50ml |

Note: The weight average molecular weight of methoxy end-capped polyethylene glycol-polylactic acid block copolymer is 10000/40000 and the structural formula of which is

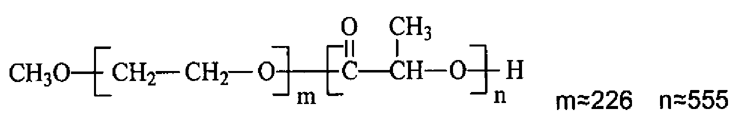

Preparation method: Methoxy end-capped polyethylene glycol-polylactic acid block copolymer is added into dichloromethane and stirred to dissolve, then dimethyl phthalate and Donepezil are added in sequence to the resulting solution and stirred to dissolve, followed by spray drying with the ring fan blowing rate of 80%, nitrogen pressure of 8L/min, inlet air temperature of 40°C and the feed speed of peristaltic pump of 20%. After finishing drying, microspheres are collected to obtain the product.

Indications: mainly used for treating senile dementia. This product can be subcutaneously injected.

Principal ingredients: Donepezil, methoxy end-capped polyethylene glycol-polylactic acid block copolymer (Mw=10000/40000)

Range of particle size and shape of microsphere: 1 ∼ 10µm; the shape of microsphere is relatively round.

Drug loading rate: drug loading rate is 27.5%, which is determined by HPLC method.

Encapsulation efficiency: 54.0%.

### Example 42:

### Preparation of Captopril microsphere

### Prescription:

| | |
|---|---|
| Captopril | 0.25g |
| methoxy end-capped polyethylene glycol-polylactic acid block copolymer | 5.0g |
| dichloromethane | 50ml |

Note: The weight average molecular weight of methoxy end-capped polyethylene glycol-polylactic acid block copolymer is 1000/30000 and the structural formula of which is

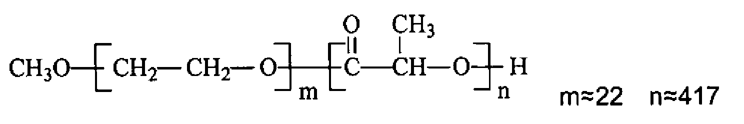

Preparation method: Captopril and methoxy end-capped polyethylene glycol-polylactic acid block copolymer are added into dichloromethane and sonicated to dissolve, followed by spray drying with the ring fan blowing rate of 90%, nitrogen pressure of 7L/min, inlet air temperature of 40°C and the feed speed of peristaltic pump of 30%. After finishing drying, microspheres are collected to obtain the product.

Indications: mainly used for hypertension. This product can be subcutaneously injected.

Principal ingredients: Captopril, methoxy end-capped polyethylene glycol-polylactic acid block copolymer (Mw=1000/30000)

Range of particle size and shape of microsphere: 1 ∼ 20µm; the shape of microsphere is relatively round.

Drug loading rate: drug loading rate is 47.7%, which is determined by HPLC method.

Encapsulation efficiency: 58.6%.

### Example 43:

### Norethindrone microsphere

### Prescription:

| | |
|---|---|
| Norethindrone | 0.2g |
| methoxy end-capped polyethylene glycol-polylactic acid block copolymer | 5.0g |
| dichloromethane | 50ml |

Note: The weight average molecular weight of methoxy end-capped polyethylene glycol-polylactic acid block copolymer is 1000/30000 and the structural formula of which is the same as the above Example.

Preparation method: Norethindrone and methoxy end-capped polyethylene glycol-polylactic acid block copolymer are added into dichloromethane and sonicated to dissolve, followed by spray drying with the ring fan blowing rate of 70%, nitrogen pressure of 6L/min, inlet air temperature of 40°C and the feed speed of peristaltic pump of 10%. After finishing drying, microspheres are collected to obtain the product.

Indications: mainly used for female contraception. This product can be subcutaneously injected.

Principal ingredients: Norethindrone, methoxy end-capped polyethylene glycol-polylactic acid block copolymer (Mw=1000/30000)

Range of particle size and shape of microsphere: 5 ∼ 20µm; the shape of microsphere is relatively round.

Drug loading rate: drug loading rate is 36.7%, which is determined by HPLC method.

Encapsulation efficiency: 56.6%.

### Example 44:

### Preparation of Gliclazide microsphere

### Prescription:

| | |
|---|---|
| Gliclazide | 0.2g |
| methoxy end-capped polyethylene glycol-polylactic acid block copolymer | 5.0g |
| dichloromethane | 50ml |

Note: The weight average molecular weight of methoxy end-capped polyethylene glycol-polylactic acid block copolymer is 2000/30000 and the structural formula of which is

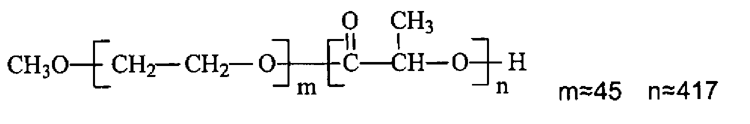

Preparation method: Gliclazide and methoxy end-capped polyethylene glycol-polylactic acid block copolymer are added into dichloromethane and sonicated to dissolve, followed by spray drying with the ring fan blowing rate of 80%, nitrogen pressure of 5L/min, inlet air temperature of 40°C and the feed speed of peristaltic pump of 20%. After finishing drying, microspheres are collected to obtain the product.

Indications: mainly used for adult diabetes, diabetic patient with adiposity or vascular lesion. This product can be subcutaneously injected.

Principal ingredients: Gliclazide, methoxy end-capped polyethylene glycol-polylactic acid block copolymer (Mw=2000/30000)

Range of particle size and shape of microsphere: 5 ∼ 20µm; the shape of microsphere is relatively round.

Drug loading rate: drug loading rate is 33.9%, which is determined by HPLC method.

Encapsulation efficiency: 51.5%.

### Example 45:

### Preparation of Melphalan microsphere

### Prescription:

| | |
|---|---|
| Melphalan | 0.1g |
| methoxy end-capped polyethylene glycol-polylactic acid block copolymer | 5.0g |
| dichloromethane | 50ml |

Note: The weight average molecular weight of methoxy end-capped polyethylene glycol-polylactic acid block copolymer is 2000/40000 and the structural formula of which is

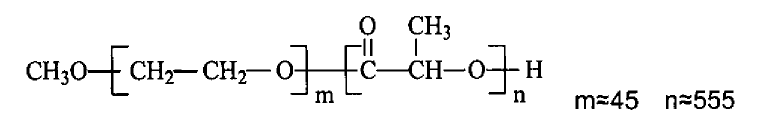

Preparation method: Melphalan and methoxy end-capped polyethylene glycol-polylactic acid block copolymer are added into dichloromethane and sonicated to dissolve, followed by spray drying with the ring fan blowing rate of 90%, nitrogen pressure of 5L/min, inlet air temperature of 40°C and the feed speed of peristaltic pump of 10%. After finishing drying, microspheres are collected to obtain the product.

Indications: mainly used for multiple myeloma, breast cancer, ovarian cancer, chronic lymphocytic and granulocytic leukemia, and malignant lymphoma etc.; used for treating limb malignant melanoma, soft tissue sarcoma and osteosarcoma by arterial perfusion. This product can be subcutaneously injected.

Principal ingredients: Melphalan, methoxy end-capped polyethylene glycol-polylactic acid block copolymer (Mw=2000/40000)

Range of particle size and shape of microsphere: 10 ∼ 20µm; the shape of microsphere is relatively round.

Drug loading rate: drug loading rate is 18.6%, which is determined by HPLC method.

Encapsulation efficiency: 53.7%.

The following Examples 46-49 provide the pharmaceutical contrast experiments and in vivo pharmacokinetic contrast experiments of the representative drug carrier nanosphere or microsphere formulations of the present invention.

### Example 46:

Pharmaceutical experimental data and preliminary study on pharmacokinetics in rats of Fulvestrant microsphere prepared with different carrier material

### 1) Pharmaceutical experimental data

### A. Sample 1: Fulvestrant microsphere, the carrier material of which is polylactic acid-glycolic acid copolymer (PLGA)

### Prescription:

| | | |
|---|---|---|
| Oil Phase : | Fulvestrant | 0.5g |
| | polylactic acid-glycolic acid copolymer (50/50, Mw=40000) | 5.0g |
| | dichloromethane | 50ml |
| Water Phase : | 1.0% Polyvinyl alcohol +0.1% Tween-80 solution | 750ml |

Note: The structural formula of polylactic acid-glycolic acid copolymer (50/50, Mw=40000) is

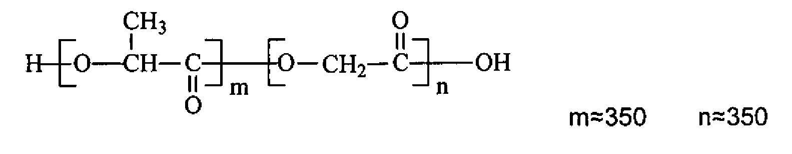

Preparation method: The in-liquid drying method is used. Fulvestrant and PLGA are added into dichloromethane and sonicated to dissolve. The resulting solution is slowly added to the water phase under 3000rpm high speed shear, and further sheared for 3 min. The resulting emulsion is placed in 30°C water bath and stirred for 2h with the stirring rate of 300rpm, further heated to 35°C and stirred for 0.5 hour, then heated to 40°C and stirred for 0.5 hour, followed by filtering with 150µm sieve mesh (there are many material solid on the sieve, which can hardly be sphere when being observed under microscope). The filtrate is collected and further filtered with 10µm sieve mesh (there are a few microspheres that are less than 10µm in the filtrate). The microspheres are collected and washed with 500ml of water for 5 times. The wet microspheres are dried at 40°C to obtain the dry product totally of 3.52g with a yield of about 64%.

Indications: mainly used for the treatment of breast cancer. This product can be subcutaneously injected.

Principal ingredients: Fulvestrant, polylactic acid-glycolic acid copolymer (50/50, Mw=40000)

Range of particle size and shape of microsphere: 10 ∼ 150µm, mostly 20 ∼ 50µm; the shape of microsphere is relatively round.

Drug loading rate: drug loading rate is 8.6%, which is determined by HPLC method.

Encapsulation efficiency: 60.2%.

### B. Sample 2: Fulvestrant microsphere, the carrier material of which is methoxy end-capped polyethylene glycol-polylactic acid block copolymer

### Prescription:

| | | |
|---|---|---|
| Oil Phase : | Fulvestrant | 0.5g |
| | methoxy end-capped polyethylene glycol-polylactic acid block copolymer | 5.0g |
| | dichloromethane | 50ml |
| Water Phase : | 1.0% Polyvinyl alcohol +0.1% Tween-80 solution | 750ml |

Note: The weight average molecular weight of methoxy end-capped polyethylene glycol-polylactic acid block copolymer is 2000/40000 and the structural formula of which is

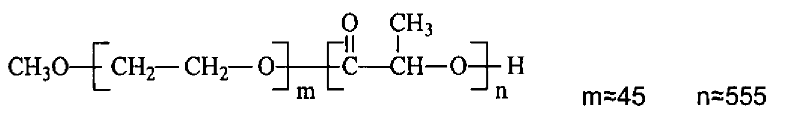

Preparation method: The in-liquid drying method is used. Fulvestrant and methoxy end-capped polyethylene glycol-polylactic acid block copolymer are added into dichloromethane and sonicated to dissolve. The resulting solution is slowly added to the water phase under 3000rpm high speed shear, and further sheared for 3 min. The resulting emulsion is placed in 30°C water bath and stirred for 2h with the stirring rate of 300rpm, further heated to 35°C and stirred for 0.5 hour, then heated to 40°C and stirred for 0.5 hour, followed by filtering with 150µm sieve mesh, (there is almost no microsphere that is greater than 150µm on the sieve). The filtrate is collected and further filtered with 10µm sieve mesh, (there are a few microspheres that are less than 10µm in the filtrate). The microspheres are collected and washed with 500ml of water for 5 times. The wet microspheres are dried at 40°C to obtain the dry product totally of 4.41 g with a yield of about 80%.

Indications: mainly used for the treatment of breast cancer. This product can be subcutaneously injected.

Principal ingredients: Fulvestrant, methoxy end-capped polyethylene glycol-polylactic acid block copolymer (Mw=2000/40000)

Range of particle size and shape of microsphere: 10 ∼ 150µm, mostly 20 ∼ 30µm; the shape of microsphere is relatively round.

Drug loading rate: drug loading rate is 11.0%, which is determined by HPLC method.

Encapsulation efficiency: 87.7%.

### 2) Pharmacokinetic experiments in rats

### Experimental Unit: China Pharmaceutical University

Instruments: Micromass Quattro micro liquid chromatograph- mass spectrograph, which contains autosampler, column oven, electrospray ionization interface, 2695 liquid chromatograph and Masslynx 4.0 MS workstation; METTLER one hundred-thousandth of electronic balance; Milli-Q Water Purifier; MICROMAX 3591 Centrifuge Desk High Speed Centrifuge (THERMO ELECTRON); Turbine mixer (Huxi analytical instrument factory, Shanghai, China).

Reagents: methanol, chromatographic grade, obtained from TEDIA Company, US; the remaining reagents are commercially available analytically pure; double distilled water, which is self prepared and purified by Milli-Q Water Purifier.

### Test drug:

Active Pharmaceutical Ingredient, Fulvestrant: 99%;
Sample 1 of Fulvestrant microsphere: 8.6%;
Sample 2 of Fulvestrant microsphere: 11.0%;
Solvent of microsphere sample: 2 bottles, 50ml/bottle;
All the above mentioned products are provided by Xi'an Libang Pharmaceutical Co., Ltd., China.

All the samples of Fulvestrant microsphere are prepared to 10mg/ml using the solvent of microsphere sample.

Emodin: internal standard, provided by the National Institute for the Control of Pharmaceutical and Biological Products, China, Batch No.: 0756-200110; and used for the content determination.

### HPLC conditions:

Mobile phase: Methanol: Water= 85:15(v:v);
Chromatographic column: 100×2.0mm, shim-pack; pre-column phenomenex C18 (ODS Octadecyl), 4mm×2.0 ID 10/pk; column temperature: 35°C; injection volume: 5µL; flow rate: 0.2 ml.min-1;
LC-MS-MS Conditions:
Capillary voltage: 3.00 kV; Cone voltage: 30 V; Extractor voltage: 3.00 V; RF Lens Voltage: 0.3 V; Source Temp: 120°C; Desolvation Temp: 400°C; Cone Gas Flow: 30 L/Hr; Desolvation gas flow: 500 L/Hr; LM1 Resolution: 13.0; HM1 Resolution: 13.0; Ion Energy: 10.5; Entrance: -2; Collision: 20; Exit: 2; LM2 Resolution: 13.0; HM2 Resolution: 13.0; Ion Energy: 10.5; Gas cell pirani pressure: 4.0e-3 millibar; Fulvestrant: [M - H+] m/z 605.6→427.4; Emodin: [M - H+] m/z 269.4→225.1.

### Experimental methods:

12 rates are all female with the body weight of 180 ∼ 220g. The rats are divided into two groups and each group has 6 rats, which are groups of Samples 1 and 2. 50mg/kg (i.e. 1ml/200g) of Fulvestrant formulation with different prescription are injected subcutaneously to the rats. 0.3ml blood is collected from the eye socket vein in a heparinized tube at 0.5, 1, 3, 6, 10, 24 hours, on Day 2, 4, 7, 10, 14, 21, 28 and Week 5, 6, 7, 8, 9, 10, 11, 12, 13 before and after administration respectively, centrifuged at 3500 rpm for 10 min. The blood plasma (0.1 ml) is collected quantificationally, and is ready for analysis.

### Data analysis:

The AUC, Tmax and Cmax as well as other parameters are calculated using the data of plasma concentration -time in each animal.

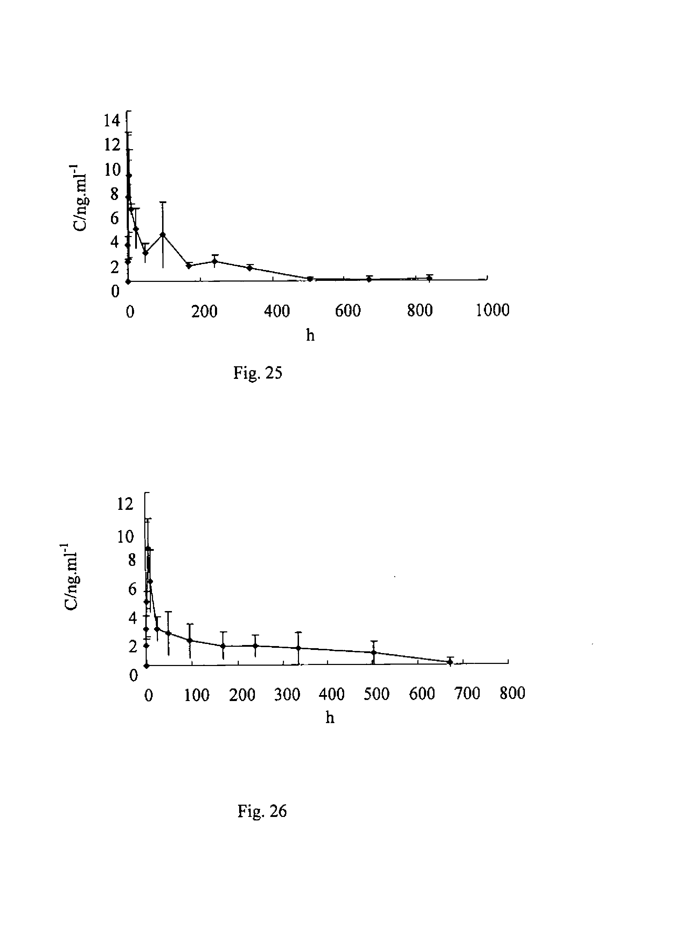

The results are shown in Fig. 25, Fig. 26 and Table 21.

**Table 21 Pharmacokinetic parameters in rats after subcutaneous injection of Fulvestrant microsphere samples**

| Parameter | AUC_{(0-∞)} | t_{1/2z} | Tₘₐₓ | Cₘₐₓ |
|---|---|---|---|---|
| Unit | ng·mL⁻¹·h | h | h | ng·mL⁻¹ |
| Sample 1 | 1456.9±806.2 | 375.9±419.8 | 5.5±1.2 | 9.8±3.2 |
| Sample 2 | 1824.2±1659.7 | 511.8±581.9 | 6.0±0.0 | 8.1±2.1 |

Experimental results: Compared with Sample 1, Sample 2 has a bigger AUC value, longer half-life, and smoother plasma drug concentration. Sample 1 has obvious burst effect, obvious double peak in the plasma drug concentration curve, and large changes in the plasma drug concentration. The results indicate that the Fulvestrant microsphere prepared by methoxy end-capped polyethylene glycol-polylactic acid block copolymer not only has greater drug loading rate and encapsulation efficiency, but also has smoother drug release rate and longer duration of sustained-release effect.

### Example 47:

Pharmaceutical experimental data and preliminary study on in vitro release of Naproxen microsphere prepared with different carrier material

### 1) Pharmaceutical experimental data

### A. Sample 1: Naproxen microsphere, the carrier material of which is polylactic acid-glycolic acid copolymer (PLGA, 75/25, molecule weight of 40000)

### Prescription:

| | | |
|---|---|---|
| Oil Phase : | Naproxen | 0.4g |
| | polylactic acid-glycolic acid copolymer (75/25, Mw= 40000) | 2.0g |
| | dichloromethane | 40ml |
| Water Phase : | 0.1 % sodium oleate solution | 800ml |

Note: the structural formula of polylactic acid-glycolic acid copolymer (75/25, Mw= 40000) is

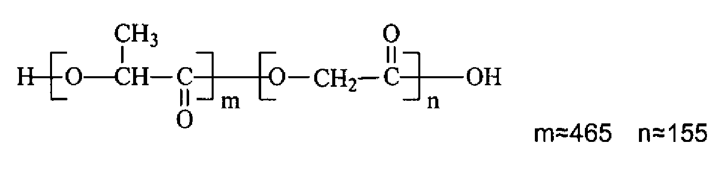

Preparation method: The in-liquid drying method is used. Naproxen and PLGA are added into dichloromethane and sonicated to dissolve. The resulting solution is slowly added to the water phase under 4000rpm high speed shear, and further sheared for 5 min. There is a lot of materials that adhere to the top of the shear to be found. The resulting emulsion is placed in 30°C water bath and stirred for 2h with the stirring rate of 300rpm, further heated to 35°C and stirred for 0.5 hour, then heated to 40°C and stirred for 0.5 hour, followed by filtering with 150µm sieve mesh, and there are many material solid on the sieve, which can hardly be sphere when being observed under microscope. The filtrate is collected and further filtered with 10µm sieve mesh, and there are fewer microspheres in the filtrate. The microspheres are collected and washed with 300ml of water for 5 times. The wet microspheres are vacuum dried at 40°C for 2h to obtain the dry microspheres of 1.45g with a yield of 60.3%.

### Indications: mainly used for rheumatoid arthritis, osteoarthritis, ankylosing spondylitis and gout etc.. This product can be subcutaneously injected and intraarticularly injected, and can also be made into oral preparations.

Principal ingredients: Naproxen, polylactic acid-glycolic acid copolymer (75/25, Mw= 40000)

Range of particle size and shape of microsphere: 10 ∼ 150µm, mostly 20 ∼ 50µm; the shape of microsphere is relatively round.

Drug loading rate: drug loading rate is 13.3%, which is determined by HPLC method.

Encapsulation efficiency: 47.6%.

### B. Sample 2: Naproxen microsphere, the carrier material of which is methoxy end-capped polyethylene glycol- polylactic acid- alanine (mPEG-PLA-alanine, Mw=2000/40000)

### Prescription:

| | | |
|---|---|---|
| Oil Phase : | Naproxen | 0.4g |
| | mPEG-PLA-alanine | 2.0g |
| | dichloromethane | 40ml |
| Water Phase : | 0.1 % sodium oleate solution | 800ml |

Note: The weight average molecular weight of mPEG-PLA-alanine is 2000/40000 and the structural formula of which is

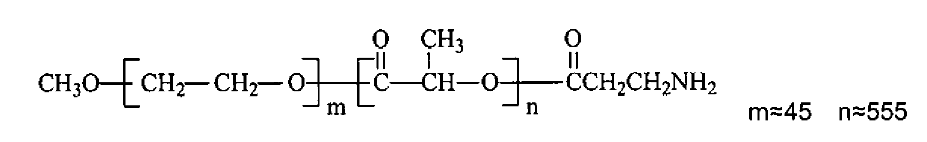

Preparation method: The in-liquid drying method is used. Naproxen and mPEG-PLA-alanine are added into dichloromethane and sonicated to dissolve. The resulting solution is slowly added to the water phase under 4000rpm high speed shear, and further sheared for 5 min. There is no material solid that adhere to the top of the shear to be found. The resulting emulsion is placed in 30°C water bath and stirred for 2h with the stirring rate of 300rpm, further heated to 35°C and stirred for 0.5 hour, then heated to 40°C and stirred for 0.5 hour, followed by filtering with 150µm sieve mesh, and there is almost no material solid on the sieve. The filtrate is collected and further filtered with 10µm sieve mesh, and there are fewer microspheres in the filtrate. The microspheres are collected and washed with 300ml of water for 5 times. The wet microspheres are vacuum dried at 40°C for 2h to obtain the dry microspheres of 2.05g with a yield of 85.4%.

Indications: mainly used for rheumatoid arthritis, osteoarthritis, ankylosing spondylitis and gout etc.. This product can be subcutaneously injected and intraarticularly injected, and can also be made into oral preparations.

Principal ingredients: Naproxen, mPEG-PLA-alanine (Mw=2000/40000).

Range of particle size and shape of microsphere: 10 ∼ 150µm, mostly 20 ∼ 40µm; the shape of microsphere is relatively round.

Drug loading rate: drug loading rate is 17.3%, which is determined by HPLC method.

Encapsulation efficiency: 88.4%.

### 2) Study on in vitro drug release

Instrument: SHA-A Water-bathing Constant Temperature Vibrator, and High Performance Liquid Chromatograph obtained from JASCO, Japan (UV-2075 UV Detector, PU-2089 injection pump, AS-2055 Autosampler).

Test drug: Naproxen with the purity of 99.0%, which is obtained from Chongqing Southwest No. 2 Pharmaceutical Factory Co., Ltd., China.

Reagents: methanol, chromatographic grade, obtained from TEDIA Company, US; potassium dihydrogen phosphate, phosphoric acid, analytically pure, obtained from Xi'an Chemical Reagent Factory, China; Tween-80, obtained from CRODA Inc., Britain.

Chromatographic conditions: The HPLC method is used. Octadecyl silane chemically bonded silica gel is used as a filler, methanol-0.01mol/L potassium dihydrogen phosphate solution (75:25, pH value is regulated to 3.0 with phosphoric acid) as a mobile phase with the detective wavelength of 240 nm, and the flow rate of 1.0ml/min.

Experimental method: 6 samples that each has 0.1g of microsphere are accurately weighed respectively, and placed in 6 glass infusion bottles respectively. 100 ml of 0.2% Tween-80 solution preheated to 37°C is accurately added to each of the glass infusion bottles. The glass infusion bottles are closed tightly with rubber stoppers and capped with aluminium caps, rapidly fixed in 37°C±2°C water bath at horizontal state, and shaked immediately with the amplitude of about 4cm at the horizontal direction and frequency of 100 times per minute. 1ml of suspension is extracted from each of the bottles through rubber stoppers at 1, 2, 4, 8, 24, 28, 32 and 48 hours after shaking (if the contents in the suspension has precipitated, it should be extracted after shaking to uniform distribution), and then 1 ml of 0.2% Tween-80 solution is supplemented to each of the bottles. The suspension is filtered through 0.2µm filter membrane as the test sample solution. In addition, appropriate amount of Naproxen is accurately weighed, to which the mobile phase is added to dissolve and dilute it to contain 50µg of Naproxen per 1ml solution as the control solution. 20µl of control solution and 20µl of test sample solution are accurately taken, and injected into the chromatograph. The chromatogram is recorded, and the cumulated release amount is calculated with peak area by external standard method.

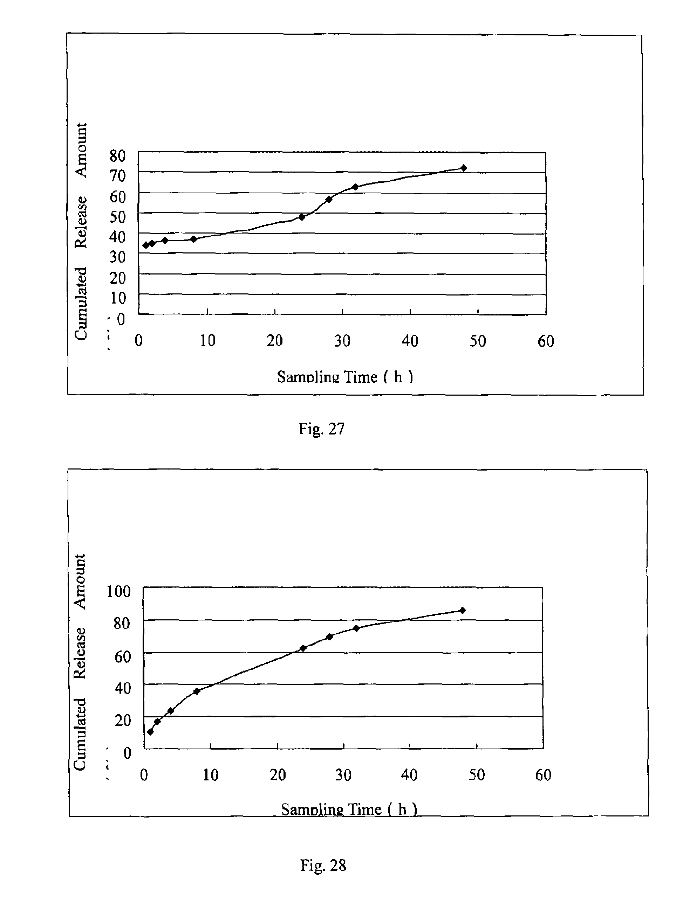

Experimental results and conclusions: The experimental results are shown in Fig. 27 and Fig. 28. The experimental results indicate that the yield, drug loading rate and encapsulation efficiency of the microsphere of Sample 2 are all apparently higher than that of Sample 1, and the in vitro drug release rate of the microsphere of Sample 2 is smoother than that of Sample 1.

The chemical structure ofNaproxen is as follows:

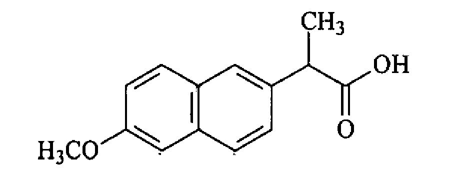

In the structure of Naproxen, it will produce an electronegative group after the ionization of carboxyl group, while the carrier material used in Sample 2 is mPEG-PLA-alanine carrying an electropositive group. Because of the special chemical property of this carrier material, the affinity between Naproxen and the carrier material has greatly enhanced in the microsphere drug carrier of Sample 2, thereby improving the drug loading rate and the encapsulation efficiency. On the other hand, it is the hydrophilic group polyethylene glycol contained in the methoxy end-capped polyethylene glycol-polylactic acid block copolymer or derivative thereof, which contributes to the formation and solidification of the microsphere, and improves the yield of drug carrier microsphere. However, the carrier material of Sample 1 does not possess this chemical property. The yield, drug loading rate and encapsulation efficiency of the microsphere of Sample 2 are all apparently higher than that of Sample 1, and the in vitro drug release rate of the microsphere of Sample 2 is smoother than that of Sample 1.

### Example 48:

Pharmaceutical experimental data and preliminary study on in vitro release of Carbamazepine microsphere prepared with different carrier material

### 1) Pharmaceutical experimental data

A. Sample 1: Carbamazepine microsphere, the carrier material of which is polylactic acid (PLA, Mw=40000)

### Prescription:

| | | |
|---|---|---|
| Oil Phase : | Carbamazepine | 0.2g |
| | polylactic acid | 2.0g |
| | dichloromethane | 20ml |
| Water Phase : | 35% saccharose +0.2% tween-80 solution | 400ml |

Note: the structural formula of polylactic acid (PLA, Mw=40000) is

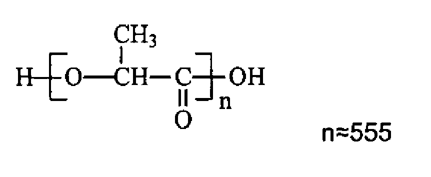

Preparation method: The in-liquid drying method is used. Carbamazepine and polylactic acid are added into dichloromethane and sonicated to dissolve. The resulting solution is slowly added to the water phase under 3000rpm high speed shear, and further sheared for 5 min. The resulting emulsion is placed in 30°C water bath and stirred for 2h with the stirring rate of 300rpm, further heated to 35°C and stirred for 0.5 hour, then heated to 40°C and stirred for 0.5 hour, followed by filtering with 150µm sieve mesh, and there are many material solid which are not formed into sphere on the sieve. The filtrate is collected and further filtered with 10µm sieve mesh, and there are fewer microspheres in the filtrate. The microspheres are collected and washed with 200ml of water for 5 times. The wet microspheres are dried at 40°C to obtain the dry microspheres of 1.40g with a yield of 63.5%.

Indications: ① anti epilepsia; ② treating trigeminal neuralgia and glossopharyngsal neuralgia; ③ treating neurogenic diabetes insipidus; ④ preventing and treating manic depression. This product can be subcutaneously injected and can also be made into oral preparations.

Principal ingredients: Carbamazepine, polylactic acid (PLA, Mw=40000).

Range of particle size and shape of microsphere: 10 ∼ 150µm, mostly 20 ∼ 50µm; the shape of microsphere is relatively round.

Drug loading rate: drug loading rate is 8.2%, which is determined by HPLC method.

Encapsulation efficiency: 56.3%.

B. Sample 2: Carbamazepine microsphere, the carrier material of which is methoxy end-capped polyethylene glycol-polylactic acid-succinic acid (mPEG-PLA-succinic acid, Mw=2000/40000)

### Prescription:

| | | |
|---|---|---|
| Oil Phase : | Carbamazepine | 0.2g |
| | mPEG-PLA-succinic acid | 2.0g |
| | dichloromethane | 20ml |
| Water Phase : | 35% saccharose +0.2% tween-80 solution | 400ml |

Note: The weight average molecular weight of mPEG-PLA-succinic acid is about 2000/40000 and the structural formula of which is

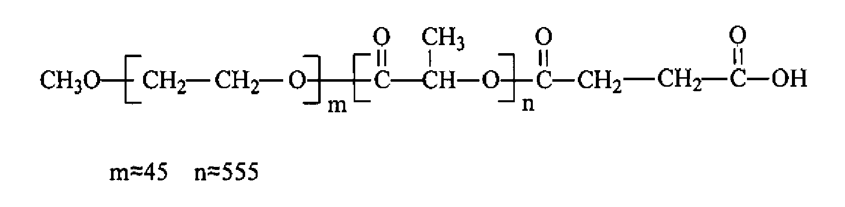

Preparation method: The in-liquid drying method is used. Carbamazepine and mPEG-PLA-succinic acid are added into dichloromethane and sonicated to dissolve. The resulting solution is slowly added to the water phase under 3000rpm high speed shear, and further sheared for 5 min. The resulting emulsion is placed in 30°C water bath and stirred for 2h with the stirring rate of 300rpm, further heated to 35°C and stirred for 0.5 hour, then heated to 40°C and stirred for 0.5 hour, followed by filtering with 150µm sieve mesh, and there is hardly any material solid or big microsphere on the sieve. The filtrate is collected and further filtered with 10µm sieve mesh, and there are a few small microspheres in the filtrate. The microspheres are collected and washed with 200ml of water for 5 times. The wet microspheres are dried at 40°C to obtain the dry microspheres of 1.75g with a yield of 79.1%.

Indications: ① anti epilepsia; ② treating trigeminal neuralgia and glossopharyngsal neuralgia; ③ treating neurogenic diabetes insipidus; ④ preventing and treating manic depression. This product can be subcutaneously injected and can also be made into oral preparations.

Principal ingredients: Carbamazepine, mPEG-PLA-succinic acid (Mw=2000/40000).

Range of particle size and shape of microsphere: 10 ∼ 150µm, mostly 20 ∼ 40µm; the shape of microsphere is relatively round.

Drug loading rate: drug loading rate is 9.3%, which is determined by HPLC method.

Encapsulation efficiency: 76.7%.

### 2) Study on in vitro drug release

Instrument: SHA-A Water-bathing Constant Temperature Vibrator, and High Performance Liquid Chromatograph obtained from JASCO, Japan (UV-2075 UV Detector, PU-2089 injection pump, AS-2055 Autosampler).

Test drug: Carbamazepine with the purity of 99.5%, which is obtained from Changzhou Yabang Pharmaceutical Co., Ltd., China.

Reagents: acetonitrile and methanol, chromatographic grade, obtained from TEDIA Company, US; glacial acetic acid, analytically pure, obtained from Xi'an Chemical Reagent Factory, China; hexadecyl trimethyl ammonium bromide, analytically pure, obtained from Tianjin Kermel Chemical Reagent Co., Ltd. China.

Chromatographic conditions: The HPLC method is used. Octadecyl silane chemically bonded silica gel is used as a filler, acetonitrile-methanol-0.05% (v/v) glacial acetic acid solution (5:5:90) as a mobile phase with the detective wavelength of 230 nm, and the flow rate of 1.0ml/min. Experimental method: 6 samples that each has 0.1g of microsphere are accurately weighed respectively, and placed in 6 glass infusion bottles respectively. 100 ml of 0.01% hexadecyl trimethyl ammonium bromide solution preheated to 37°C is accurately added to each of the glass infusion bottles. The glass infusion bottles are closed tightly with rubber stoppers and capped with aluminium caps, rapidly fixed in 37°C±2°C water bath at horizontal state, and shaked immediately with the amplitude of about 4cm at the horizontal direction and frequency of 100 times per minute. 1 ml of suspension is extracted from each of the bottles through rubber stopper at 1,2, 4, 8, 24, 28, 32, 40 and 48 hours after shaking (if the contents in the suspension has precipitated, it should be extracted after shaking to uniform distribution), and then 1 ml of 0.01% hexadecyl trimethyl ammonium bromide solution is supplemented to each of the bottles. The suspension is filtered through 0.2µm filter membrane as the test sample solution. In addition, appropriate amount of Carbamazepine is accurately weighed, to which the mobile phase is added to dissolve and dilute it to contain 25µg of Carbamazepine per 1ml solution as control solution. 20µl of control solution and 20µl of test sample solution are accurately taken, and injected into the chromatograph. The chromatogram is recorded, and the cumulated release amount is calculated with peak area by external standard method.

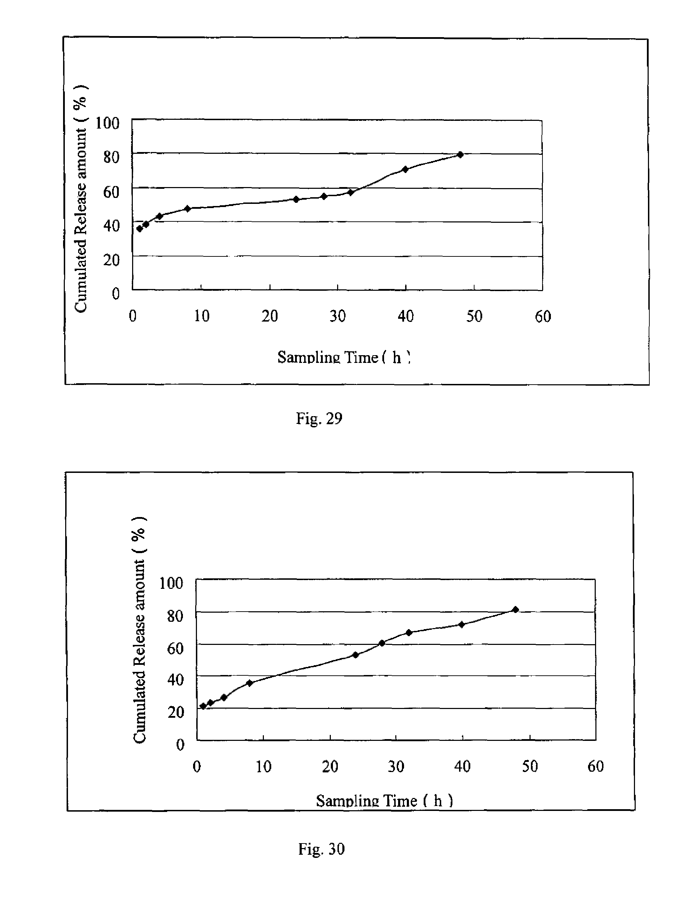

Experimental results and conclusions: The experimental results are shown in Fig. 29 and Fig. 30. The experimental results indicate that the yield, drug loading rate and encapsulation efficiency of the microsphere of Sample 2 are all apparently higher than that of Sample 1, the in vitro drug release rate of the microsphere of Sample 2 is smoother than that of Sample 1, and there is no burst effect in Sample 2 as that of in Sample 1.

The chemical structure of Carbamazepine is as follows:

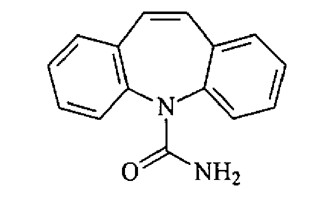

In the structure of Carbamazepine, it will produce an electropositive group when the amido group is ionizing, while the carrier material used in Sample 2 is mPEG-PLA-succinic acid with an electronegative group. Because of the special chemical property of this carrier material, the affinity between Carbamazepine and the carrier material has greatly enhanced, thereby improving the drug loading rate and the encapsulation efficiency in the microsphere drug carrier of Sample 2. On the other hand, mPEG-PLA-succinic acid includes a hydrophilic group polyethylene glycol, which contributes to the formation and solidification of the microsphere, and improves the yield of drug carrier microsphere. However, the carrier material of Sample 1 does not possess this chemical property. The yield, drug loading rate and encapsulation efficiency of the microsphere of Sample 2 are all apparently higher than that of Sample 1, and the in vitro drug release rate of the microsphere of Sample 2 is smoother than that of Sample 1.

### Example 49:

Influence of compound carrier material and single mPEG-PLA carrier material on the in vitro drug release behaviour of Carbamazepine microsphere

### 1) Preparation of Carbamazepine microsphere

A. Sample 1: Carbamazepine microsphere, the carrier material of which is methoxy end-capped polyethylene glycol-polylactic acid block copolymer (mPEG-PLA, Mw=1 000/5000)

### Prescription:

| | |
|---|---|
| Carbamazepine | 0.3g |
| mPEG-PLA(Mw=1000/5000) | 3.0g |
| dichloromethane | 30ml |

Preparation method: The spray drying method is used. Carbamazepine and mPEG-PLA are added into dichloromethane and sonicated to dissolve, followed by spray drying with the ring fan blowing rate of 90%, nitrogen flowing rate of 5L/min, inlet air temperature of 30°C and the feed speed of peristaltic pump of 10%. After finishing drying, microspheres are collected to obtain the product.

Indications: ① anti epilepsia; ② treating trigeminal neuralgia and glossopharyngsal neuralgia; ③ treating neurogenic diabetes insipidus; ④ preventing and treating manic depression. This product can be subcutaneously injected and can also be made into oral preparations.

Principal ingredients: Carbamazepine, methoxy end-capped polyethylene glycol-polylactic acid block copolymer (Mw=1000/5000).

Range of particle size and shape of microsphere: 10 ∼ 20µm, the shape of microsphere is relatively round.

Drug loading rate: drug loading rate is 9.0%, which is determined by HPLC method.

Encapsulation efficiency: 33.30%.

B. Sample 2: Carbamazepine microsphere, the carrier material of which is compound carrier material, wherein including methoxy end-capped polyethylene glycol-polylactic acid block copolymer (mPEG-PLA, Mw=1000/5000) of 50%, and polylactic acid (PLA, Mw=40000) of 50%.

### Prescription:

| | |
|---|---|
| Carbamazepine | 0.3g |
| mPEG-PLA (Mw=1000/5000) | 1.5g |
| PLA(Mw=40000) | 1.5g |
| dichloromethane | 30ml |

Preparation method: The spray drying method is used. Carbamazepine, mPEG-PLA and PLA are added into dichloromethane and sonicated to dissolve, followed by spray drying with the ring fan blowing rate of 90%, nitrogen flowing rate of 5L/min, inlet air temperature of 30°C and the feed speed of peristaltic pump of 10%. After finishing drying, microspheres are collected to obtain the product.

Indications: ① anti epilepsia; ② treating trigeminal neuralgia and glossopharyngsal neuralgia; ③ treating neurogenic diabetes insipidus; ④ preventing and treating manic depression. This product can be subcutaneously injected and can also be made into oral preparations.

Principal ingredients: Carbamazepine, methoxy end-capped polyethylene glycol-polylactic acid block copolymer, and polylactic acid.

Range of particle size and shape of microsphere: 10 ∼ 30µm, the shape of microsphere is relatively round.

Drug loading rate: drug loading rate is 9.2%, which is determined by HPLC method.

Encapsulation efficiency: 36.3%.

### 2) Study on in vitro drug release with the same method as Example 48.

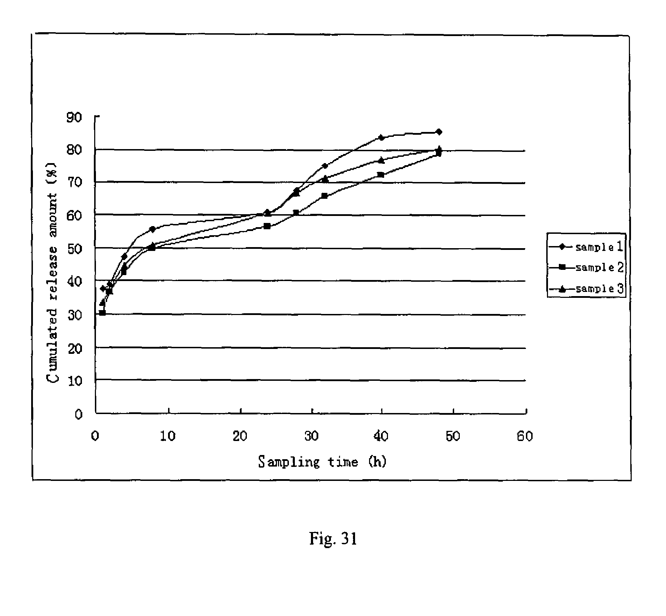

Experimental results and conclusions: The experimental results are shown in Fig. 31. The experimental results indicate that the shape, drug loading rate and encapsulation efficiency of these two samples are similar. Both of these two samples have certain burst effect, while Sample 2 is less than Sample 1. The drug release rate of Sample 2 is slower than that of Sample 1, which is consistent with the theoretical deduction. This experimental result suggest that, to mPEG-PLA polymer, adding appropriate other polymer with different properties, such as polylactic acid etc., can regulate the drug release rate of the carrier microsphere.

## Claims

1. A nanosphere or microsphere drug carrier, wherein the carrier includes a biodegradable methoxy end-capped polyethylene glycol-polylactic acid block copolymer or a derivative thereof represented by the following formula (I): 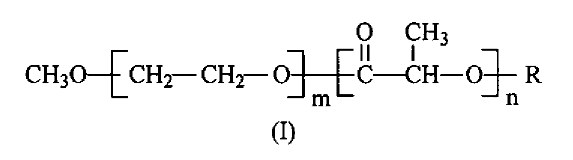 wherein:
m = 4 ∼ 454, preferably 20 ∼ 454, more preferably 120 ∼ 230 or 20 ∼ 45, and most preferably 45;
n = 4 ∼ 2778, preferably 60 ∼ 1400, more preferably 300 ∼ 1400 or 60 ∼ 150, and most preferably 400 ∼ 555;
substituent group R is selected from:
a. a neutral terminal group
-H, -CH₃, -CH₂CH₃, -CH₂(CH₂)ₓCH₃, wherein x=1-8;
b. a negatively charged terminal group
one negative charge: -COCH₂CH₂CO₂H
two negative charges: -COCH₂CH₂CONHCH(CO₂H)(CH₂)₂CO₂H
four negative charges:
-COCH₂CH₂CONHCH[CONHCH(CO₂)(CH₂)₂CO₂H](CH₂)₂[CONHCH(CO₂)(CH₂)₂CO₂H];
and
c. a positively charged terminal group
one positive charge: -COCH₂CH₂NH₂
two positive charges: -COCH₂CH₂NHCOCH(NH₂)(CH₂)₄NH₂
four positive charges:
-COCH₂CH₂NHCOCH[NHCOCH(NH₂)(CH₂)₄NH₂](CH₂)₄NH[COCH(NH₂)(CH₂)₄NH₂].

2. The drug carrier according to claim 1, wherein the HLB value of the methoxy end-capped polyethylene glycol-polylactic acid block copolymer or derivative thereof is 0.01 ∼ 19.84.

3. The drug carrier according to claim 1 or 2, wherein the drug carrier further includes one or more other high molecular materials for regulating the drug release rate; preferably, the other high molecular material is polylactic acid, polyglycolic acid, poly(lactic-co-glycolic acid), or polycaprolactone; preferably, the mass ratio of the other high molecular material to the biodegradable copolymer or derivative thereof represented by formula (I) is 0% ∼ 50%.

4. A nanosphere or microsphere drug formulation, wherein the drug formulation includes the drug carrier according to any one of claims 1-3.

5. The nanosphere or microsphere drug formulation according to claim 4, wherein the nanosphere or microsphere is the nanosphere or microsphere prepared by the drug carrier of any one of claims 1-3 enwrapping an active pharmaceutical ingredient.

6. The nanosphere or microsphere drug formulation according to claim 5, wherein the active pharmaceutical ingredient is selected from one or more of the following: antituberculosis drugs, antileprosy drugs, antiviral drugs, antimalarial drugs, antiamebic drugs, antitrichomonal drugs, antifilarial drugs, anthelminthic drugs, broad-spectrum antibiotics, antifungal drugs, analgesic drugs, analgesic-antipyretic drugs, antigout drugs, antiepileptic drugs, antiparkinsonism drugs, antipsychotic drugs, antianxiety drugs, antidepressant drugs, drugs affecting brain blood vessels, cerebral metabolism and nootropic drugs, calcium antagonists, drugs for treating chronic cardiac insufficiency, antiarrhythmic drugs, peripheral vasodilators, blood lipid regulating and antiarteriosclerotic drugs, drugs for promoting proliferation of leukocyte, antiplatelet drugs, hormons drugs, contraceptive drugs, hypoglycemic drugs, thyroid hormones drugs and antithyroid drugs, antineoplastic drugs, drugs affecting immunity, slimming drugs, anti-osteoporotic drugs and drugs against prostatic hyperplasia;
preferably, the active pharmaceutical ingredient is selected from one or more of the following: Rifampin, Amlodipine, Stavudine, Azithromycin, Naproxen, Ropinirole, Paroxetine, Cinnarizine, Lovastatin, Fulvestrant, Orlistat, Fluconazol, Tramadol hydrochloride, Carbamazepine, Clarithromycin, Meloxicam, Probenecid, Thioridazine hydrochloride, Timiperone, Chlorprothixene, Risperidone, Alprazolam, Trazodone, Famciclovir, Amitriptyline hydrochloride, Nimodipine, Donepezil, Captopril, Norethindrone, Gliclazide and Melphalan;
more preferably, the active pharmaceutical ingredient is Fulvestrant, Naproxen, or Carbamazepine.

7. The nanosphere or microsphere drug formulation according to any one of claims 4-6, wherein the particle size of the drug carrier nanosphere or microsphere is 100nm ∼ 1 mm; the drug loading rate is 0.01% ∼ 30%, preferably 5% ∼ 30%, more preferably 10% ∼ 30%, and most preferably 20% ∼ 30%.

8. A method for preparing the nanosphere or microsphere drug formulation according to any one of claims 4-7, wherein the method includes:
a. dispersing the active pharmaceutical ingredient in a solvent system containing the dissolved carrier material according to any one of claims 1-3;
b. adding into a nonsolvent system to form nanospheres or microspheres;
c. solidifying, collecting, washing and drying;
preferably, the solvent suitable for the carrier material is one or more of dichloromethane, chloroform, tetrahydrofuran, ethanol, and ethyl acetate;
preferably, the concentration of the carrier material in the solvent system is 0.1% ∼ 50% (g/ml);
preferably, the concentration of the active pharmaceutical ingredient in the solvent system which contains the dissolved carrier material is 0.01% ∼ 80% (g/ml);
preferably, the nonsolvent system is ethyl ether, petroleum ether, n-hexane, cyclohexane, or acetone;
preferably, the volume ratio of the solvent system to the nonsolvent system is 10:1 ∼ 1:10;
preferably, the nonsolvent system is slowly added and dispersed under stirring or high speed shearing or high pressure homogenizing or using microjet pump; more preferably, the stirring rate is 100 ∼ 1000rpm, the shearing rate is 1000 ∼ 10000rpm, the pressure of the high pressure homogenizer is 200 ∼ 2000bar for from one to 10 times, the pressure of the microjet pump is 100 ∼ 2000bar for from one to 10 times; and/or
preferably, adding one or more of polyisobutyl ester, polyethylene, and butyl rubber into the nonsolvent system as an antisticking agent; more preferably, the mass ratio of the antisticking agent to the carrier material is 0:10 ∼ 2:10.

9. A method for preparing the nanosphere or microsphere drug formulation according to any one of claims 4-7, wherein the method includes:
a. dissolving the active pharmaceutical ingredient and the carrier material of any one of claims 1-3 in an organic solvent to make an oil phase;
b. adding the oil phase in an aqueous phase and emulsifying to obtain an O/W type emulsion;
c. stirring and warming up the O/W type emulsion to completely volatilize the organic solvent in the O/W type emulsion;
d. filtering, washing, collecting and drying;
preferably, the solvent suitable for the carrier material is one or more of dichloromethane, chloroform, tetrahydrofuran, ethanol, and ethyl acetate;
preferably, the mass ratio of the drug to the carrier material is 1:50-1:3; a preferable concentration of the carrier material in the oil phase is 1% ∼ 50% (g/ml);
preferably, the aqueous phase is one of or a mixed solution of two or more of surfactant solution, monosaccharide or polysaccharide solution, polylol solution, cellulose solution, and colloidal solution, and the pH value of the aqueous phase is in the range of 3.0 ∼ 10.5;
preferably, the pH adjusting agent used is selected from an inorganic acid, an organic acid, an inorganic base, an organic base and a buffer salt;
preferably, the volume ratio of the oil phase to the aqueous phase is 1:300 ∼ 1:5; and/or
preferably, emulsifying with mechanical agitation or high speed shearing or high pressure homogenizing or microjet pump; more preferably, the stirring rate of the mechanical agitation is 100 ∼ 1000rpm, the shearing rate is 1000 ∼ 10000rpm, the pressure of the high pressure homogenizer and the pressure of the microjet pump are 100 ∼ 1500bar for from one to 10 times.

10. A method for preparing the nanosphere or microsphere drug formulation according to any one of claims 4-7, wherein the method includes:
a. dissolving or dispersing the drug in a solvent system containing the dissolved carrier material according to any one of claims 1-3;
b. spraying into the drying tower of a spray drying equipment in the form of spray, and drying, isolating, and collecting;
wherein the solvent suitable for the carrier material is one or more of dichloromethane, chloroform, tetrahydrofuran, ethanol, and ethyl acetate;
preferably, the concentration of the carrier material in the solvent system is 0.1% ∼ 50% (g/ml);
preferably, the concentration of the drug dissolved or dispersed in the solvent system of the carrier material is 0.01% ∼ 50% (g/ml); preferably, the inlet air temperature is 30°C ∼ 80°C;
preferably, the carrier material further comprises a plasticizer; more preferably, the plasticizer is one or more of dimethyl phthalate, diethyl phthalate, dibutyl benzoate, dibutyl sebacate, tributyl citrate, tributyl acetylcitrate, and glyceryl triacetate; the mass ratio of the plasticizer to the carrier material is 0% ∼ 50%; and/or
preferably, the solvent system further comprises an antisticking agent, the antisticking agent is one or more of cholesterol, glycerol monostearate, talc powder, silica gel, and magnesium stearate, the mass ratio of the antisticking agent to the carrier material is 0% ∼ 100%.

11. Use of a biodegradable methoxy end-capped polyethylene glycol-polylactic acid block copolymer or a derivative thereof represented by the following formula (I) in the preparation of a drug carrier, 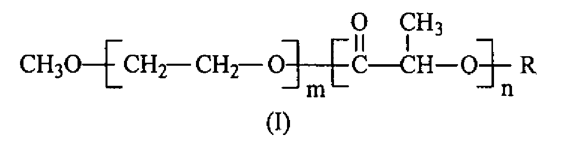 wherein:
m =4 ∼ 454, preferably 20 ∼ 454, more preferably 120 ∼ 230 or 20 ∼ 45, and most preferably 45;
n = 4 ∼ 2778, preferably 60 ∼ 1400, more preferably 300 ∼ 1400 or 60 ∼ 150, and most preferably 400 ∼ 555;
substituent group R is selected from:
a. a neutral terminal group
-H, -CH₃, -CH₂CH₃, -CH₂(CH₂)ₓCH₃, wherein x=1-8;
b. a negatively charged terminal group
one negative charge: -COCH₂CH₂CO₂
two negative charges: -COCH₂CH₂CONHCH(CO₂H)(CH₂)₂CO₂H
four negative charges:
-COCH₂CH₂CONHCH[CONHCH(CO₂)(CH₂)₂CO₂H](CH₂)_{2[}CONHCH(CO₂)(CH₂)₂CO₂H];
and
c. a positively charged terminal group
one positive charge: -COCH₂CH₂NH₂
two positive charges: -COCH₂CH₂NHCOCH(NH₂)(CH₂)₄NH₂
four positive charges:
-COCH₂CH₂NHCOCH[NHCOCH(NH₂)(CH₂)₄NH_{2]}(CH₂)₄NH[COCH(NH₂)(CH₂)₄NH_{2]}.
